# EUROPEAN PATENT APPLICATION

(11) **EP 4 174 058 A1**
(43) Date of publication of application: **03.05.2023**
(21) Application number: 21828572.4
(22) Date of filing: 24.06.2021
(51) Int. Cl.: C07D 307/91, C07D 409/12, C07D 407/12, H01L 51/00, H01L 51/50

(54) **HETEROCYCLIC COMPOUND AND ORGANIC LIGHT-EMITTING DEVICE USING SAME**

(30) Priority: 26.06.2020 KR 20200078425
(71) Applicant: LT Materials Co., Ltd., Yongin-si, Gyeonggi-do 17118 (KR)
(72) Inventor: LEE, Nam-Jin, Yongin-si, Gyeonggi-do 17118 (KR); PARK, Ji-Yeon, Yongin-si, Gyeonggi-do 17118 (KR); HEO, Yu-Jin, Yongin-si, Gyeonggi-do 17118 (KR); JEONG, Won-Jang, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/007986
(87) International publication number: WO 2021/261946

(57) **Abstract**

The present application provides a hetero-cyclic compound capable of significantly enhancing lifetime, efficiency, electrochemical stability and thermal stability of an organic light emitting device, and an organic light emitting device comprising the hetero-cyclic compound in an organic material layer.

## Description

### [Technical Field]

This application claims priority to and the benefits of Korean Patent Application No. 10-2020-0078425, filed with the Korean Intellectual Property Office on June 26, 2020, the entire contents of which are incorporated herein by reference.

The present application relates to a hetero-cyclic compound, and an organic light emitting device using the same.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

### Prior Art Documents

### Patent Documents

US Patent No. 4,356,429

### [Disclosure]

### [Technical Problem]

The present disclosure is directed to providing a hetero-cyclic compound, and an organic light emitting device comprising the same.

### [Technical Solution]

One embodiment of the present application provides a hetero-cyclic compound represented by the following Chemical Formula 1.

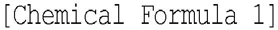

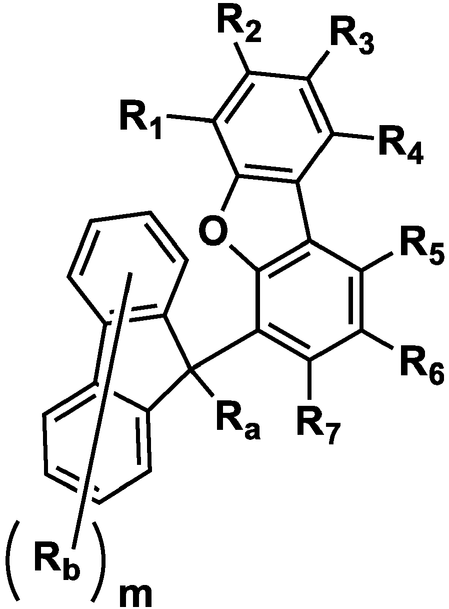

In Chemical Formula 1,
Rₐ is a halogen group; a cyano group; a substituted or unsubstituted alkyl group having 1 to 60 carbon atoms; or a substituted or unsubstituted aryl group having 6 to 60 carbon atoms,
R_{b} is hydrogen; deuterium; a halogen group; a cyano group; a substituted or unsubstituted alkyl group having 1 to 60 carbon atoms; a substituted or unsubstituted aryl group having 6 to 60 carbon atoms; or a substituted or unsubstituted heteroaryl group having 2 to 60 carbon atoms,
when Rₐ is a substituted or unsubstituted aryl group having 6 to 60 carbon atoms, only one of R₁, R₂ and R₄ to R₇ is -(La)p-A, and the rest of R₁ to R₇ are the same as or different from each other and each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms,
when Rₐ is a halogen group; a cyano group; or a substituted or unsubstituted alkyl group having 1 to 60 carbon atoms, only one of R₁ to R₇ is -(La)p-A, and the rest of R₁ to R₇ are the same as or different from each other and each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms,
La is a direct bond; a substituted or unsubstituted arylene group having 6 to 60 carbon atoms or a substituted or unsubstituted heteroarylene group having 2 to 60 carbon atoms, p is an integer of 0 to 3, and when p is 2 or greater, Las are the same as or different from each other,
A is a substituted or unsubstituted amine group, and
m is an integer of 0 to 8, and when m is 2 or greater, R_{b}s are the same as or different from each other.

Another embodiment of the present application provides an organic light emitting device comprising an anode, a cathode, and one or more organic material layers provided between the anode and the cathode, wherein one or more layers of the organic material layers comprise the hetero-cyclic compound represented by Chemical Formula 1.

### [Advantageous Effects]

A hetero-cyclic compound according to one embodiment of the present application can be used as a material of an organic material layer of an organic light emitting device. The hetero-cyclic compound can be used as a material of a hole injection layer, a hole transfer layer, a light emitting layer, an electron transfer layer, an electron injection layer, a charge generation layer and the like in an organic light emitting device. Particularly, the hetero-cyclic compound represented by Chemical Formula 1 can be used as a material of a light emitting layer of an organic light emitting device. In addition, using the hetero-cyclic compound represented by Chemical Formula 1 in an organic light emitting device is capable of lowering a driving voltage of the device, enhancing light efficiency, and enhancing lifetime properties of the device by thermal stability of the compound.

### [Description of Drawings]

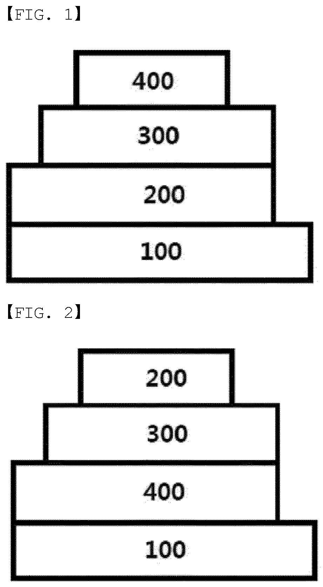

FIG. 1 to FIG. 3 are diagrams each schematically illustrating a lamination structure of an organic light emitting device according to one embodiment of the present application.

### [Mode for Disclosure]

Hereinafter, the present application will be described in detail.

One embodiment of the present application provides a hetero-cyclic compound represented by the following Chemical Formula 1.

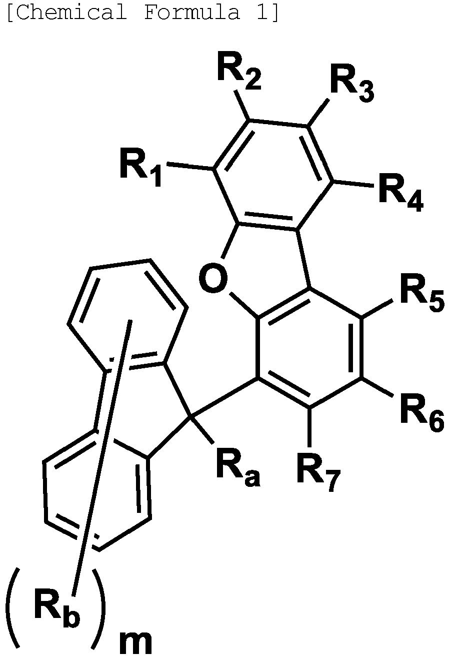

In Chemical Formula 1,
Rₐ is a halogen group; a cyano group; a substituted or unsubstituted alkyl group having 1 to 60 carbon atoms; or a substituted or unsubstituted aryl group having 6 to 60 carbon atoms,
R_{b} is hydrogen; deuterium; a halogen group; a cyano group; a substituted or unsubstituted alkyl group having 1 to 60 carbon atoms; a substituted or unsubstituted aryl group having 6 to 60 carbon atoms; or a substituted or unsubstituted heteroaryl group having 2 to 60 carbon atoms,
when Rₐ is a substituted or unsubstituted aryl group having 6 to 60 carbon atoms, only one of R₁, R₂ and R₄ to R₇ is -(La)p-A, and the rest of R₁ to R₇ are the same as or different from each other and each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms,
when Rₐ is a halogen group; a cyano group; or a substituted or unsubstituted alkyl group having 1 to 60 carbon atoms, only one of R₁ to R₇ is -(La)p-A, and the rest of R₁ to R₇ are the same as or different from each other and each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms,
La is a direct bond; a substituted or unsubstituted arylene group having 6 to 60 carbon atoms or a substituted or unsubstituted heteroarylene group having 2 to 60 carbon atoms, p is an integer of 0 to 3, and when p is 2 or greater, Las are the same as or different from each other,
A is a substituted or unsubstituted amine group, and
m is an integer of 0 to 8, and when m is 2 or greater, R_{b}s are the same as or different from each other.

By the compound represented by Chemical Formula 1 having a specific substituent comprising a dibenzofuran group on the fluorene, pi-pi stacking of the aromatic ring is suppressed, and by the dibenzofuran group having an amine group with hole properties as a substituent, a wide band gap and stability are obtained by having excellent hole transfer properties. As a result, using the compound represented by Chemical Formula 1 in an organic light emitting device is effective in lowering a driving voltage, and obtaining superior efficiency and lifetime properties in the organic light emitting device.

In the present specification, the term "substitution" means a hydrogen atom bonding to a carbon atom of a compound being changed to another substituent, and the position of substitution is not limited as long as it is a position at which the hydrogen atom is substituted, that is, a position at which a substituent can substitute, and when two or more substituents substitute, the two or more substituents may be the same as or different from each other.

In the present specification, the "substituted or unsubstituted" means being substituted with one or more substituents selected from the group consisting of deuterium; a halogen group; a cyano group; linear or branched alkyl having 1 to 60 carbon atoms; linear or branched alkenyl having 2 to 60 carbon atoms; linear or branched alkynyl having 2 to 60 carbon atoms; monocyclic or polycyclic cycloalkyl having 3 to 60 carbon atoms; monocyclic or polycyclic heterocycloalkyl having 2 to 60 carbon atoms; monocyclic or polycyclic aryl having 6 to 60 carbon atoms; monocyclic or polycyclic heteroaryl having 2 to 60 carbon atoms; -SiRR'R"; -P(=O)RR'; alkylamine having 1 to 20 carbon atoms; monocyclic or polycyclic arylamine having 6 to 60 carbon atoms; and monocyclic or polycyclic heteroarylamine having 2 to 60 carbon atoms, or being unsubstituted, or being substituted with a substituent linking two or more substituents selected from among the substituents illustrated above, or being unsubstituted, and R, R' and R" are the same as or different from each other and each independently hydrogen; deuterium; halogen; substituted or unsubstituted alkyl having 1 to 60 carbon atoms; substituted or unsubstituted aryl having 6 to 60 carbon atoms; or substituted or unsubstituted heteroaryl having 2 to 60 carbon atoms.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0%, a hydrogen content being 100% or substituents being all hydrogen.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 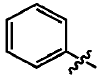 means that the total number of substituents that the phenyl group may have is 5 (T1 in the formula), and the number of deuterium among these is 1 (T2 in the formula) . In other words, having a deuterium content of 20% in a phenyl group may be represented by the following structural formulae.

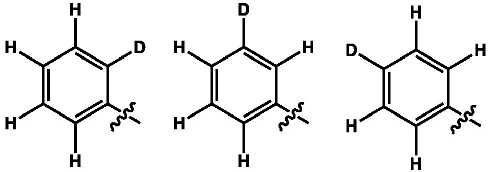

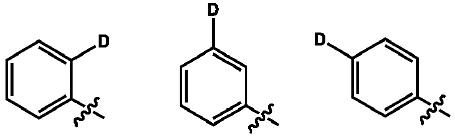

In addition, in one embodiment of the present application, "a phenyl group having a deuterium content of 0%" may mean a phenyl group that does not include a deuterium atom, that is, a phenyl group that has 5 hydrogen atoms.

In the present specification, the halogen may be fluorine, chlorine, bromine or iodine.

In the present specification, the alkyl group includes linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group includes monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group includes a spiro group. The number of carbon atoms of the aryl group may be from 6 to 60, specifically from 6 to 40 and more specifically from 6 to 25. Specific examples of the aryl group may include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the phosphine oxide group is represented by -P(=O)R101R102, and R101 and R102 are the same as or different from each other and may be each independently a substituent formed with at least one of hydrogen; deuterium; a halogen group; an alkyl group; an alkenyl group; an alkoxy group; a cycloalkyl group; an aryl group; and a hetero-cyclic group. Specific examples of the phosphine oxide group may include a diphenylphosphine oxide group, a dinaphthylphosphine oxide group and the like, but are not limited thereto.

In the present specification, the silyl group is a substituent including Si, having the Si atom directly linked as a radical, and is represented by -SiR104R105R106. R104 to R106 are the same as or different from each other, and may be each independently a substituent formed with at least one of hydrogen; deuterium; a halogen group; an alkyl group; an alkenyl group; an alkoxy group; a cycloalkyl group; an aryl group; and a hetero-cyclic group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the fluorenyl group may be substituted, and adjacent substituents may bond to each other to form a ring.

In the present specification, the spiro group is a group including a spiro structure, and may have 15 to 60 carbon atoms. For example, the spiro group may include a structure in which a 2,3-dihydro-1H-indene group or a cyclohexane group spiro bonds to a fluorenyl group. Specifically, the spiro group may include any one of groups of the following structural formulae.

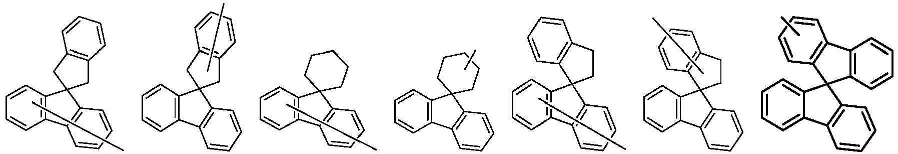

In the present specification, the heteroaryl group includes S, O, Se, N or Si as a heteroatom, includes monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydrodibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, 5,10-dihydrobenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except that these are each a divalent group. In addition, the heteroarylene group means the heteroaryl group having two bonding sites, that is, a divalent group. The descriptions on the heteroaryl group provided above may be applied thereto except that these are each a divalent group.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

The hetero-cyclic compound according to one embodiment of the present application is represented by Chemical Formula 1. More specifically, by having a core structure and structural properties of the substituents as above, the hetero-cyclic compound represented by Chemical Formula 1 may be used as a material of an organic material layer of an organic light emitting device.

In one embodiment of the present application, the compound represented by Chemical Formula 1 may have a deuterium content of greater than or equal to 0% and less than or equal to 100%.

In one embodiment of the present application, the compound represented by Chemical Formula 1 may have a deuterium content of greater than 0% and less than or equal to 100%.

In one embodiment of the present application, the compound represented by Chemical Formula 1 may have a deuterium content of greater than or equal to 10% and less than or equal to 100%.

In one embodiment of the present application, Rₐ of Chemical Formula 1 may be a halogen group; a cyano group; a substituted or unsubstituted alkyl group having 1 to 60 carbon atoms; or a substituted or unsubstituted aryl group having 6 to 60 carbon atoms.

In one embodiment of the present application, Rₐ may be a halogen group; a cyano group; a substituted or unsubstituted alkyl group having 1 to 40 carbon atoms; or a substituted or unsubstituted aryl group having 6 to 40 carbon atoms.

In one embodiment of the present application, Rₐ may be a halogen group; a cyano group; a substituted or unsubstituted alkyl group having 1 to 20 carbon atoms; or a substituted or unsubstituted aryl group having 6 to 20 carbon atoms.

In one embodiment of the present application, Rₐ may be a halogen group; a cyano group; a substituted or unsubstituted methyl group; or a substituted or unsubstituted phenyl group.

In another embodiment, Rₐ may be a substituted or unsubstituted methyl group; or a substituted or unsubstituted phenyl group.

In another embodiment, Rₐ is a methyl group unsubstituted or substituted with deuterium; or a phenyl group unsubstituted or substituted with deuterium.

In another embodiment, Rₐ is a methyl group; or a phenyl group.

In another embodiment, Rₐ is a methyl group.

In another embodiment, Rₐ is a phenyl group.

In one embodiment of the present application, R_{b} of Chemical Formula 1 may be hydrogen; deuterium; a halogen group; a cyano group; a substituted or unsubstituted alkyl group having 1 to 60 carbon atoms; a substituted or unsubstituted aryl group having 6 to 60 carbon atoms; or a substituted or unsubstituted heteroaryl group having 2 to 60 carbon atoms.

In one embodiment of the present application, R_{b} may be hydrogen; deuterium; a halogen group; a cyano group; a substituted or unsubstituted alkyl group having 1 to 60 carbon atoms; a substituted or unsubstituted aryl group having 6 to 40 carbon atoms; or a substituted or unsubstituted heteroaryl group having 2 to 40 carbon atoms.

In one embodiment of the present application, R_{b} may be hydrogen; deuterium; a halogen group; a cyano group; a substituted or unsubstituted alkyl group having 1 to 60 carbon atoms; a substituted or unsubstituted aryl group having 6 to 20 carbon atoms; or a substituted or unsubstituted heteroaryl group having 2 to 20 carbon atoms.

In another embodiment, R_{b} is hydrogen; deuterium; a halogen group; a cyano group; or a substituted or unsubstituted alkyl group having 1 to 60 carbon atoms.

In another embodiment, R_{b} is hydrogen; deuterium; or a halogen group.

In another embodiment, R_{b} is hydrogen; or deuterium.

In another embodiment, R_{b} is hydrogen.

In another embodiment, R_{b} is deuterium.

In another embodiment, R_{b} is a substituted or unsubstituted alkyl group having 1 to 60 carbon atoms; a substituted or unsubstituted aryl group having 6 to 60 carbon atoms; or a substituted or unsubstituted heteroaryl group having 2 to 60 carbon atoms.

In one embodiment of the present application, when Rₐ is a substituted or unsubstituted aryl group having 6 to 60 carbon atoms, only one of R₁, R₂ and R₄ to R₇ is -(La)p-A in R₁ to R₇ of Chemical Formula 1, and the rest of R₁ to R₇ are the same as or different from each other and each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms, and when Rₐ is a halogen group; a cyano group; or a substituted or unsubstituted alkyl group having 1 to 60 carbon atoms, only one of R₁ to R₇ is -(La)p-A, and the rest of R₁ to R₇ are the same as or different from each other and may be each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms.

In one embodiment of the present application, when Rₐ is a substituted or unsubstituted aryl group having 6 to 60 carbon atoms, only one of R₁, R₂ and R₄ to R₇ is -(La)p-A in R₁ to R₇, and the rest of R₁ to R₇ are the same as or different from each other and may be each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms.

In one embodiment of the present application, when Rₐ is a substituted or unsubstituted aryl group having 6 to 60 carbon atoms, only one of R₁, R₂ and R₄ to R₇ is -(La)p-A in R₁ to R₇, and the rest of R₁ to R₇ are the same as or different from each other and may be each independently selected from the group consisting of hydrogen; deuterium; a halogen group; a cyano group; a substituted or unsubstituted alkyl group having 1 to 40 carbon atoms; a substituted or unsubstituted aryl group having 6 to 40 carbon atoms; and a substituted or unsubstituted heteroaryl group having 2 to 40 carbon atoms.

In one embodiment of the present application, when Rₐ is a substituted or unsubstituted aryl group having 6 to 60 carbon atoms, only one of R₁, R₂ and R₄ to R₇ is -(La)p-A in R₁ to R₇, and the rest of R₁ to R₇ are the same as or different from each other and may be each independently selected from the group consisting of hydrogen; deuterium; a halogen group; a cyano group; a substituted or unsubstituted alkyl group having 1 to 20 carbon atoms; a substituted or unsubstituted aryl group having 6 to 20 carbon atoms; and a substituted or unsubstituted heteroaryl group having 2 to 60 carbon atoms.

The descriptions and the definitions on R₁ to R₇ when Rₐ is a substituted or unsubstituted aryl group having 6 to 60 carbon atoms also apply in the same manner when Rₐ is a substituted or unsubstituted aryl group having 6 to 40 carbon atoms; or a substituted or unsubstituted aryl group having 6 to 20 carbon atoms.

In other words, when Rₐ is a substituted or unsubstituted aryl group having 6 to 40 carbon atoms, only one of R₁, R₂ and R₄ to R₇ is -(La)p-A, and the rest of R₁ to R₇ are the same as or different from each other and may be each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms.

Likewise, when Rₐ is a substituted or unsubstituted aryl group having 6 to 20 carbon atoms, only one of R₁, R₂ and R₄ to R₇ is -(La)p-A, and the rest of R₁ to R₇ are the same as or different from each other and may be each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms.

In one embodiment of the present application, when Rₐ is a halogen group; a cyano group; or a substituted or unsubstituted alkyl group having 1 to 60 carbon atoms, only one of R₁ to R₇ is -(La)p-A in R₁ to R₇, and the rest of R₁ to R₇ are the same as or different from each other and may be each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms.

In one embodiment of the present application, when Rₐ is a halogen group; a cyano group; or a substituted or unsubstituted alkyl group having 1 to 60 carbon atoms, only one of R₁ to R₇ is -(La)p-A in R₁ to R₇, and the rest of R₁ to R₇ are the same as or different from each other and may be each independently selected from the group consisting of hydrogen; deuterium; a halogen group; a cyano group; a substituted or unsubstituted alkyl group having 1 to 40 carbon atoms; a substituted or unsubstituted aryl group having 6 to 40 carbon atoms; and a substituted or unsubstituted heteroaryl group having 2 to 40 carbon atoms.

In one embodiment of the present application, when Rₐ is a halogen group; a cyano group; or a substituted or unsubstituted alkyl group having 1 to 60 carbon atoms, only one of R₁ to R₇ is -(La)p-A in R₁ to R₇, and the rest of R₁ to R₇ are the same as or different from each other and may be each independently selected from the group consisting of hydrogen; deuterium; a halogen group; a cyano group; a substituted or unsubstituted alkyl group having 1 to 20 carbon atoms; a substituted or unsubstituted aryl group having 6 to 60 carbon atoms; and a substituted or unsubstituted heteroaryl group having 2 to 20 carbon atoms.

The descriptions and the definitions on R₁ to R₇ when Rₐ is a halogen group; a cyano group; or a substituted or unsubstituted alkyl group having 1 to 60 carbon atoms also apply in the same manner when Rₐ is a halogen group; a cyano group; or a substituted or unsubstituted alkyl group having 1 to 40 carbon atoms, or when Rₐ is a halogen group; a cyano group; or a substituted or unsubstituted alkyl group having 1 to 20 carbon atoms.

In other words, when Rₐ is a halogen group; a cyano group; or a substituted or unsubstituted alkyl group having 1 to 40 carbon atoms, only one of R₁ to R₇ is -(La)p-A, and the rest of R₁ to R₇ are the same as or different from each other and may be each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms.

Likewise, when Rₐ is a halogen group; a cyano group; or a substituted or unsubstituted alkyl group having 1 to 20 carbon atoms, only one of R₁ to R₇ is -(La)p-A, and the rest of R₁ to R₇ are the same as or different from each other and may be each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms.

In one embodiment of the present application, La is a direct bond; a substituted or unsubstituted arylene group having 6 to 60 carbon atoms or a substituted or unsubstituted heteroarylene group having 2 to 60 carbon atoms, p is an integer of 0 to 3, and when p is 2 or greater, Las are the same as or different from each other, and A is a substituted or unsubstituted amine group.

In one embodiment of the present application, La is a direct bond; a substituted or unsubstituted arylene group having 6 to 60 carbon atoms or a substituted or unsubstituted heteroarylene group having 2 to 60 carbon atoms, p is an integer of 0 to 3, and when p is 2 or greater, Las are the same as or different from each other.

In one embodiment of the present application, La may be a direct bond; a substituted or unsubstituted arylene group having 6 to 40 carbon atoms or a substituted or unsubstituted heteroarylene group having 2 to 40 carbon atoms.

In one embodiment of the present application, La may be a direct bond; or a substituted or unsubstituted arylene group having 6 to 40 carbon atoms.

In one embodiment of the present application, La may be a direct bond; or a substituted or unsubstituted arylene group having 6 to 20 carbon atoms.

In one embodiment of the present application, La is a direct bond.

In one embodiment of the present application, La is a phenylene group.

In one embodiment of the present application, A is a substituted or unsubstituted amine group.

In one embodiment of the present application, m of Chemical Formula 1 is an integer of 0 to 8, and when m is 2 or greater, R_{b}s are the same as or different from each other.

In one embodiment of the present application, Chemical Formula 1 may be represented by any one of the following Chemical Formulae 2 to 7.

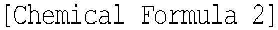

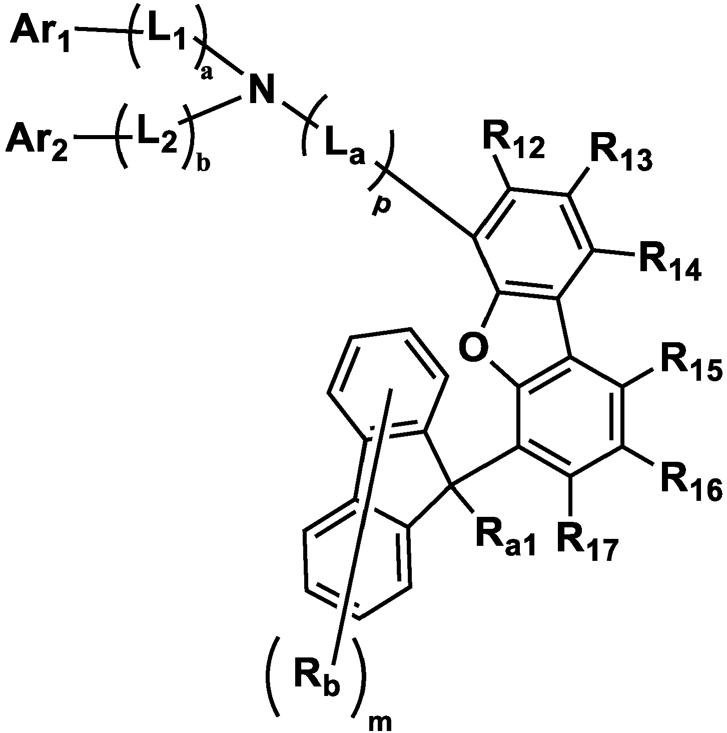

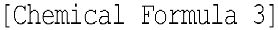

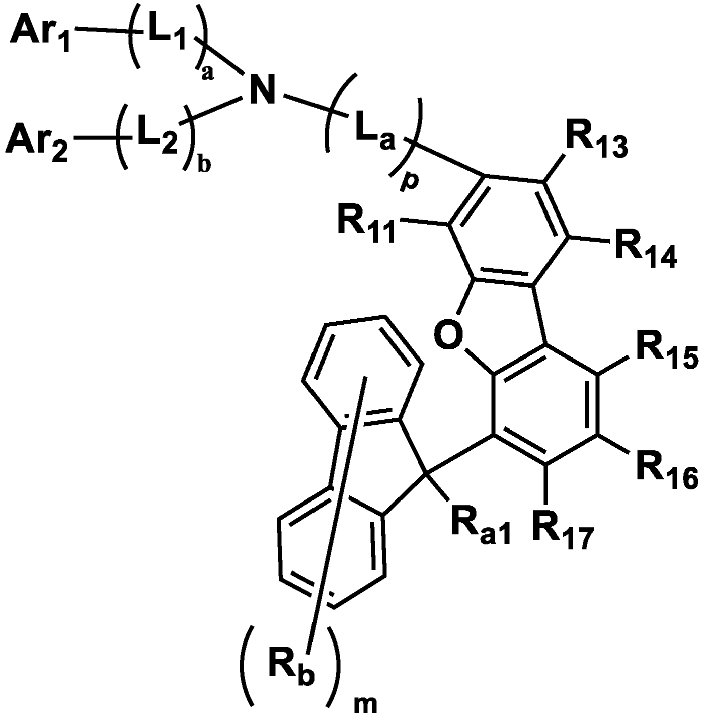

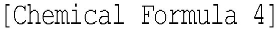

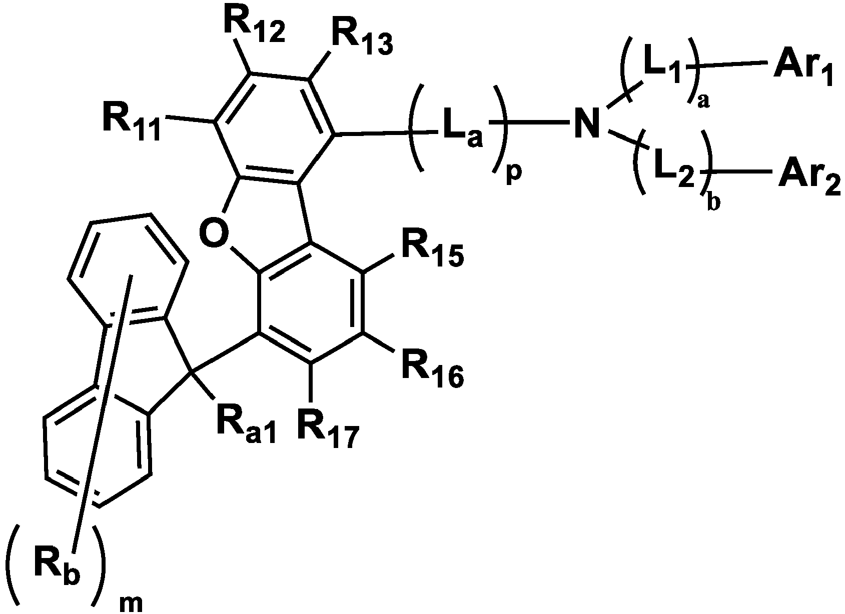

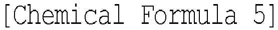

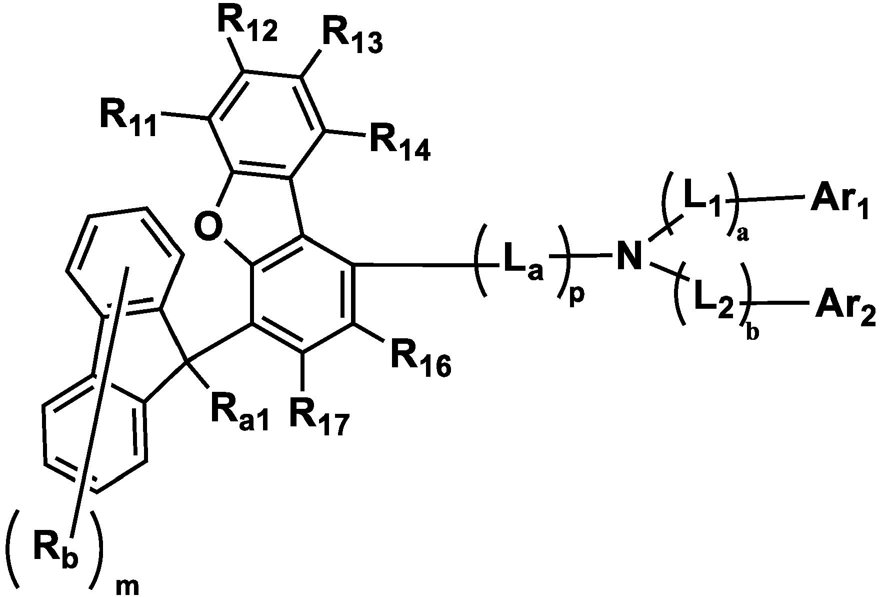

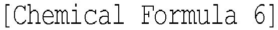

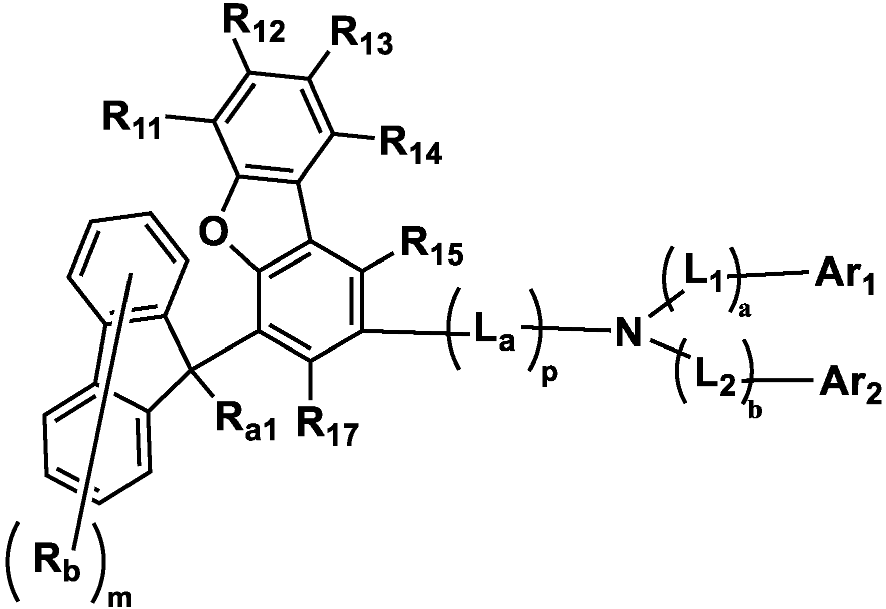

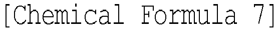

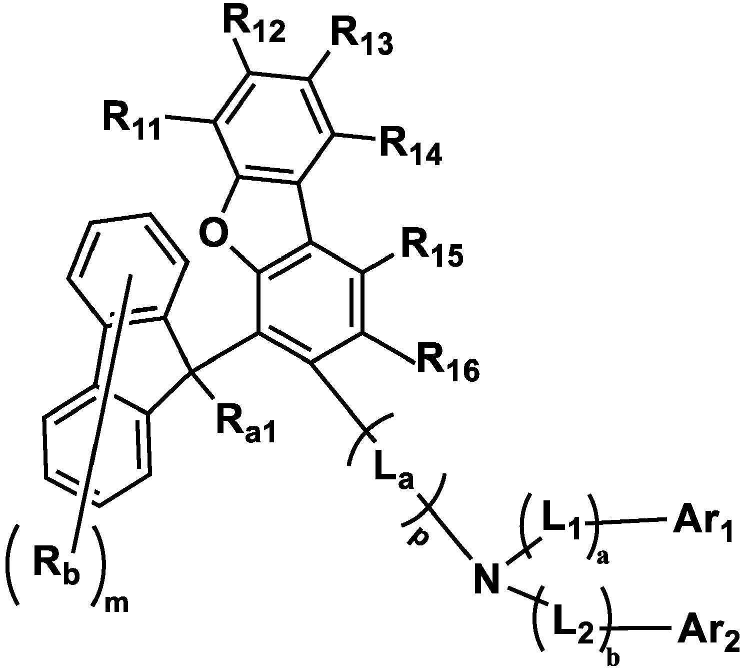

In Chemical Formulae 2 to 7,
Rₐ₁ is a substituted or unsubstituted aryl group having 6 to 60 carbon atoms,
L₁ and L₂ are the same as or different from each other, and each independently a direct bond; a substituted or unsubstituted arylene group having 6 to 60 carbon atoms or a substituted or unsubstituted heteroarylene group having 2 to 60 carbon atoms,
Ar₁ and Ar₂ are the same as or different from each other, and each independently a halogen group; a cyano group; a substituted or unsubstituted alkyl group having 1 to 60 carbon atoms; a substituted or unsubstituted aryl group having 6 to 60 carbon atoms or a substituted or unsubstituted heteroaryl group having 2 to 60 carbon atoms,
R₁₁ to R₁₇ are the same as or different from each other, and each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms, and
a and b are 0 or 1, and Lₐ, p, R_{b} and m have the same definitions as in Chemical Formula 1.

In the compounds represented by Chemical Formulae 2 to 7 corresponding to cases when Rₐ of Chemical Formula 1 is an aryl group, pi-pi stacking of the aromatic ring may be more suppressed. Accordingly, using the compounds represented by Chemical Formulae 2 to 7 in an organic light emitting device may lower a driving voltage of the organic light emitting device. In other words, it is more effective in preventing a problem of device property decline that may cause from a high driving voltage of the organic light emitting device.

In one embodiment of the present application, Chemical Formula 1 may be represented by any one of the following Chemical Formulae 8 to 14.

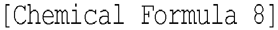

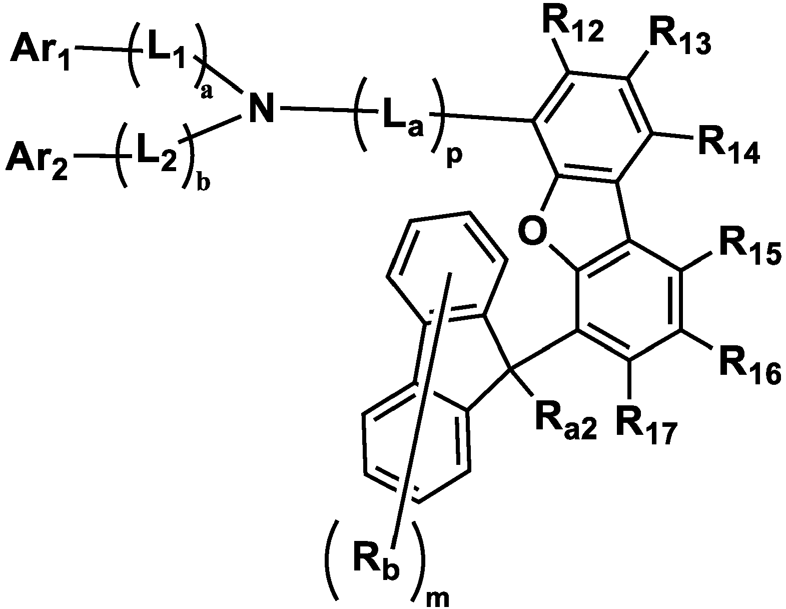

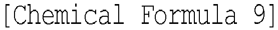

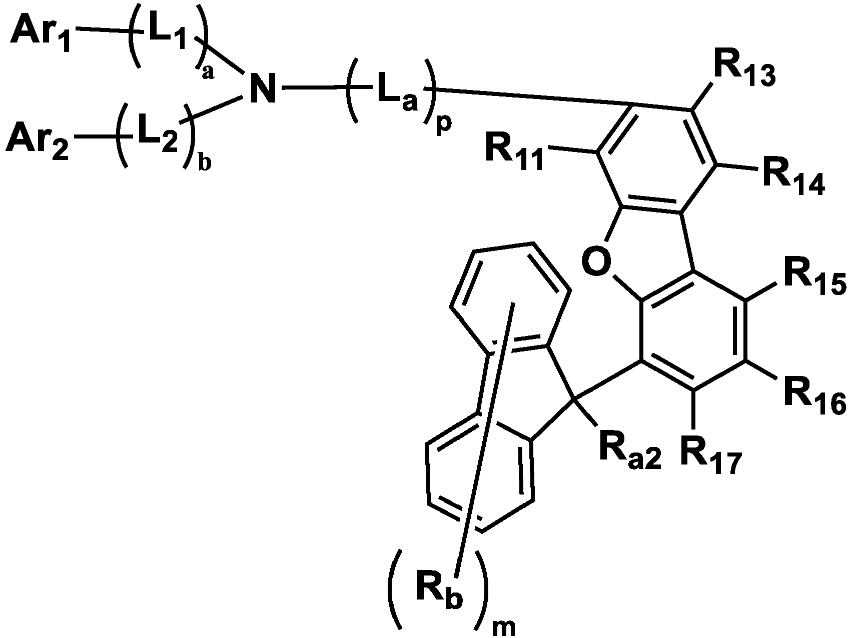

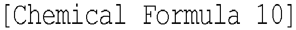

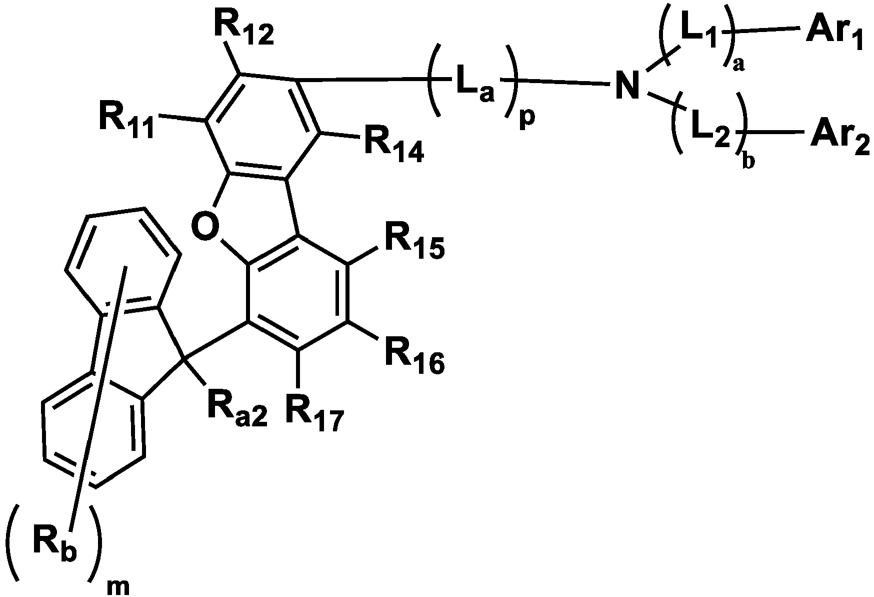

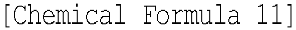

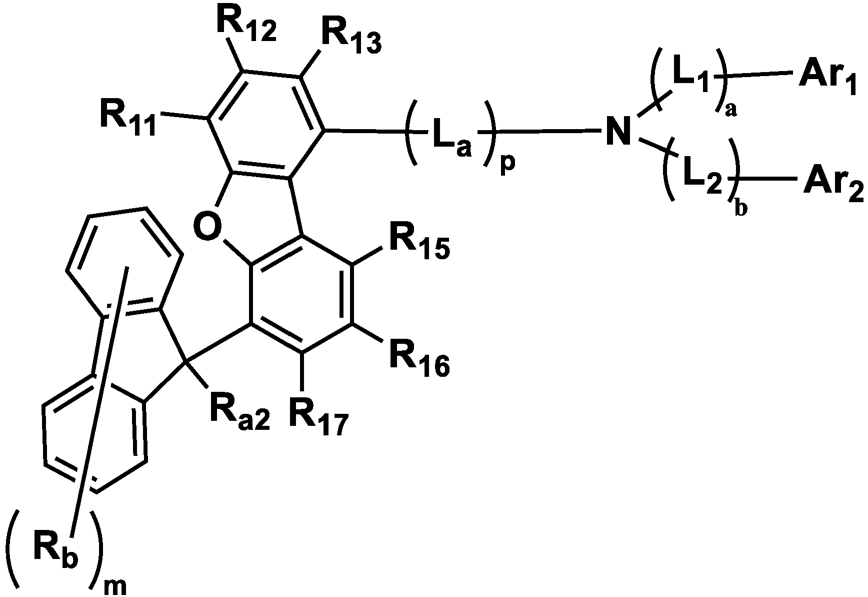

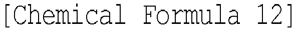

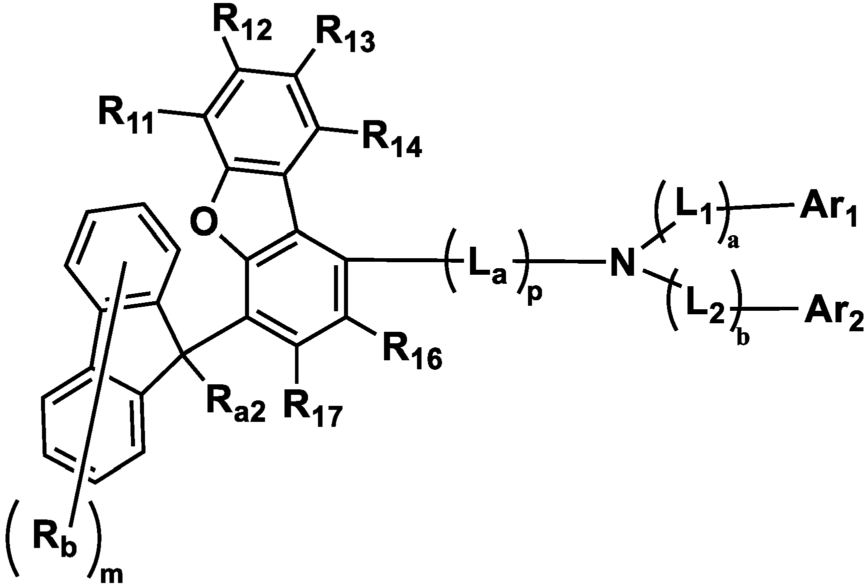

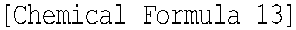

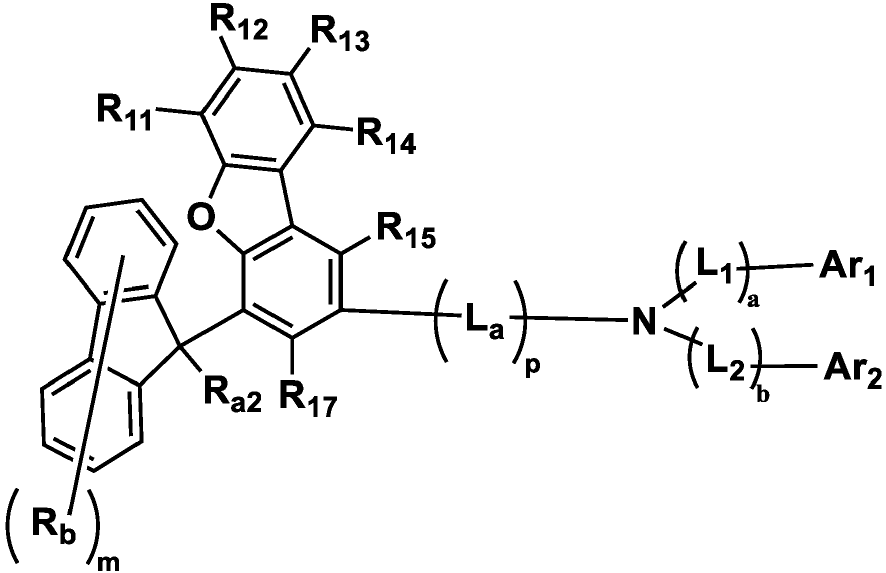

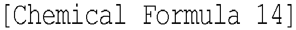

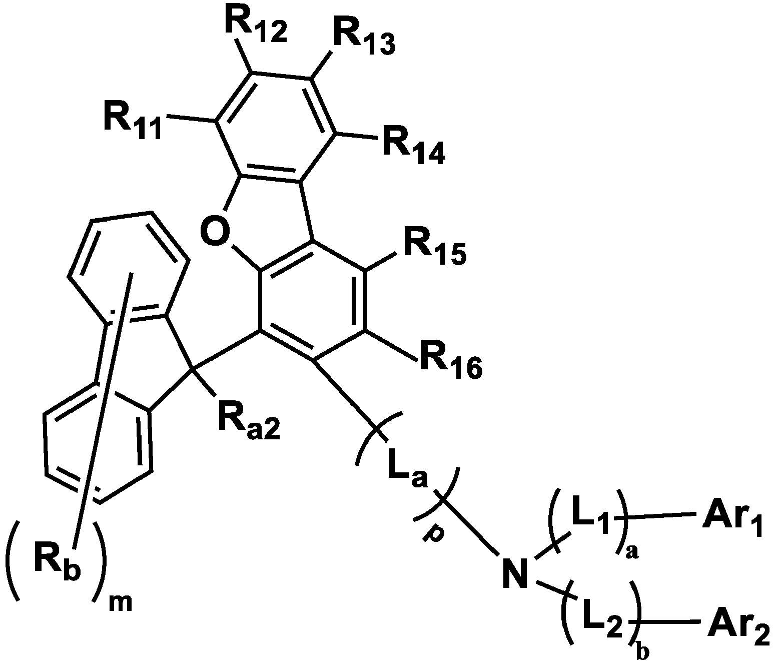

In Chemical Formulae 8 to 14,
Rₐ₂ is a halogen group; a cyano group; or a substituted or unsubstituted alkyl group having 1 to 60 carbon atoms, and L₁ and L₂ are the same as or different from each other and each independently a direct bond; a substituted or unsubstituted arylene group having 6 to 60 carbon atoms or a substituted or unsubstituted heteroarylene group having 2 to 60 carbon atoms,
Ar₁ and Ar₂ are the same as or different from each other, and each independently a halogen group; a cyano group; a substituted or unsubstituted alkyl group having 1 to 60 carbon atoms; a substituted or unsubstituted aryl group having 6 to 60 carbon atoms or a substituted or unsubstituted heteroaryl group having 2 to 60 carbon atoms,
R₁₁ to R₁₇ are the same as or different from each other, and each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms, and
a and b are 0 or 1, and Lₐ, p, R_{b} and m have the same definitions as in Chemical Formula 1.

When using the compounds represented by Chemical Formulae 8 to 14 corresponding to cases when Rₐ of Chemical Formula 1 is a halogen group; a cyano group; or a substituted or unsubstituted alkyl group having 1 to 60 carbon atoms instead of an aryl group in an organic light emitting device, a higher T1 value is obtained due to steric hindrance occurring from the structures of the compounds, and a device with more superior driving and efficiency may be obtained. Herein, the T1 value means an energy level value in a triplet state.

In one embodiment of the present application, L₁ and L₂ of Chemical Formulae 2 to 14 are the same as or different from each other, and may be each independently a direct bond; a substituted or unsubstituted arylene group having 6 to 60 carbon atoms or a substituted or unsubstituted heteroarylene group having 2 to 60 carbon atoms.

In one embodiment of the present application, L₁ and L₂ of Chemical Formulae 2 to 14 are the same as or different from each other, and may be each independently a direct bond; a substituted or unsubstituted arylene group having 6 to 40 carbon atoms or a substituted or unsubstituted heteroarylene group having 2 to 40 carbon atoms.

In one embodiment of the present application, L₁ and L₂ are the same as or different from each other, and may be each independently a direct bond; or a substituted or unsubstituted arylene group having 6 to 40 carbon atoms.

In one embodiment of the present application, L₁ and L₂ are the same as or different from each other, and may be each independently a direct bond; or a substituted or unsubstituted arylene group having 6 to 20 carbon atoms.

In one embodiment of the present application, L₁ and L₂ are the same as or different from each other, and may be each independently a direct bond; or a substituted or unsubstituted phenylene group.

In one embodiment of the present application, L₁ is a direct bond.

In one embodiment of the present application, L₁ is a phenylene group.

In one embodiment of the present application, L₂ is a direct bond.

In one embodiment of the present application, L₂ is a phenylene group.

In one embodiment of the present application, Ar₁ and Ar₂ of Chemical Formulae 2 to 14 are the same as or different from each other, and may be each independently a halogen group; a cyano group; a substituted or unsubstituted alkyl group having 1 to 60 carbon atoms; a substituted or unsubstituted aryl group having 6 to 60 carbon atoms; or a substituted or unsubstituted heteroaryl group having 2 to 60 carbon atoms.

In one embodiment of the present application, Ar₁ and Ar₂ are the same as or different from each other, and may be each independently a substituted or unsubstituted aryl group having 6 to 60 carbon atoms; or a substituted or unsubstituted heteroaryl group having 2 to 60 carbon atoms.

In one embodiment of the present application, Ar₁ and Ar₂ are the same as or different from each other, and may be each independently a substituted or unsubstituted aryl group having 6 to 40 carbon atoms; or a substituted or unsubstituted heteroaryl group having 2 to 40 carbon atoms.

In one embodiment of the present application, Ar₁ and Ar₂ are the same as or different from each other, and may be each independently a substituted or unsubstituted phenyl group; a substituted or unsubstituted biphenyl group; a substituted or unsubstituted terphenyl group; a substituted or unsubstituted naphthyl group; a fluorenyl group unsubstituted or substituted with one or more selected from the group consisting of deuterium, an alkyl group having 1 to 10 carbon atoms and an aryl group having 6 to 10 carbon atoms; a substituted or unsubstituted dibenzofuran group; a substituted or unsubstituted dibenzothiophene group; or a substituted or unsubstituted spirobifluorenyl group.

In one embodiment of the present application, Ar₁ and Ar₂ are the same as or different from each other, and may be each independently a substituted or unsubstituted phenyl group; a substituted or unsubstituted biphenyl group; a substituted or unsubstituted terphenyl group; a substituted or unsubstituted naphthyl group; a fluorenyl group unsubstituted or substituted with one or more selected from the group consisting of deuterium, a methyl group and a phenyl group; a substituted or unsubstituted dibenzofuran group; a substituted or unsubstituted dibenzothiophene group; or a substituted or unsubstituted spirobifluorenyl group.

In one embodiment of the present application, Ar₁ and Ar₂ are the same as or different from each other, and may be each independently a phenyl group unsubstituted or substituted with deuterium; a biphenyl group unsubstituted or substituted with deuterium; a terphenyl group unsubstituted or substituted with deuterium; a naphthyl group unsubstituted or substituted with deuterium; a fluorenyl group unsubstituted or substituted with one or more selected from the group consisting of deuterium, a methyl group unsubstituted or substituted with deuterium and a phenyl group unsubstituted or substituted with deuterium; a dibenzofuran group unsubstituted or substituted with deuterium; a dibenzothiophene group unsubstituted or substituted with deuterium; or a spirobifluorenyl group unsubstituted or substituted with deuterium.

According to one embodiment of the present application, Chemical Formula 1 may be represented by any one of the following compounds, but is not limited thereto.

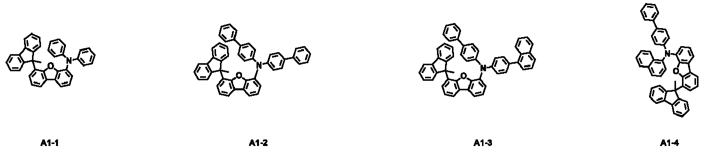

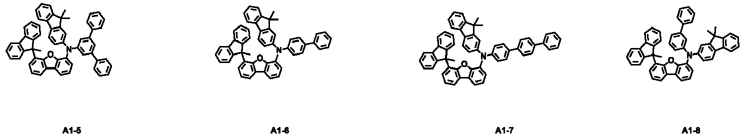

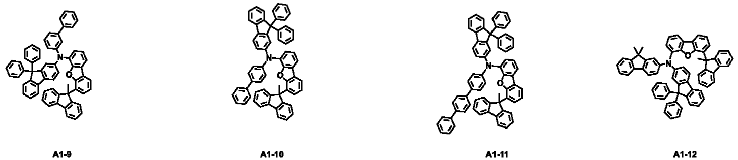

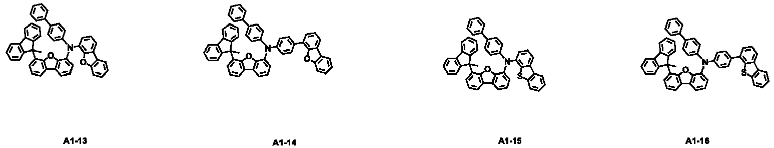

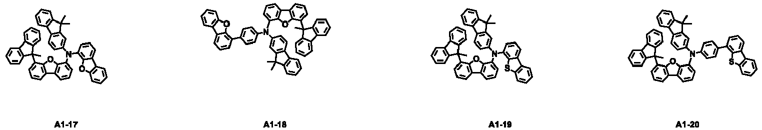

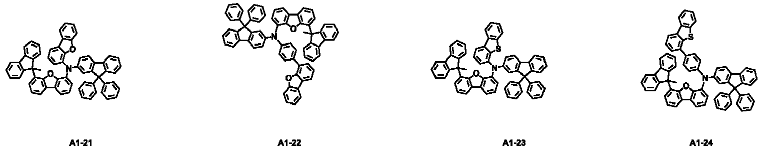

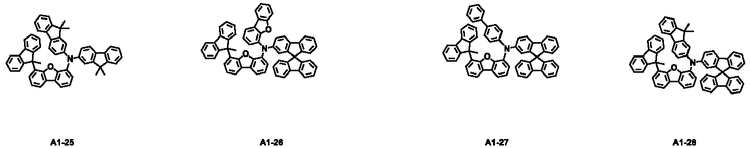

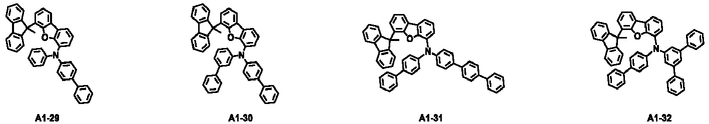

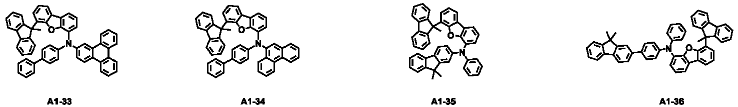

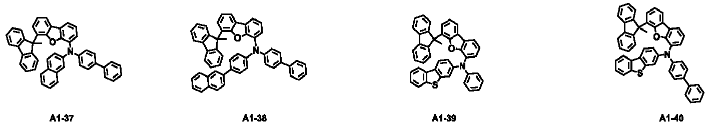

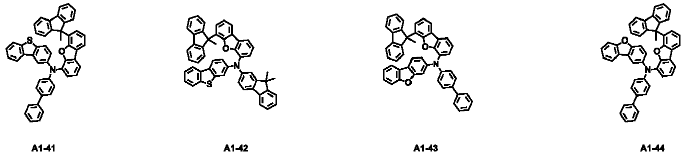

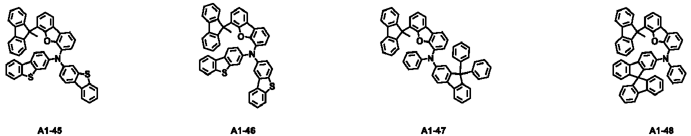

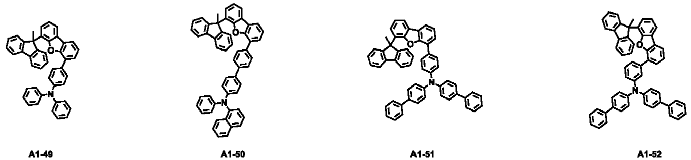

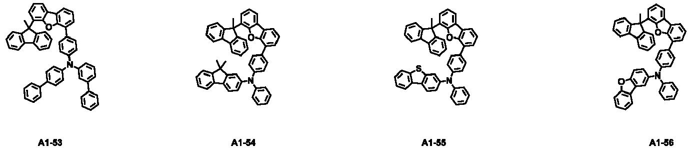

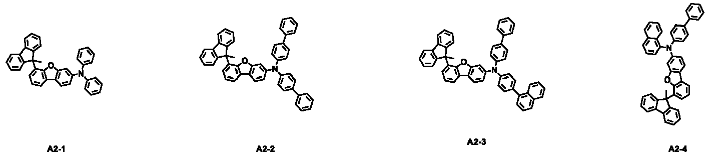

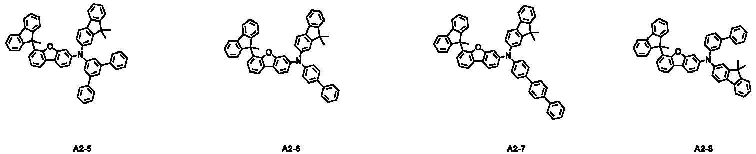

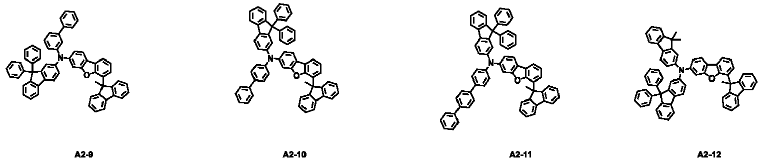

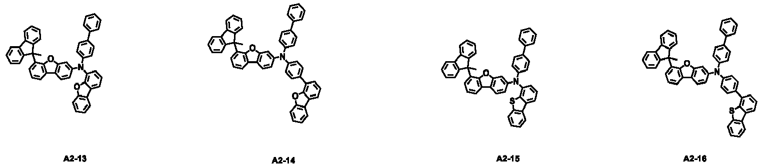

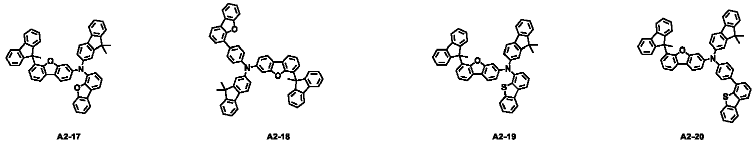

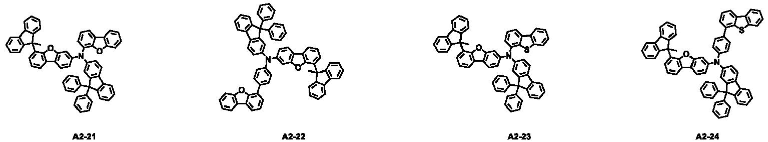

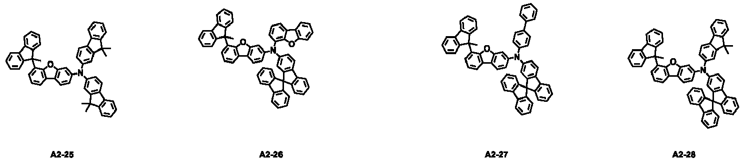

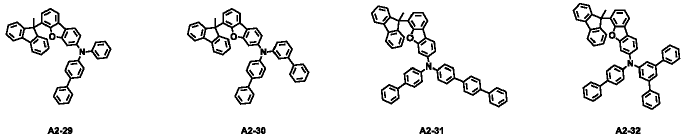

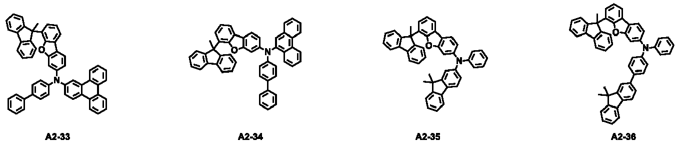

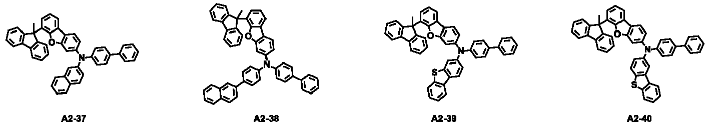

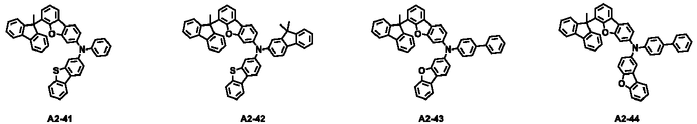

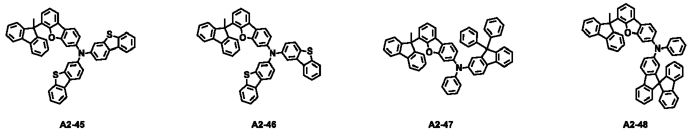

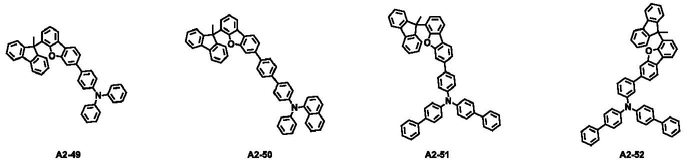

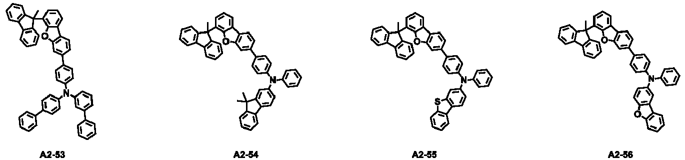

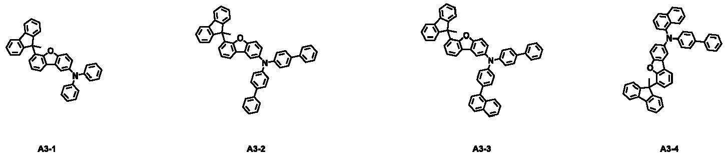

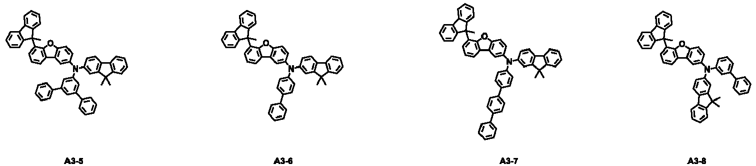

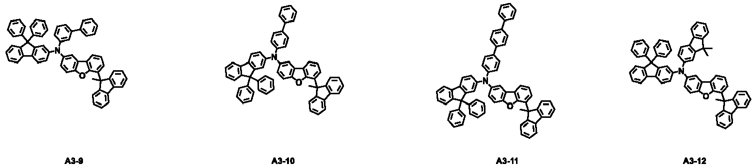

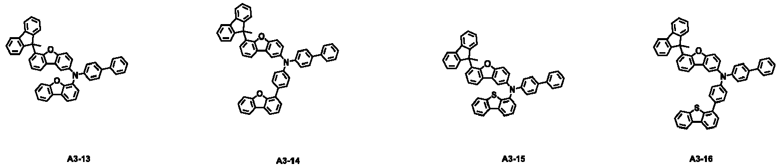

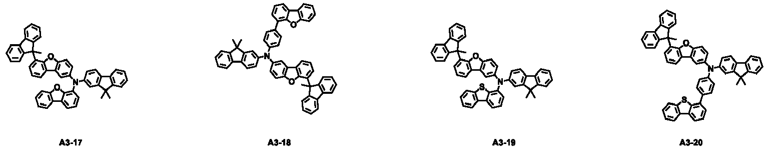

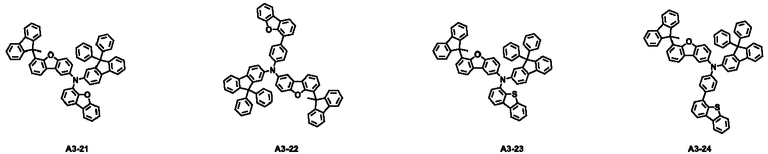

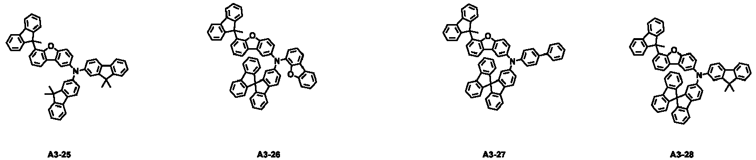

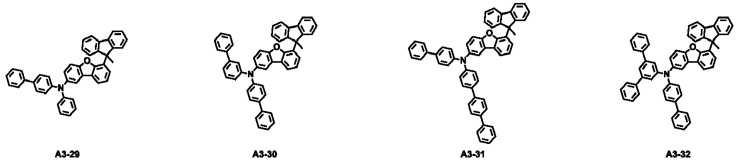

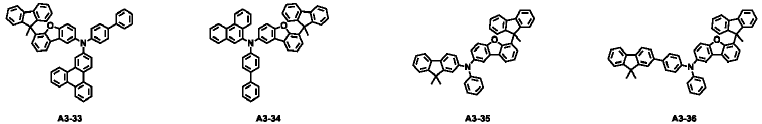

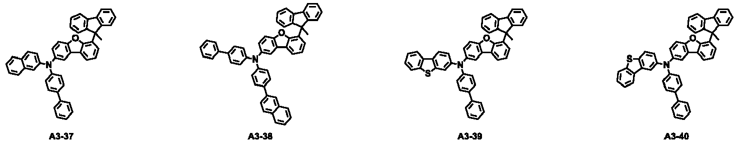

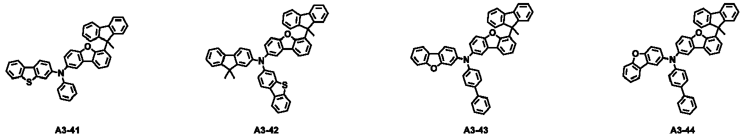

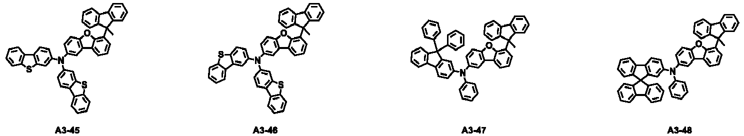

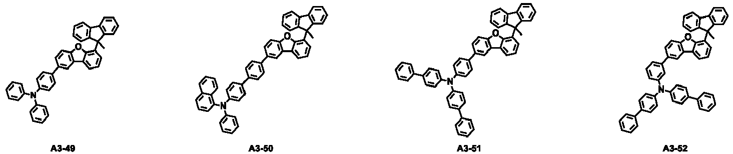

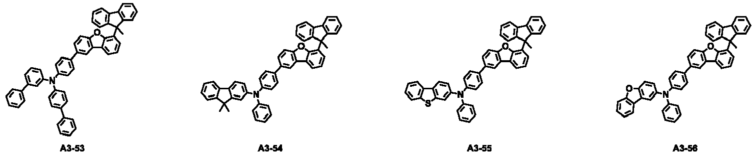

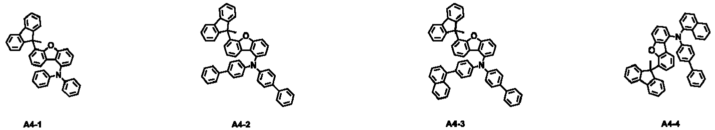

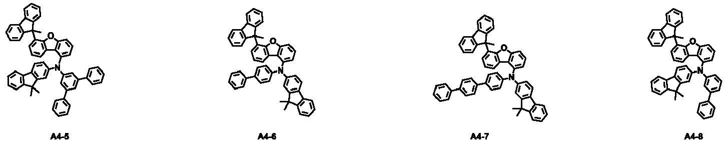

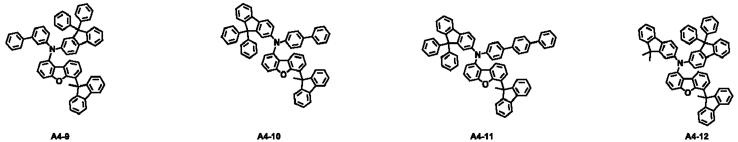

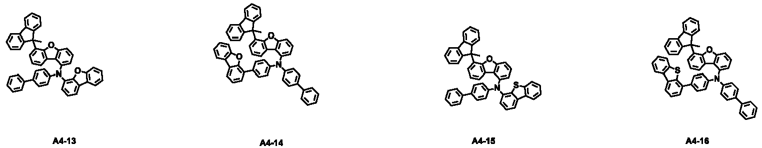

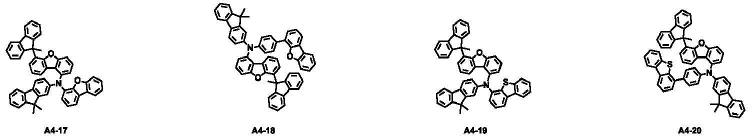

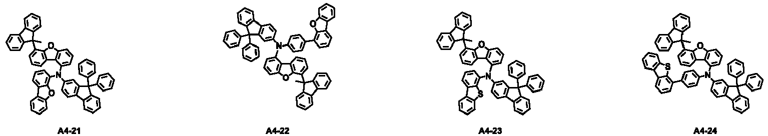

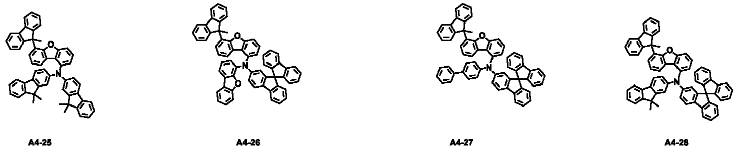

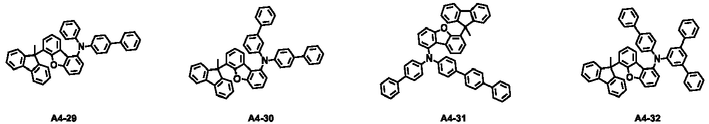

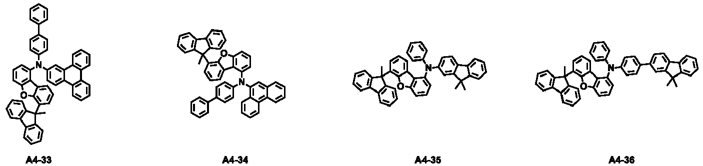

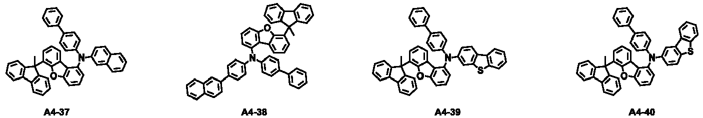

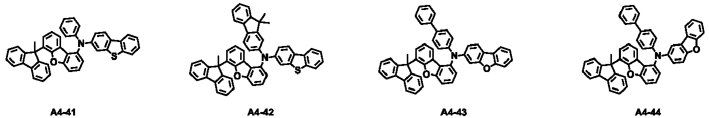

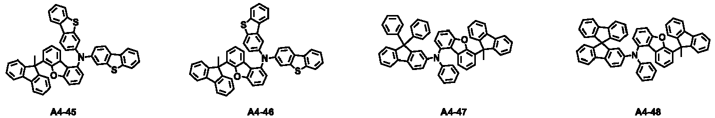

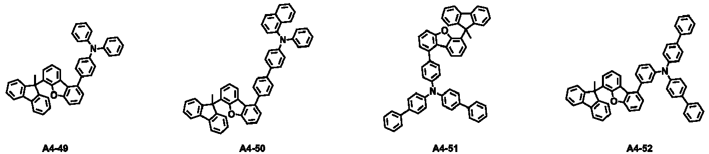

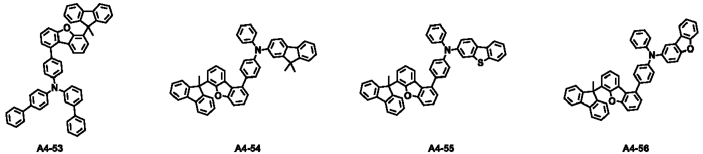

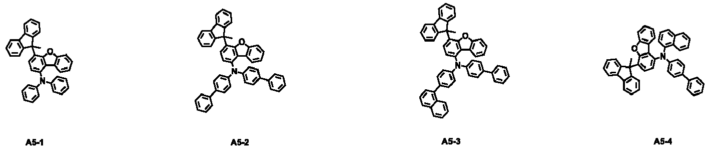

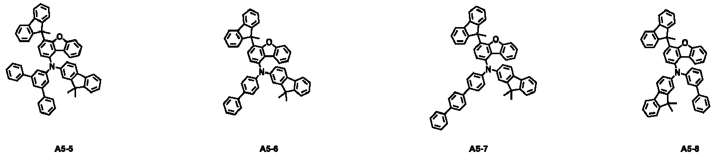

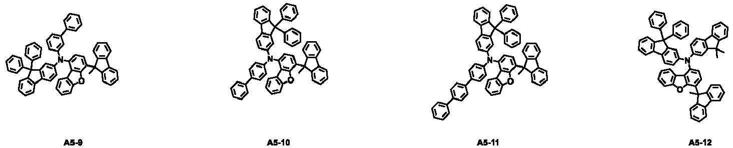

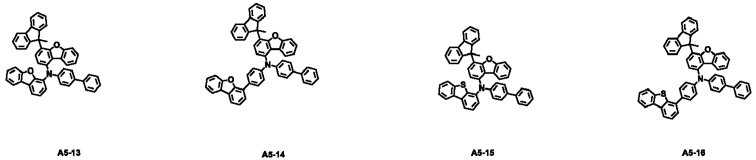

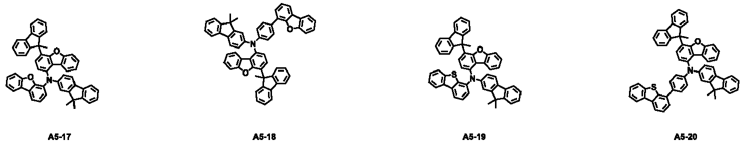

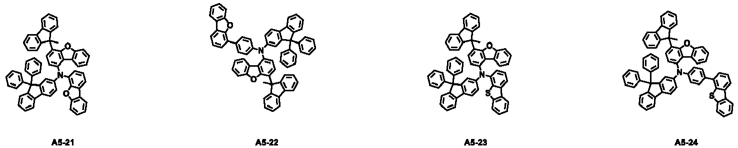

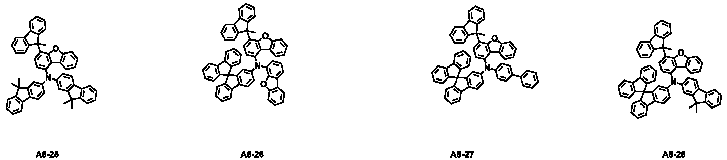

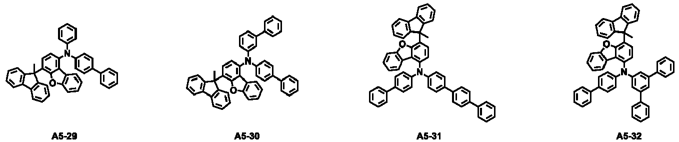

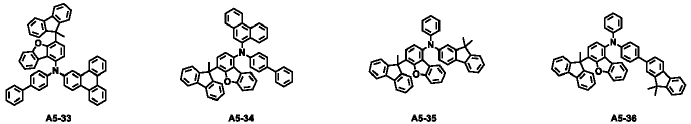

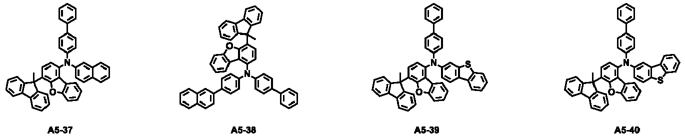

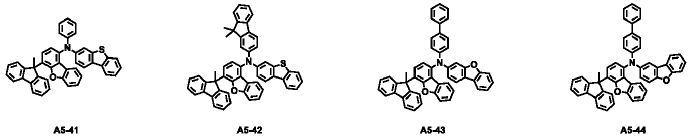

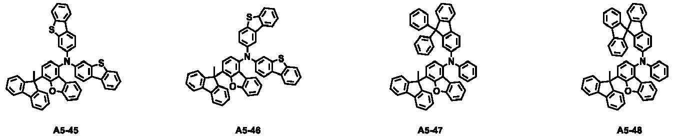

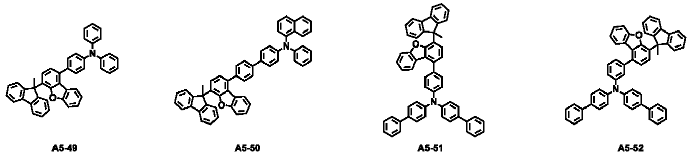

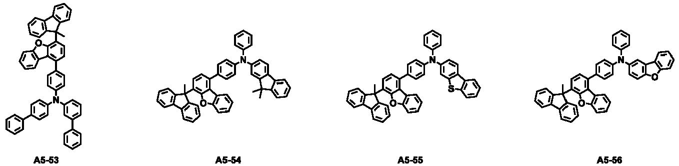

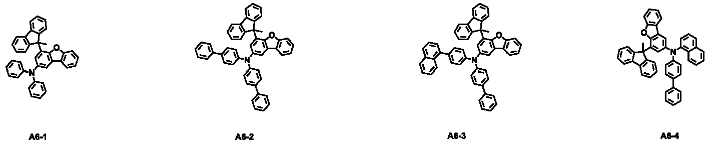

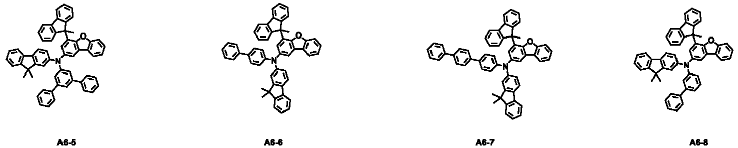

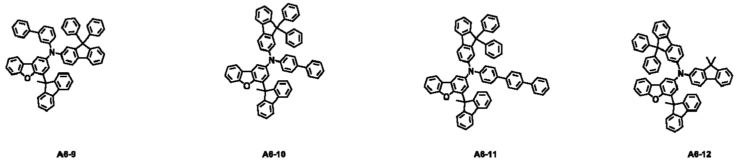

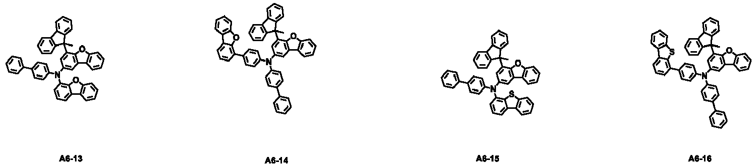

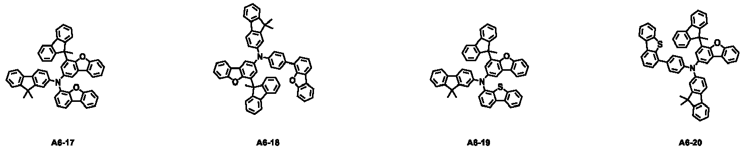

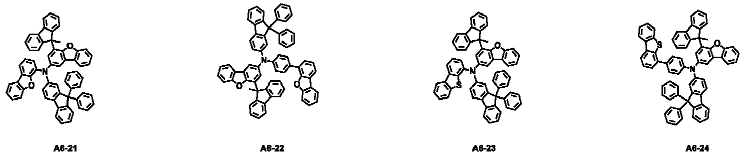

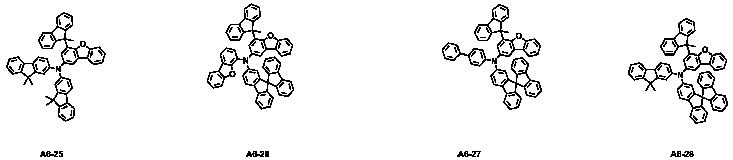

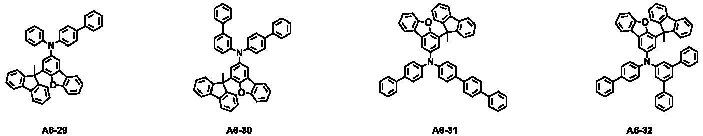

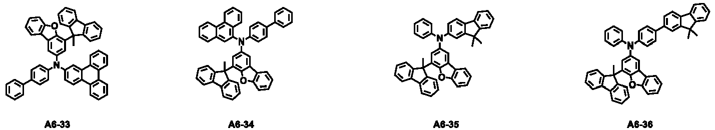

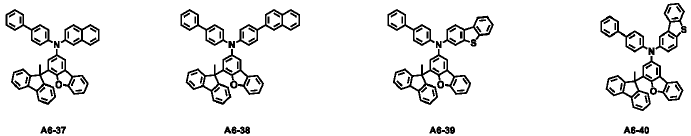

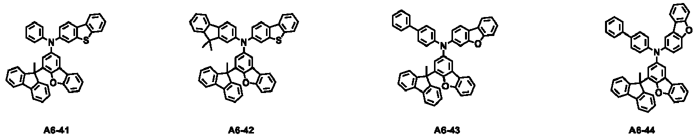

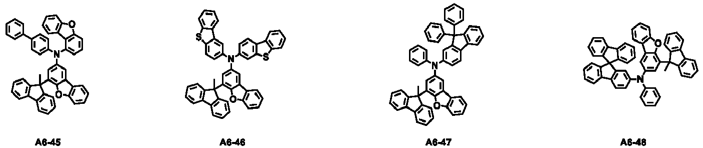

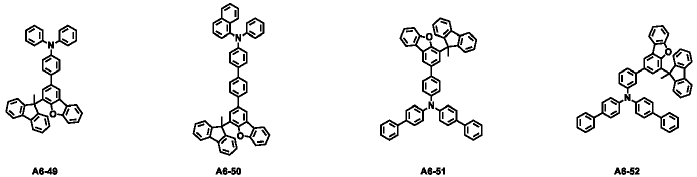

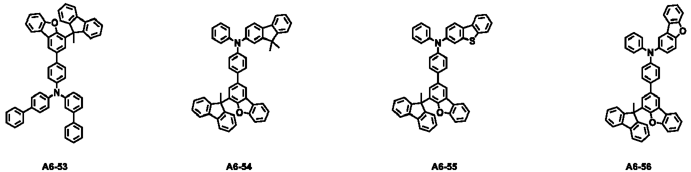

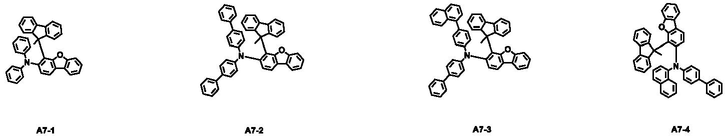

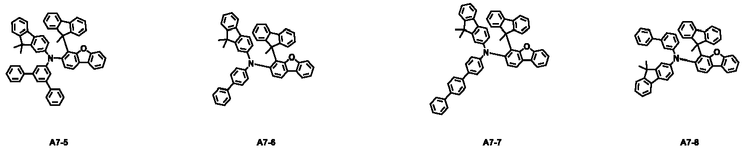

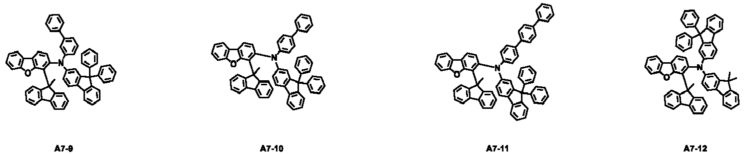

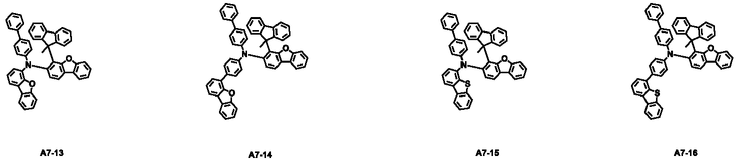

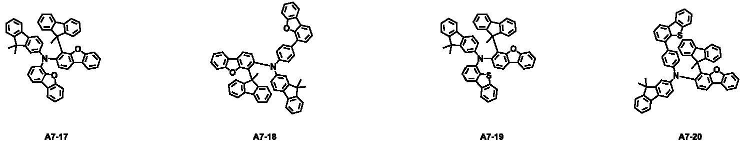

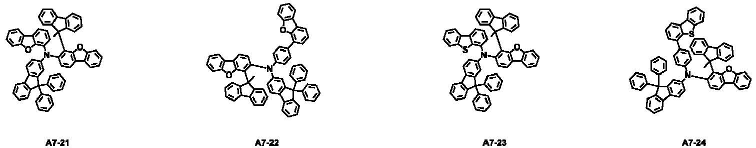

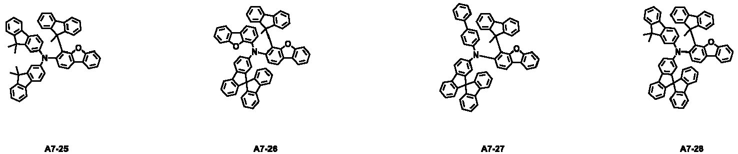

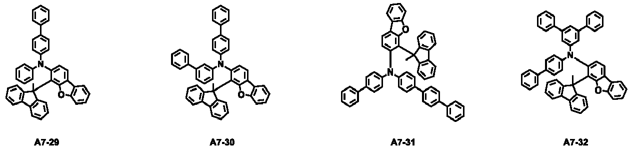

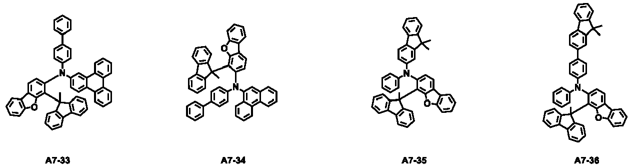

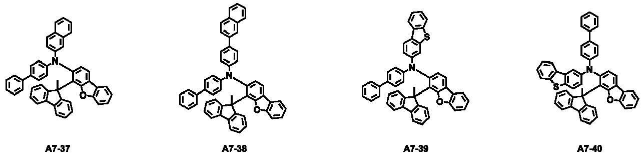

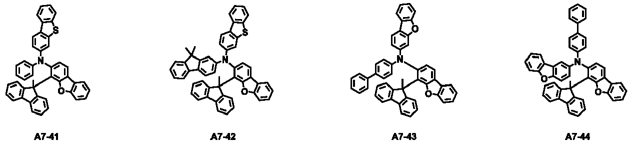

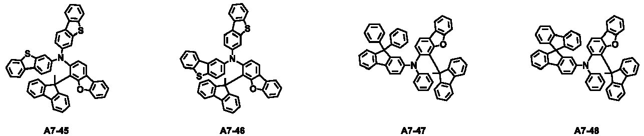

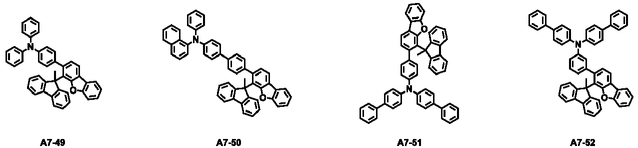

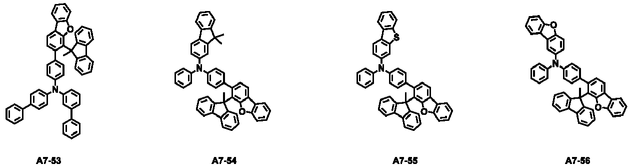

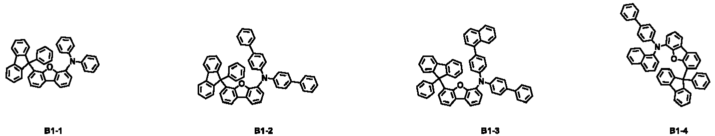

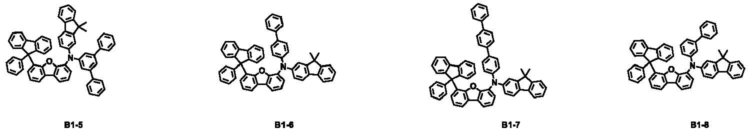

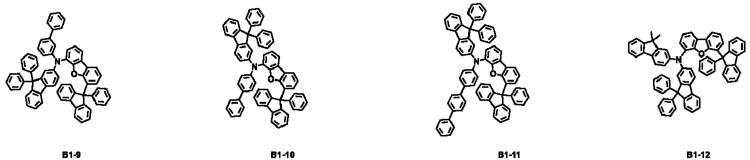

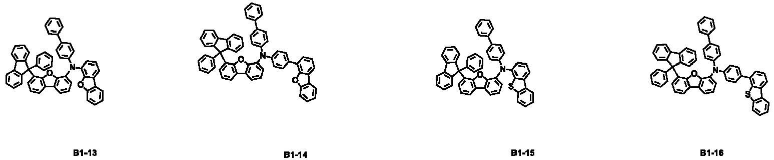

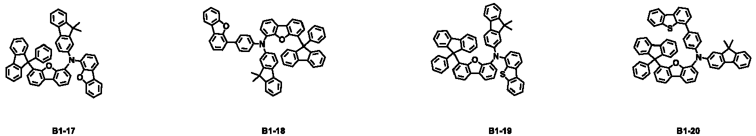

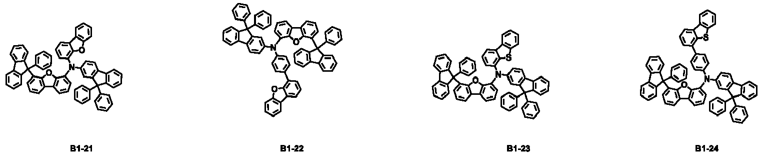

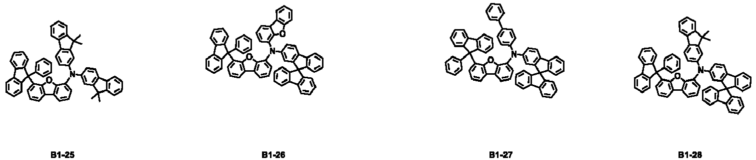

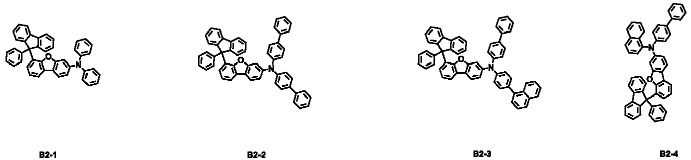

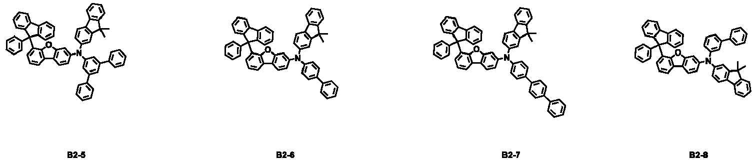

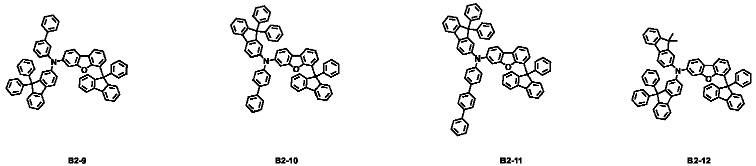

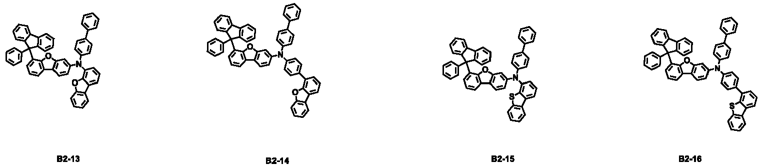

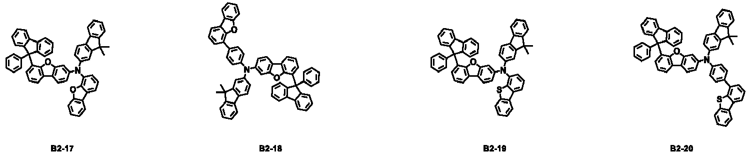

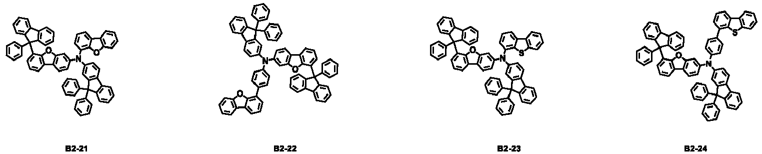

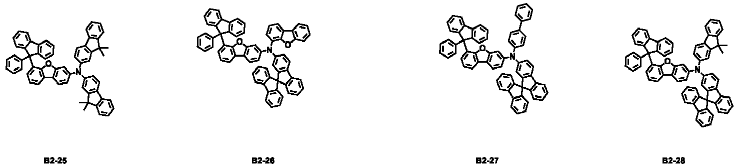

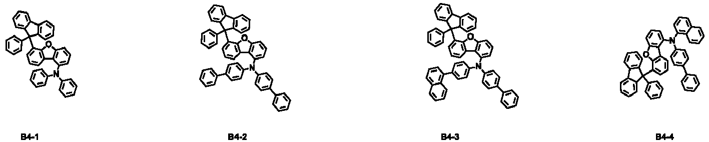

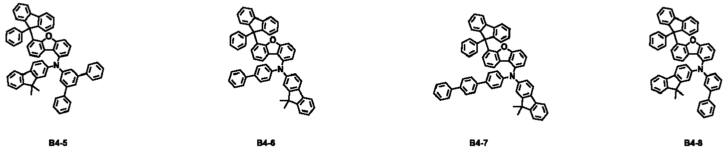

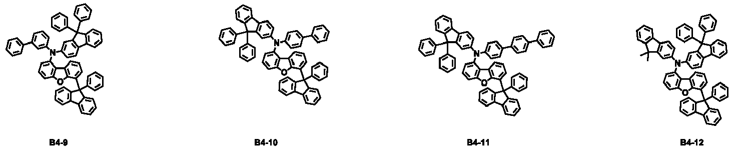

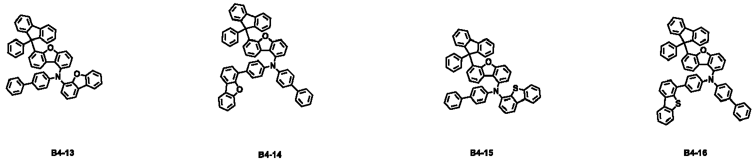

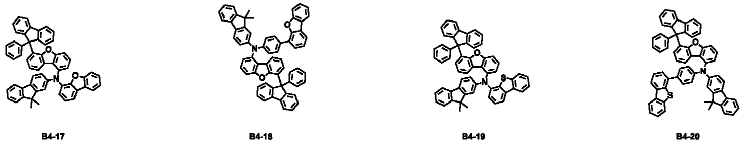

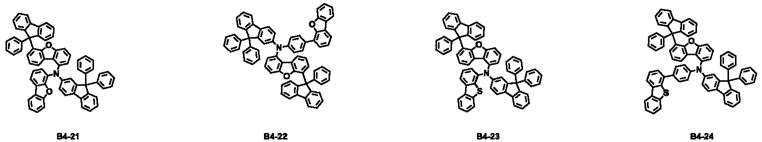

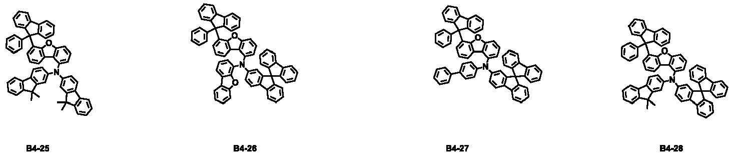

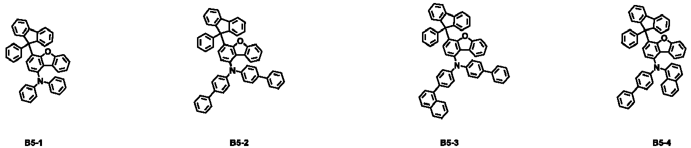

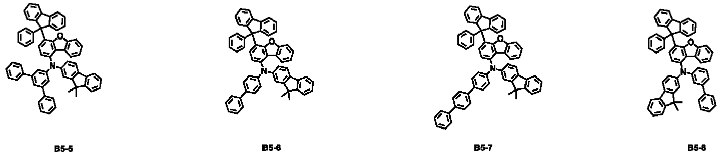

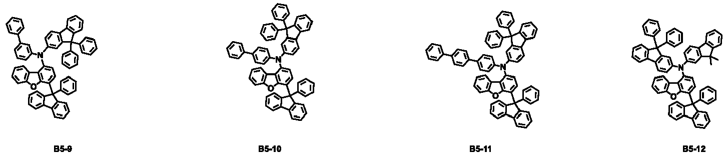

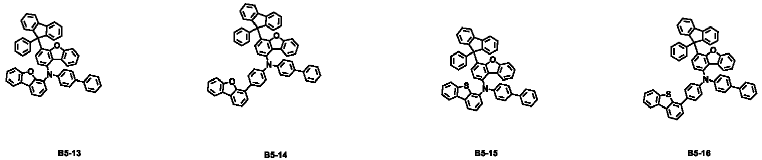

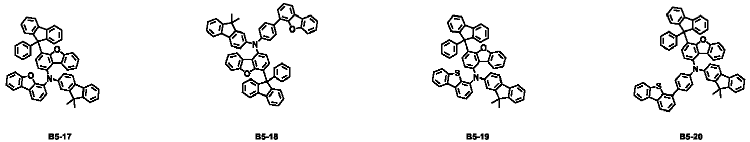

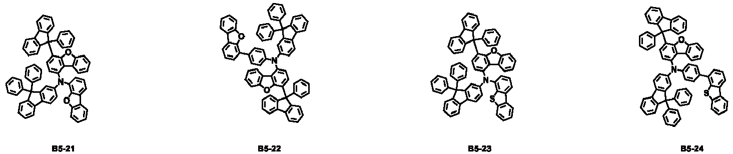

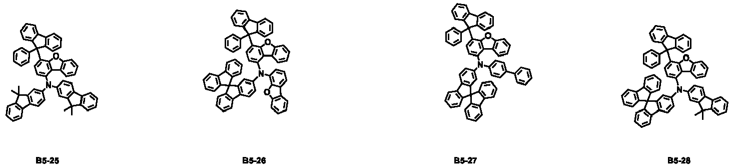

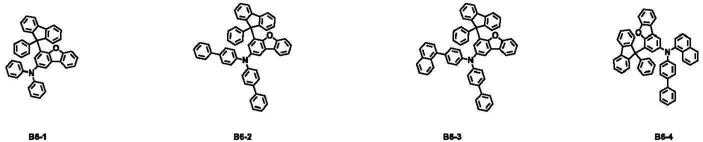

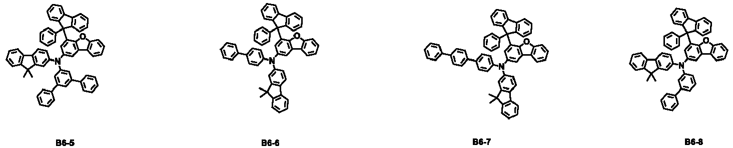

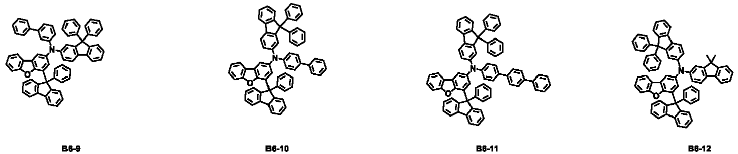

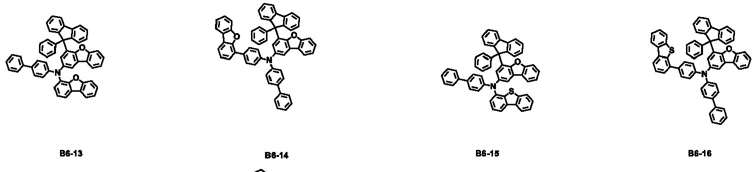

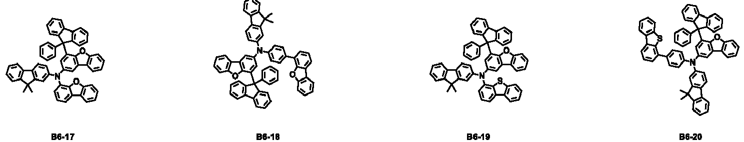

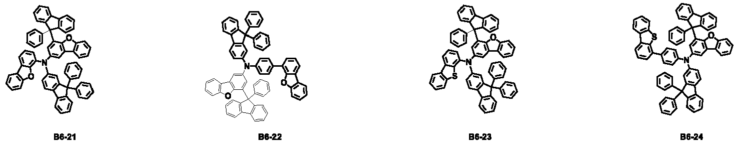

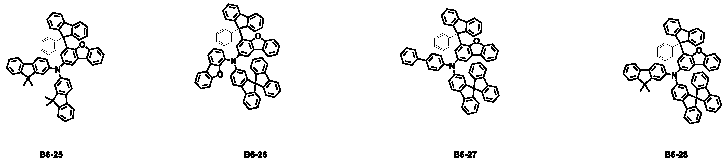

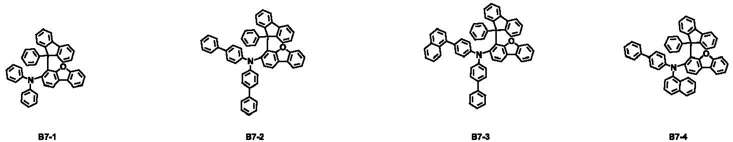

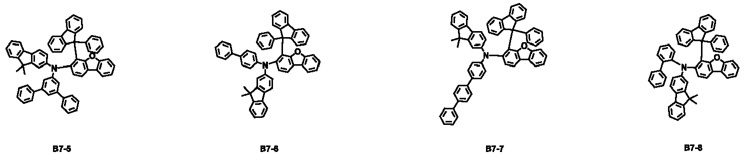

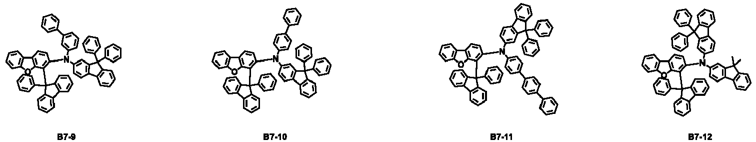

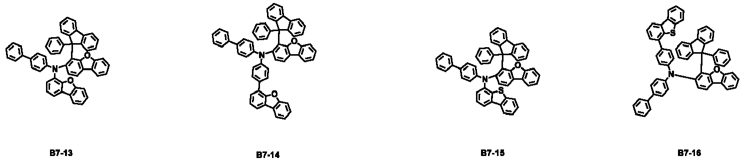

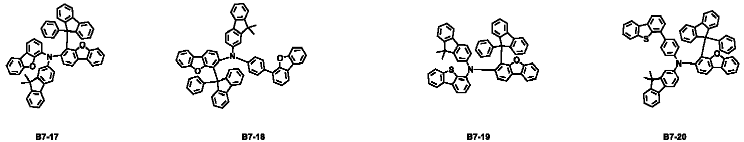

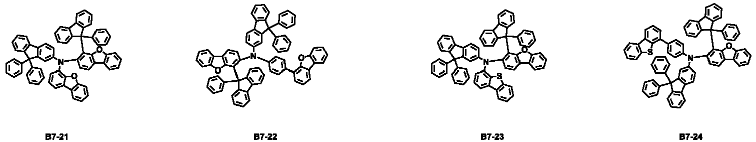

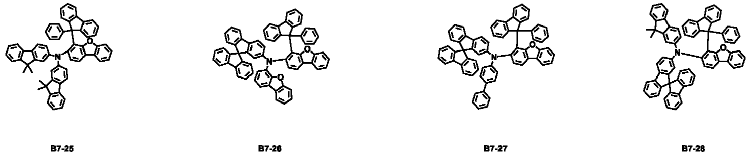

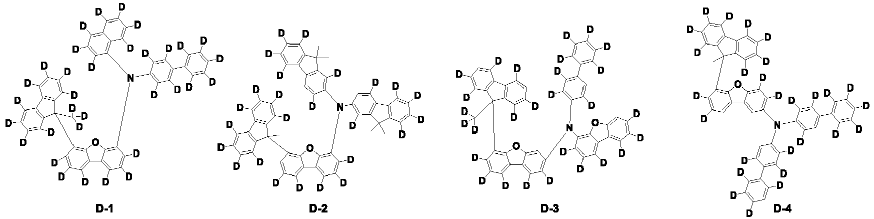

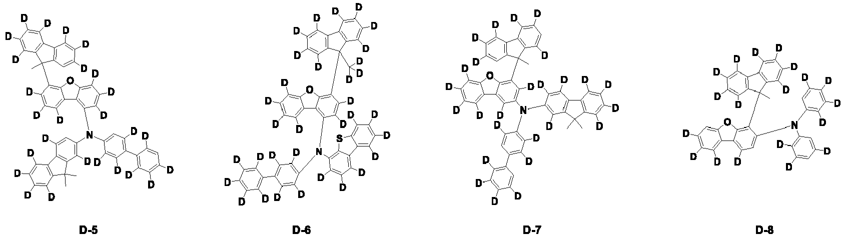

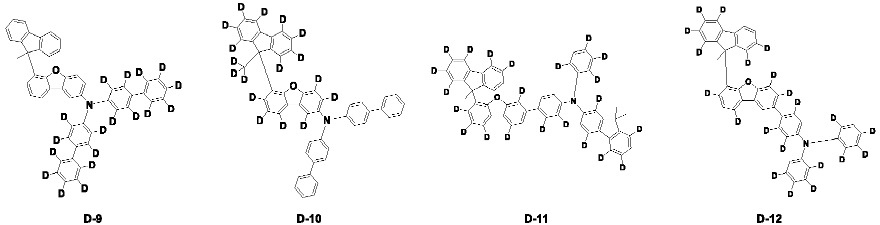

In addition, by introducing various substituents to the structure of Chemical Formulae 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

Meanwhile, the hetero-cyclic compound has a high glass transition temperature (Tg), and has excellent thermal stability. Such an increase in the thermal stability becomes an important factor providing driving stability to a device.

The hetero-cyclic compound according to one embodiment of the present application may be prepared using a multi-step chemical reaction. Some intermediate compounds are prepared first, and from the intermediate compounds, the hetero-cyclic compound of Chemical Formula 1 may be prepared. More specifically, the hetero-cyclic compound according to one embodiment of the present application may be prepared based on preparation examples to describe later.

Another embodiment of the present application provides an organic light emitting device comprising the hetero-cyclic compound represented by Chemical Formula 1. The "organic light emitting device" may be expressed in terms such as an "organic light emitting diode", an "OLED", an "OLED device" and an "organic electroluminescent device".

The hetero-cyclic compound may be formed into an organic material layer using a solution coating method as well as a vacuum deposition method when manufacturing the organic light emitting device. Herein, the solution coating method means spin coating, dip coating, inkjet printing, screen printing, a spray method, roll coating and the like, but is not limited thereto.

Specifically, the organic light emitting device according to one embodiment of the present application comprises an anode, a cathode, and one or more organic material layers provided between the anode and the cathode, and one or more layers of the organic material layers comprise the hetero-cyclic compound represented by Chemical Formula 1. When comprising the hetero-cyclic compound represented by Chemical Formula 1 in the organic material layer, the organic light emitting device has superior light emission efficiency and lifetime.

In addition, the organic material layer comprises a hole transfer layer, and the hole transfer layer comprises the hetero-cyclic compound represented by Chemical Formula 1. When comprising the hetero-cyclic compound represented by Chemical Formula 1 in the hole transfer layer among the organic material layers, the organic light emitting device has more superior light emission efficiency and lifetime.

In the organic light emitting device of the present application, the organic material layer comprises an electron blocking layer, and the electron blocking layer may comprise the hetero-cyclic compound as a host material of a light emitting material.

The organic light emitting device of the present disclosure may further comprise one, two or more layers selected from the group consisting of a light emitting layer, a hole injection layer, a hole transfer layer, an electron blocking layer, an electron injection layer, an electron transfer layer, a hole auxiliary layer and a hole blocking layer.

In the organic light emitting device of the present application, the light emitting layer may comprise two or more host materials.

In the organic light emitting device of the present application, two or more host materials may be pre-mixed and used as the light emitting layer. The pre-mixing means placing and mixing two or more host materials of the light emitting layer in one source of supply before depositing on the organic material layer.

In the organic light emitting device of the present application, the light emitting layer may comprise two or more host materials, and the two or more host materials may each comprise one or more p-type host materials and n-type host materials.

The organic light emitting device according to one embodiment of the present application may be manufactured using common organic light emitting device manufacturing methods and materials except that the organic material layer is formed using the hetero-cyclic compound described above.
FIG. 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.
FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

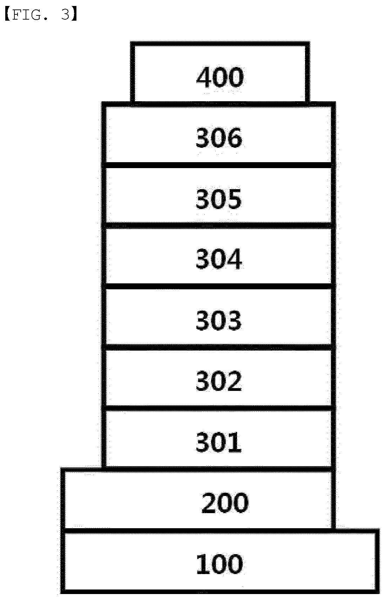

FIG. 3 illustrates a case of the organic material layer being a multilayer. The organic light emitting device according to FIG. 3 includes a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further added.

In the organic light emitting device according to one embodiment of the present application, materials other than the hetero-cyclic compound of Chemical Formula 1 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material comprise metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDT), polypyrrole and polyaniline, and the like, but are not limited thereto.

As the cathode material, materials having relatively small work function may be used, and metals, metal oxides, conductive polymers or the like may be used. Specific examples of the cathode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrenesulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

The organic light emitting device according to one embodiment of the present application may be a top-emission type, a bottom-emission type or a dual-emission type depending on the materials used.

The hetero-cyclic compound according to one embodiment of the present application may also be used in an organic electronic device comprising an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example 1> Preparation of Compound A1-2

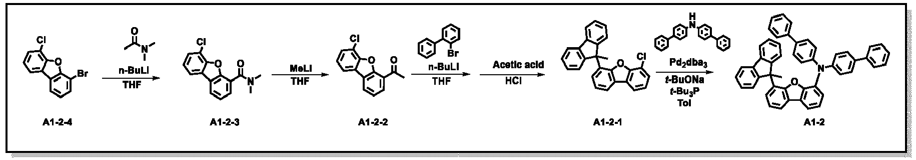

### 1) Preparation of Compound A1-2-3

4-Bromo-6-chlorodibenzo[b,d]furan (A) (50 g, 0.178 mol, 1.0 eq.) was introduced to tetrahydrofuran (hereinafter, THF) (750 ml), and, after substituted with N₂, n-BuLi (12.5 g, 0.195 mol, 1.1 eq.) was added dropwise thereto at 0°C, and the mixture was stirred for 30 minutes. After that, N,N-dimethylacetamide (17 g, 0.195 mol, 1.1 eq.) was added dropwise thereto, and the result was stirred for 3 hours (h) at room temperature. Then, the reaction was terminated by introducing water (distilled water) thereto, and the result was extracted using methyl chloride (hereinafter, MC) and water. After that, moisture was removed with anhydrous MgSO₄. After removing the moisture, the result was separated using a silica gel column to obtain Compound A1-2-3 (41 g) in a 84% yield.

### 2) Preparation of Compound A1-2-2

Compound A1-2-3 (41 g, 0.15 mol, 1 eq.) was introduced to THF (600 ml), and, after substituted with N₂, the mixture was stirred at 0°C. MeLi (B) (3.7 g, 0.17 mol, 1.1 eq.) was slowly added dropwise thereto, and the result was stirred for 2 h at 0°C. Then, the reaction was terminated by introducing water (distilled water) thereto, and the result was extracted using MC and water. After that, moisture was removed with anhydrous MgSO₄. After removing the moisture, the result was separated using a silica gel column to obtain Compound A1-2-2 (20 g) in a 54% yield.

### 3) Preparation of Compound A1-2-1

2-Bromo-1,1'-biphenyl (21 g, 0.09 mol, 1.1 eq.) was introduced to THF (40 ml), and, after substituted with N₂, n-BuLi 2.5 M (36 ml, 0.09 mol, 1.1 eq.) was added dropwise thereto at 0°C, and the mixture was stirred for 1 hour. Compound A1-2-1 (20 g, 0.08 mol, 1 eq.) dissolved in THF (600 ml) was slowly added dropwise to the reaction material, and the result was stirred for 6 h at room temperature (RT). Then, the result was extracted using EA and water. After that, HCl (20 ml) and acetic acid (200 ml) were introduced thereto, and the result was stirred for 6 h at 110°C. After the reaction was finished, water was introduced thereto, and produced solids were filtered to obtain Compound A1-2-1 (20 g) in a 60% yield.

### 4) Preparation of Compound A1-2

Compound A1-2-1 (20 g, 0.05 mol, 1 eq.), N-phenyl-[1,1'-biphenyl]-4-amine (C) (19 g, 0.06 mol, 1.1 eq.), Nat-BuO (9.6 g, 0.1 mol, 2 eq.), Pd₂(dba)₃ (2.3 g, 0.0025 mol, 0.05 eq.) and t-Bu₃P (2.3 ml 0.005 mol, 0.1 eq.) were introduced to toluene (200 ml), and the mixture was stirred for 6 h at 110°C. Then, the reaction was terminated by introducing water (distilled water) thereto, and the result was extracted using MC and water. After that, moisture was removed with anhydrous MgSO₄. After removing the moisture, the result was separated using a silica gel column to obtain Compound A1-2 (25.6 g) in a 77% yield.

### 5) Preparation of Compounds of Table 1

Compounds of the following Table 1 were synthesized in the same manner as in Preparation Example 1 except that Intermediate A of the following Table 1 was used instead of 4-bromo-6-chlorodibenzo[b,d]furan (A), and Intermediates B and C of the following Table 1 were respectively used instead of MeLi (B) and N-phenyl-[1,1'-biphenyl]-4-amine (C).

**[Table 1]**

| Compoun d No. | Intermediate A | Intermed iate B | Intermediate C | Yiel d |
|---|---|---|---|---|
| A1-1 | 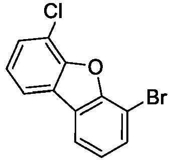 | MeLi | 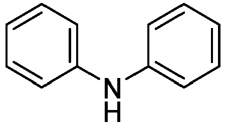 | 80% |
| A1-6 | 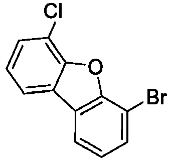 | MeLi | 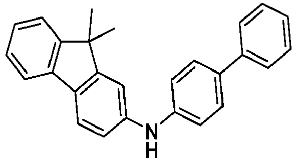 | 77% |
| A1-7 | 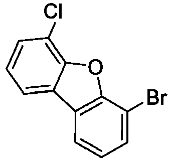 | MeLi | 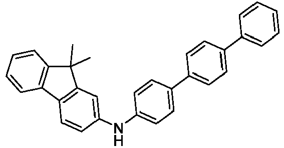 | 69% |
| A1-10 | 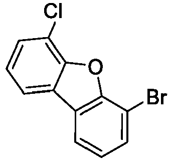 | MeLi | 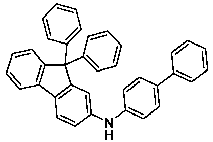 | 71% |
| A1-11 | 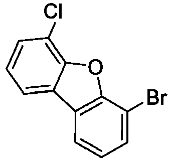 | MeLi | 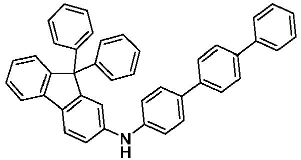 | 72% |
| A1-13 | 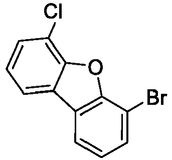 | MeLi | 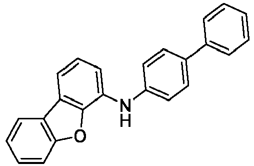 | 70% |
| A1-14 | 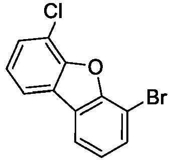 | MeLi | 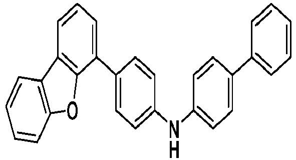 | 65% |
| A1-30 | 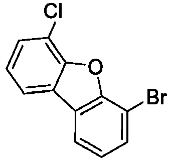 | MeLi | 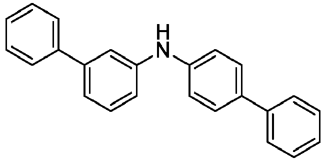 | 73% |
| A1-41 | 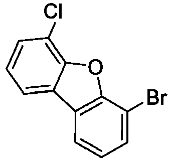 | MeLi | 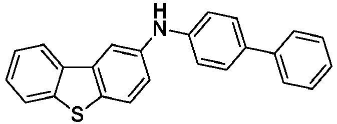 | 79% |
| A1-47 | 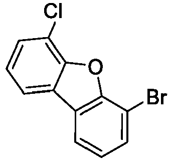 | MeLi | 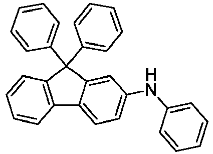 | 82% |
| A2-1 | 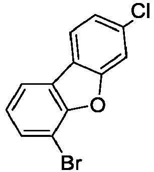 | MeLi | 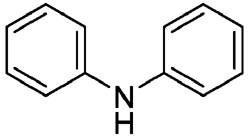 | 85% |
| A2-6 | 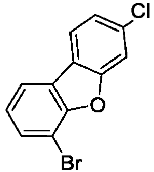 | MeLi | 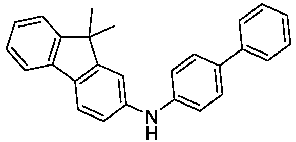 | 85% |
| A2-7 | 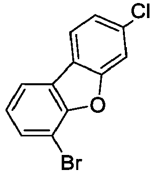 | MeLi | 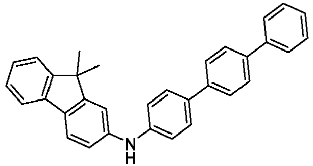 | 71% |
| A2-10 | 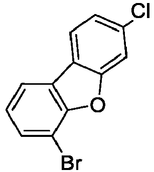 | MeLi | 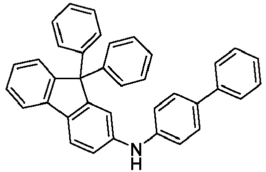 | 78% |
| A2-11 | 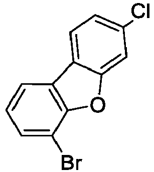 | MeLi | 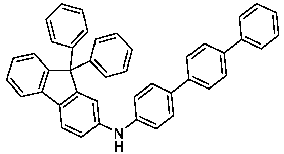 | 80% |
| A2-13 | 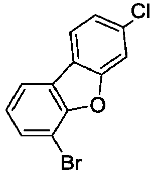 | MeLi | 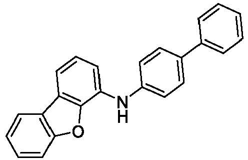 | 76% |
| A2-14 | 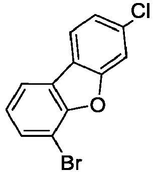 | MeLi | 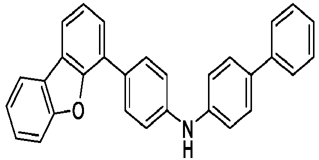 | 70% |
| A2-19 | 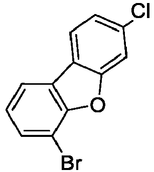 | MeLi | 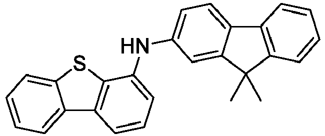 | 69% |
| A2-38 | 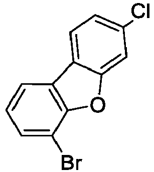 | MeLi | 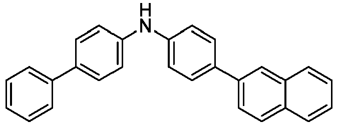 | 77% |
| A2-39 | 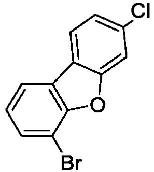 | MeLi | 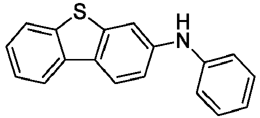 | 80% |
| A2-48 | 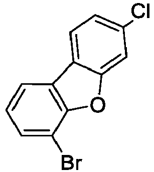 | MeLi | 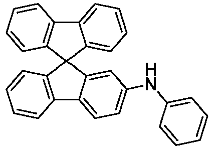 | 71% |
| A3-2 | 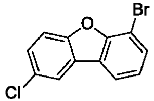 | MeLi | 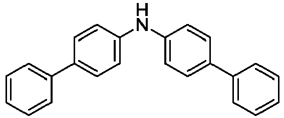 | 78% |
| A3-3 | 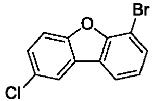 | MeLi | 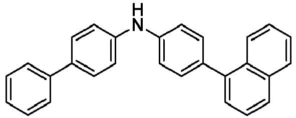 | 73% |
| A3-4 | 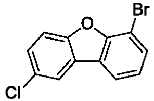 | MeLi | 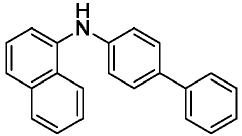 | 72% |
| A3-14 | 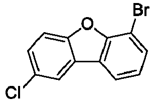 | MeLi | 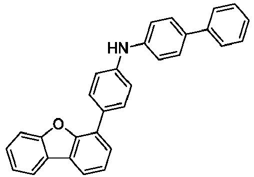 | 74% |
| A3-31 | 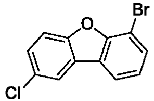 | MeLi | 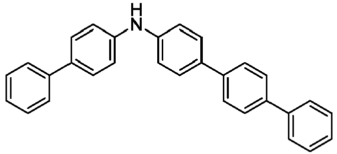 | 78% |
| A3-33 | 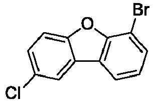 | MeLi | 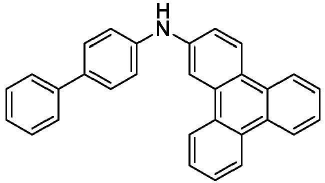 | 81% |
| A3-34 | 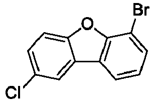 | MeLi | 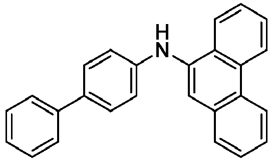 | 76% |
| A3-36 | 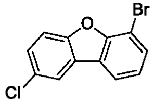 | MeLi | 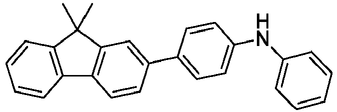 | 77% |
| A4-2 | 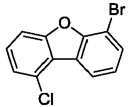 | MeLi | 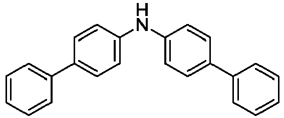 | 78% |
| A4-6 | 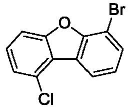 | MeLi | 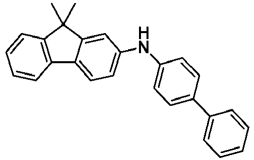 | 70% |
| A4-17 | 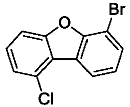 | MeLi | 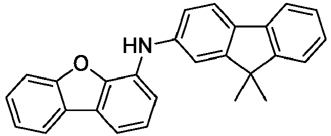 | 72% |
| A4-32 | 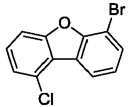 | MeLi | 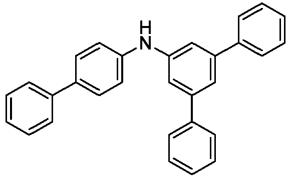 | 73% |
| A4-35 | 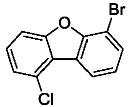 | MeLi | 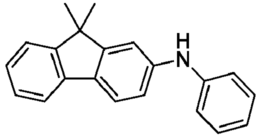 | 72% |
| A4-42 | 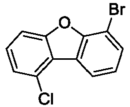 | MeLi | 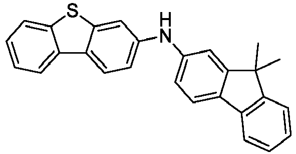 | 78% |
| A4-44 | 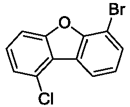 | MeLi | 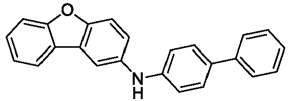 | 74% |
| A5-4 | 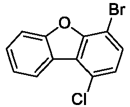 | MeLi | 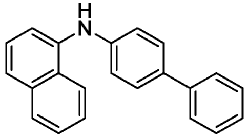 | 70% |
| A5-15 | 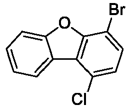 | MeLi | 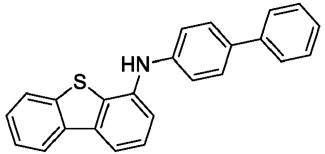 | 71% |
| A5-19 | 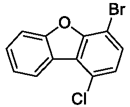 | MeLi | 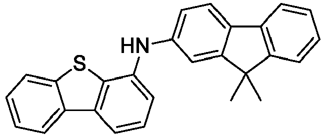 | 80% |
| A5-29 | 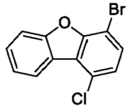 | MeLi | 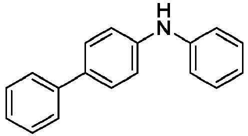 | 73% |
| A5-43 | 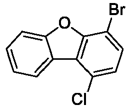 | MeLi | 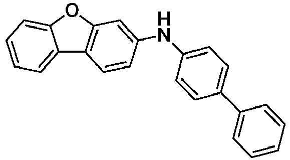 | 75% |
| A5-48 | 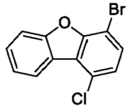 | MeLi | 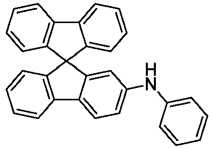 | 68% |
| A6-4 | 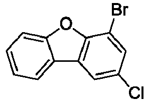 | MeLi | 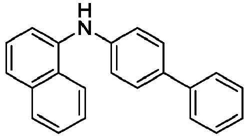 | 78% |
| A6-5 | 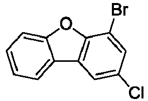 | MeLi | 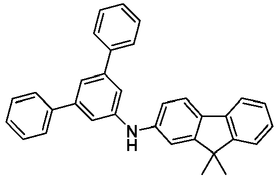 | 70% |
| A6-15 | 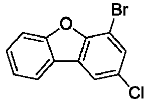 | MeLi | 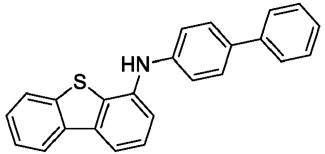 | 71% |
| A6-30 | 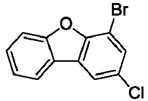 | MeLi | 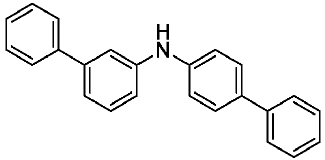 | 77% |
| A6-37 | 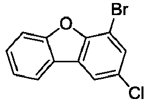 | MeLi | 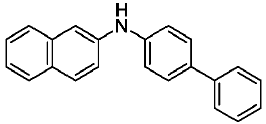 | 72% |
| A6-39 | 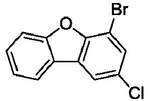 | MeLi | 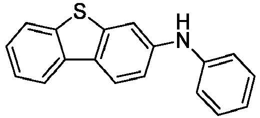 | 79% |
| A6-40 | 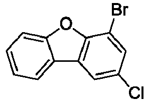 | MeLi | 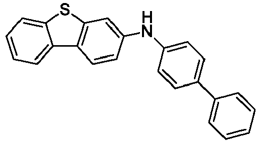 | 68% |
| A7-2 | 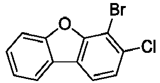 | MeLi | 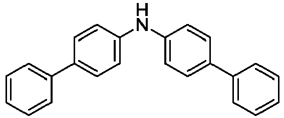 | 72% |
| A7-29 | 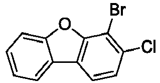 | MeLi | 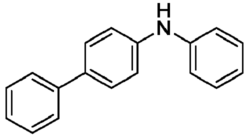 | 74% |
| A7-41 | 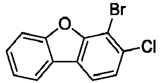 | MeLi | 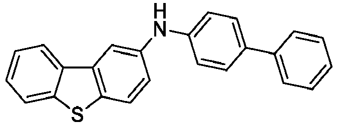 | 77% |
| B1-1 | 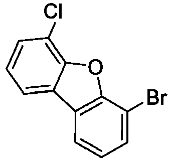 | PhLi | 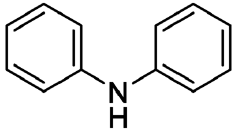 | 72% |
| B1-2 | 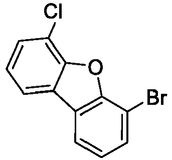 | PhLi | 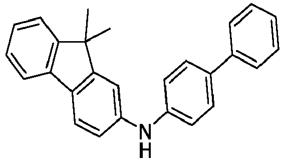 | 66% |
| B1-6 | 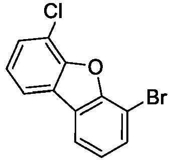 | PhLi | 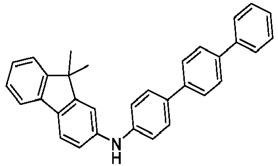 | 65% |
| B1-10 | 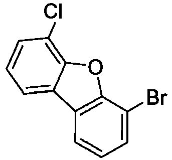 | PhLi | 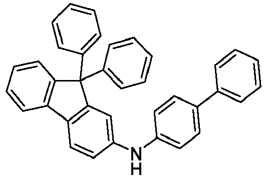 | 71% |
| B1-11 | 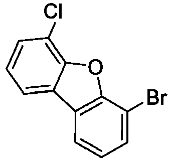 | PhLi | 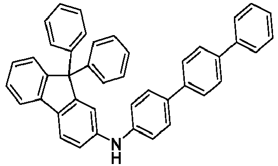 | 77% |
| B1-13 | 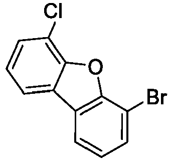 | PhLi | 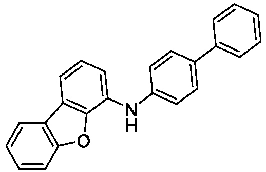 | 69% |
| B1-14 | 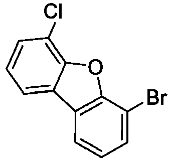 | PhLi | 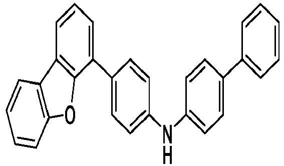 | 65% |
| B2-1 | 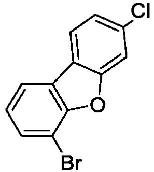 | PhLi | 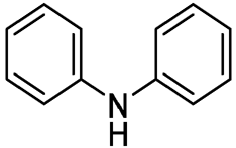 | 85% |
| B2-2 | 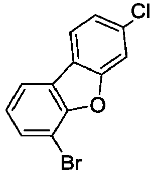 | PhLi | 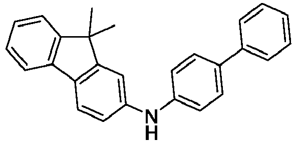 | 70% |
| B2-6 | 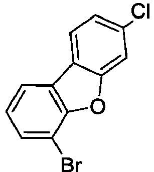 | PhLi | 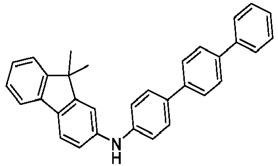 | 80% |
| B2-10 | 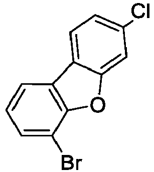 | PhLi | 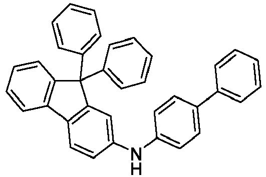 | 82% |
| B2-11 | 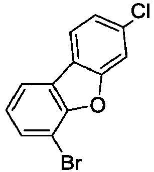 | PhLi | 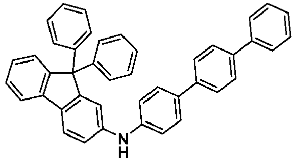 | 85% |
| B2-13 | 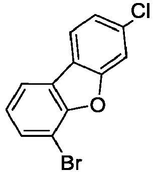 | PhLi | 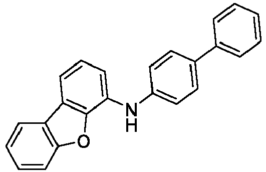 | 67% |
| B2-14 | 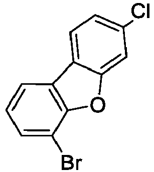 | PhLi | 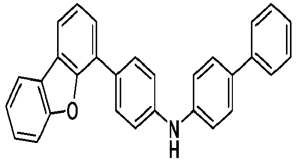 | 69% |

### <Preparation Example 2> Preparation of Compound A1-51

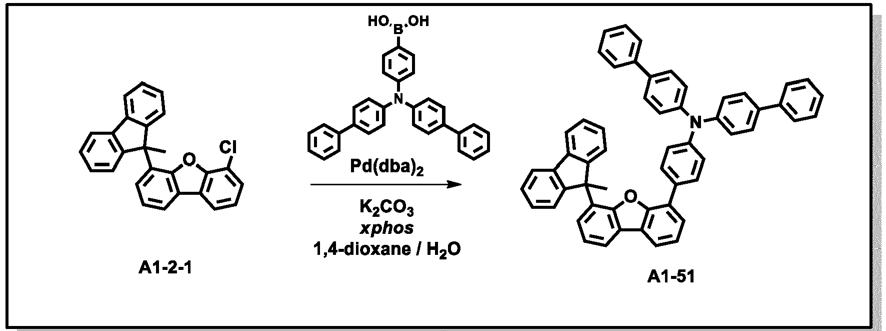

### 1) Preparation of Compound A1-2-3

After dissolving Compound A1-2-1 (D) (10 g, 26.26 mmol) and (4-(di([1,1'-biphenyl]-4-yl)amino)phenyl)boronic acid (E) (11.59 g, 26.26 mmol) in 1,4-dioxane (100 ml) and distilled water (20 ml), Pd(dba)₂ (0.75 g, 1.31 mol), xphos (1.25 g, 2.63 mmol) and K₂CO₃ (9.07 g, 65.64 mmol) were introduced thereto, and the result was stirred for 12 hours under reflux. After the reaction was completed, the reaction solution was extracted with dichloromethane and distilled water, and after drying the organic layer with anhydrous MgSO₄, the solvent was removed using a rotary evaporator. The result was purified by column chromatography using dichloromethane and hexane as a developing solvent to obtain Compound A1-51 (14 g, 72%).

### 2) Preparation of Compounds of Table 2

Compounds of the following Table 2 were synthesized in the same manner as in Preparation Example 2 except that Intermediate D of the following Table 2 was used instead of Compound A1-2-1 (D), and Intermediate E of the following Table 2 was used instead of (4-(di([1,1'-biphenyl]-4-yl)amino)phenyl)boronic acid (E).

**[Table 2]**

| Compoun d No. | Intermediate D | Intermediate E | Yiel d |
|---|---|---|---|
| A2-54 | 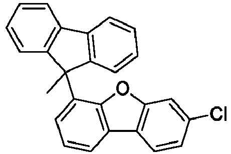 | 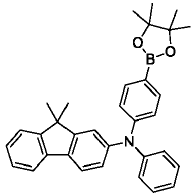 | 77% |
| A3-49 | 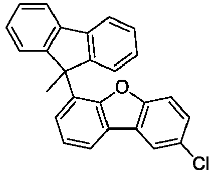 | 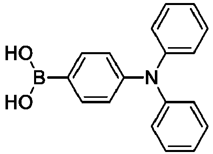 | 72% |
| A4-52 | 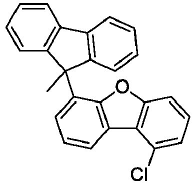 | 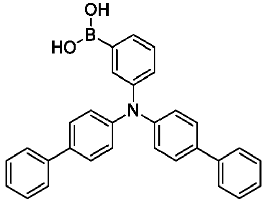 | 69% |
| A5-51 | 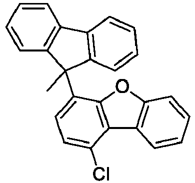 | 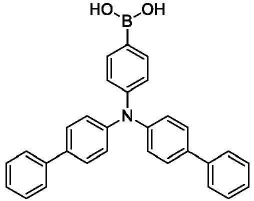 | 78% |
| A6-50 | 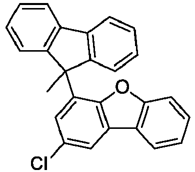 | 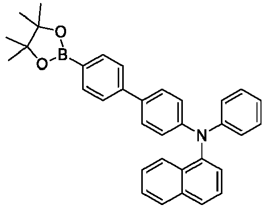 | 72% |
| A7-49 | 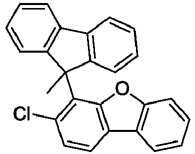 | 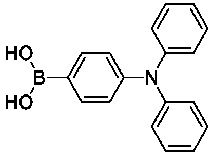 | 75% |

### <Preparation Example 3> Preparation of Compound D-5

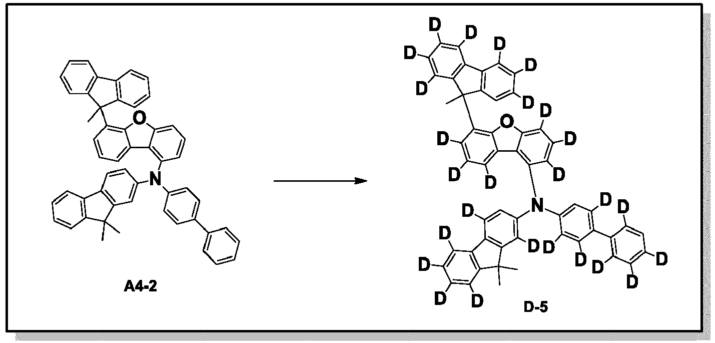

### 1) Preparation of Compound D-5

Compound A4-2 (10 g, 14.17 mmol), trifluoromethanesulfonic acid (3.19 g, 21.25 mmol) and D6-benzene (200 ml) were introduced into a reaction flask, and then stirred for 5 hours under reflux. After the reaction was completed, the reaction was terminated by introducing water thereto, and the result was extracted using dichloromethane and distilled water. After drying the organic layer with anhydrous MgSO₄, the solvent was removed using a rotary evaporator, and the result was purified by column chromatography using dichloromethane and hexane as a developing solvent to obtain Compound D-5 (9 g, 87%). Through an LC/MS analysis, an average of 26 deuterium substitutions were identified.

### <Preparation Example 4> Preparation of Compound D-11

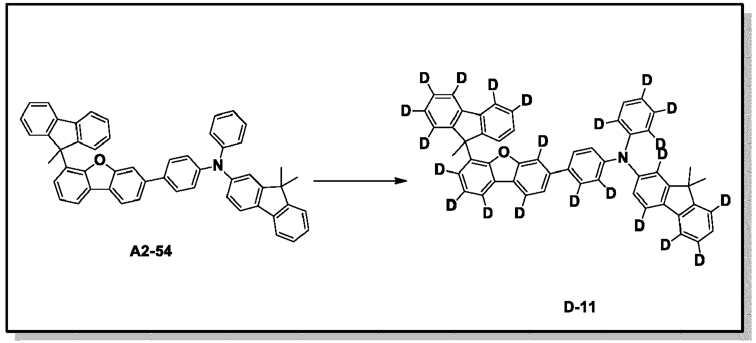

### 1) Preparation of Compound D-11

Compound A2-54 (10 g, 14.17 mmol), trifluoromethanesulfonic acid (3.19 g, 21.25 mmol) and D6-benzene (200 ml) were introduced into a reaction flask, and then stirred for 5 hours under reflux. After the reaction was completed, the reaction was terminated by introducing water thereto, and the result was extracted using dichloromethane and distilled water. After drying the organic layer with anhydrous MgSO₄, the solvent was removed using a rotary evaporator, and the result was purified by column chromatography using dichloromethane and hexane as a developing solvent to obtain Compound D-11 (8 g, 78%). Through an LC/MS analysis, an average of 22 deuterium substitutions were identified.

Compounds described in the present specification were prepared in the same manner as in the preparation examples, and in order to identify synthesis identification results of the prepared compounds, ¹H NMR (CDCl₃, 200 Mz) and FD-mass spectrometry (FD-MS: Field desorption mass spectrometry) were used, and the measurement values are shown in the following Table 3 and Table 4. The following Table 3 shows measurement values of ¹H NMR (CDCl₃, 200 Mz) for some of the prepared compounds, and the following Table 4 shows measurement values of FD-mass spectrometry (FD-MS: Field desorption mass spectrometry) for the prepared compounds.

**[Table 3]**

| Compou nd | ¹H NMR (CDCl₃, 200 Mz) |
|---|---|
| A1-1 | δ=8.03 (1H, s), 7.88∼7.90 (3H, m), 7.80 (1H, d), 7.75 (2H, m), 7.24∼7.38 (9H, m), 7.00∼7.08 (7H, m), 6.91 (1H, d), 2.28 (3H, s) |
| A1-2 | δ=8.03 (1H, s), 7.88∼7.90 (3H, m), 7.80∼7.75 (5H, m), 7.55~7.28 (21H, m), 7.00∼7.09 (2H, m), 2.28 (3H, s) |
| A1-6 | δ=8.03 (1H, d), 7.90∼7.75 (8H, m), 7.49∼7.28 (18H, m), 7.16-09 (2H, q), 6.91 (1H, d), 2.28 (3H, s), 1.69 (6H, s) |
| A1-10 | δ=7.98 (1H, d), 7.90∼7.75 (8H, m), 7.49∼7.18 (29H, m), 6.91 (1H, d), 2.28 (3H, s), |
| A1-11 | 5=7.88-7.97 (5H, m), 7.75 (2H, d), 7.64 (1H, d), 7.55∼7.18 (33H, m), 6.79 (1H, d), 2.28 (3H, s) |
| A1-13 | δ=7.98 (1H, d), 7.88∼7.90 (3H, m), 7.75 (2H, m), 7.28~7.55 (21H, m), 7.09 (1H, d), 6.97 (2H, d), 2.28 (3H, s) |
| A1-14 | δ=7.98 (1H, d), 7.88∼7.90 (3H, m), 7.75 (2H, m), 7.28~7.55 (25H, m), 7.09 (1H, d), 6.97 (2H, d), 2.28 (3H, s) |
| A1-30 | δ=7.87 (2H, d), 7.71 (1H, d), 7.55∼7.38 (17H, m), 7.28~7.25 (4H, m), 7.07∼7.05 (2H, m), 6.89∼6.88 (2H, m), 6.69 (2H, d), 6.59 (1H, d), 6.39 (1H, d), 2.28 (3H, s) |
| A1-41 | δ=8.45 (1H, d), 7.98 (1H, d), 7.87 (2H, d), 7.73∼7.71 (2H, m), 7.55∼7.38 (14H, m), 7.28∼7.25 (4H, m), 7.07∼7.05 (2H, m), 6.86 (1H, d), 6.69 (2H, d), 6.39 (1H, d), 2.28 (3H, s) |
| A1-47 | δ=7.87 (2H, d), 7.71 (1H, d), 7.62~7.55 (4H, m), 7.38∼7.07 (22H, m), 6.81 (1H, t), 6.75 (1H, s), 6.63 (2H, d), 6.58 (1H, d), 6.39 (1H, d), 2.28 (3H, s) |
| A1-51 | 5=7.87-7.81 (4H, m), 7.71 (1H, d), 7.55∼7.38 (21H, m), 7.28∼7.27 (3H, m), 7.05 (1H, d), 6.69 (6H, d), 2.28 (3H, s) |
| A2-1 | δ=8.03 (1H, s), 7.88∼7.90 (3H, m), 7.80 (1H, d), 7.75 (2H, m), 7.24∼7.38 (9H, m), 7.00~7.08 (7H, m), 6.91 (1H, d), 2.28 (3H, s) |
| A2-2 | δ=8.03 (1H, s), 7.88∼7.90 (3H, m), 7.80∼7.75 (5H, m), 7.55∼7.28 (21H, m), 7.00∼7.09 (2H, m), 2.28 (3H, s) |
| A2-6 | δ=8.03 (1H, d), 7.90∼7.75 (8H, m), 7.49∼7.28 (18H, m), 7.16-09 (2H, q), 6.91 (1H, d), 2.28 (3H, s), 1.69 (6H, s) |
| A2-10 | δ=8.03 (1H, d), 7.90∼7.75 (8H, m), 7.49∼7.18 (29H, m), 6.91 (1H, d), 2.28 (3H, s) |
| A2-11 | δ=8.03 (1H, d), 7.90∼7.75 (8H, m), 7.49∼7.18 (33H, m), 6.91 (1H, d), 2.28 (3H, s) |
| A2-13 | δ=8.03 (1H, d), 7.97 (1H, d), 7.90~7.88 (3H, m), 7.75~7.80 (3H, m), 7.64 (1H, d) 7.54~7.28 (18H, m), 7.09 (1H, d), 6.97~6.91 (2H, q), 2.28 (3H, s) |
| A2-14 | δ=8.03 (1H, d), 7.97 (1H, d), 7.90~7.88 (3H, m), 7.75~7.80 (3H, m), 7.64 (1H, d) 7.54~7.28 (22H, m), 7.09 (1H, d), 6.97~6.91 (2H, q), 2.28 (3H, s) |
| A2-19 | δ=8.45 (1H, d), 7.98 (1H, d), 7.87 (3H, d), 7.81 (1H, d), 7.55~7.28 (17H, m), 7.05 (1H, d), 6.86 (1H, d), 6.75 (1H, s), 6.58 (1H, d), 6.33 (1H, d), 2.28 (3H, s), 1.72 (6H, s) |
| A2-38 | δ=8.00 (2H, d), 7.92∼7.87 (3H, m), 7.73 (1H, d), 7.71 (1H, d), 7.64∼7.38 (18H, m), 7.28∼7.27 (3H, m), 705 (1H, d), 6.69 (4H, d), 6.33 (1H, d), 2.28 (3H, s) |
| A2-39 | δ=8.45 (1H, d), 7.98 (1H, d), 7.87 (2H, d), 7.80 (1H, d), 7.71 (1H, d), 7.64 (1H, d), 7.55∼7.38 (14H, m), 7.28∼7.27 (3H, m), 7.06∼7.05 (2H, m), 6.88 (1H, d), 6.69 (2H, d), 6.33 (1H, d), 2.28 (3H, s) |
| A2-48 | δ=7.87 (4H, d), 7.75∼7.64 (3H, m), 7.55∼7.50 (5H, m), 7.43∼7.16 (15H, m), 7.05 (1H, d), 6.81 (1H, t), 6.63 (2H, d), 6.55 (1H, s), 6.39 (1H, d), 6.33 (1H, d), 2.28 (3H, s) |
| A2-54 | δ=7.95 (1H, d), 7.87 (3H, d), 7.75∼7.71 (2H, m), 7.64∼7.54 (7H, m), 7.38∼7.20 (9H, m), 7.05 (1H, d), 6.81∼6.58 (7H, m), 2.28 (3H, s), 1.72 (6H, s) |
| A3-2 | δ=7.87 (2H, d), 7.71 (1H, d), 7.65 (1H, s), 7.55∼7.38 (19H, m), 7.28∼7.27 (3H, m), 7.05 (1H, d), 6.69 (4H, d), 6.39 (1H, d), 2.28 (3H, s) |
| A3-3 | δ=8.55 (1H, d), 8.42 (1H, d), 8.08∼8.04 (2H, m), 7.87 (2H, d), 7.71∼7.38 (19H, m), 7.28∼7.27 (3H, m), 7.05 (1H, d), 6.69 (4H, d), 6.39 (1H, d), 2.28 (3H, s) |
| A3-4 | δ=8.07 (1H, d), 8.02 (1H, d), 7.87 (2H, d), 7.71 (1H, d), 7.65 (1H, s), 7.57∼7.38 (16H, m), 7.28∼7.27 (3H, m), 7.05 (1H, d), 6.98 (1H, d), 6.69 (2H, d), 6.39 (H, d), 2.28 (3H, s) |
| A3-14 | 5=7.89-7.81 (5H, m), 7.71∼7.65 (3H, m), 7.54∼7.27 (20H, m), 7.05 (1H, d), 6.69 (4H, d), 6.39 (1H, d), 2.28 (3H, s) |
| A3-31 | δ=7.87 (2H, d), 7.71 (1H, d), 7.65 (1H, s), 7.55∼7.38 (19H, m), 7.28∼7.25 (7H, m), 7.05 (1H, d), 6.69 (4H, d), 6.39 (1H, d), 2.28 (3H, s) |
| A3-33 | δ=8.93 (2H, d), 8.13~8.12 (3H, m), 7.88∼7.87 (7H, m), 7.71 (1H, d), 7.65 (1H, s), 7.55∼7.38 (12H, m), 7.28∼7.27 (3H, m), 7.05 (1H, d), 7.02 (1H, d), 6.69 (2H, d), 6.39 (1H, d), 2.28 (3H, s) |
| A3-34 | δ=8.93 (2H, d), 8.12 (2H, d), 7.88∼7.82 (6H, m), 7.71 (1H, d), 7.65 (1H, s), 7.55∼7.38 (12H, m), 7.28∼7.27 (3H, m), 7.05 (1H, d), 6.91 (1H, s), 6.69 (2H, d), 6.38 (1H, d), 2.28 (3H, s) |
| A3-36 | δ=7.93 (1H, d), 7.87 (3H, d), 7.77 (1H, s), 7.71∼7.63 (3H, m), 7.55∼7.54 (5H, m), 7.41∼7.38 (4H, m), 7.28∼7.20 (6H, m), 7.05 (1H, d), 6.81 (1H, t), 6.69 (2H, d), 6.63 (2H, d), 6.39 (1H, d), 2.28 (3H, s), 1.72 (6H, s) |
| A3-49 | δ=7.87 (2H, d), 7.81 (1H, d), 7.72∼7.71 (3H, m), 7.55∼7.54 (4H, m), 7.38∼7.20 (9H, m), 7.05 (1H, d), 6.81 (2H, t), 6.69 (2H, d), 6.63 (4H, d), 2.28 (3H, s) |
| A4-2 | δ=7.87 (2H, d), 7.71 (1H, d), 7.55∼7.38 (18H, m), 7.28∼7.27 (3H, m), 7.13 (1H, t), 7.05∼7.02 (2H, m), 6.69 (4H, d), 6.33 (1H, d), 2.28 (3H, s) |
| A4-6 | δ=7.87 (3H, d), 7.71 (1H, d), 7.62∼7.51 (10H, m), 7.41∼7.38 (4H, m), 7.28∼7.27 (4H, m), 7.13 (1H, t), 7.05∼7.02 (2H, m), 6.75 (1H, s), 6.69 (2H, d), 6.58 (1H, d), 6.33 (1H, d), 2.28 (3H, s), 1.72 (6H, s) |
| A4-17 | δ=7.89 (1H, d), 7.87 (3H, d), 7.71∼7.55 (6H, m), 7.38∼7.25 (10H, m), 7.13∼7.02 (4H, m), 6.75 (1H, s), 6.58 (1H, d), 6.39 (1H, d), 6.33 (1H, d), 2.28 (3H, s) |
| A4-32 | δ=7.87 (2H, d), 7.71 (1H, d), 7.55∼7.38 (21H, m), 7.28∼7.27 (3H, m), 7.13 (1H, t), 7.06∼7.02 (3H, m), 6.85 (2H, s), 6.69 (2H, d), 6.33 (1H, d), 2.28 (3H, s) |
| A4-35 | δ=7.87 (3H, d), 6.61 (1H, d), 7.62 (1H, d), 6.55 (3H, d), 7.38 (3H, t), 7.28∼7.13 (7H, m), 7.05∼7.02 (2H, m), 6.81 (1H, t), 6.75 (1H, s), 6.63 (2H, d), 6.58 (1H, d), 6.33 (1H, d), 2.28 (3H, s), 1.72 (6H, s) |
| A4-42 | δ=8.45 (1H, d), 7.98 (1H, d), 7.87 (3H, d), 7.80 (1H, d), 7.71 (1H, d), 7.55∼7.50 (6H, m), 7.38∼7.28 (6H, m), 7.13∼7.02 (4H, m), 6.88 (1H, d), 6.75 (1H, s), 6.58 (1H, d), 6.33 (1H, d), 2.28 (3H, s), 1.72 (6H, s) |
| A4-44 | δ=7.89 (1H, d), 7.87 (2H, d), 7.71∼7.65 (3H, m), 7.55∼7.28 (17H, m), 7.13 (1H, t), 7.05 (1H, d), 7.02 (1H, d), 6.69 (2H, d), 6.39 (1H, d), 6.33 (1H, d), 2.28 (3H, s) |
| A4-52 | δ=7.87 (2H, d), 7.75 (1H, d), 7.71 (1H, d), 7.62∼7.38 (21H, m), 7.28∼7.27 (3H, m), 7.05 (1H, d), 6.89~6.88 (2H, m), 6.69 (4H, d), 6.59 (1H, d), 2.28 (3H, s) |
| A5-4 | δ=8.07 (1H, d), 8.02 (1H, d), 7.89 (1H, d), 7.87 (2H, d), 7.66 (1H, d), 7.57∼7.28 (19H, m), 6.98 (1H, d), 6.80 (1H, d), 6.28 (1H, d), 2.28 (3H, s) |
| A5-15 | δ=8.45 (1H, d), 7.98 (1H, d), 7.89 (1H, d), 7.81 (1H, d), 7.87 (2H, d), 7.66 (1H, d), 7.55∼7.27 (18H, m), 6.86 (1H, d), 6.80 (1H, d), 6.69 (2H, d), 6.28 (1H, d), 2.28 (3H, s) |
| A5-19 | δ=8.45 (1H, d), 7.98 (1H, d), 7.89~7.81 (5H, m), 7.66~7.50 (7H, m), 7.38∼7.27 (9H, m), 6.86~6.75 (3H, m), 6.58 (1H, d), 6.28 (1H, d), 2.28 (3H, s), 1.72 (6H, s) |
| A5-29 | δ=7.89 (1H, d), 7.87 (2H, d), 7.66 (1H, d), 7.55~7.20 (17H, m), 6.81~6.80 (2H, m), 6.69 (2H, d), 6.63 (2H, d), 6.28 (1H, d), 2.28 (3H, s) |
| A5-43 | δ=7.89 (2H, d), 7.87 (2H, d), 7.66∼7.64 (3H, m), 7.55∼7.28 (18H, m), 6.80 (1H, d), 6.69 (2H, d), 6.33 (1H, d), 6.28 (1H, d), 2.28 (3H, s) |
| A5-48 | δ=7.89 (1H, d), 7.87 (4H, d), 7.75 (1H, d), 7.66 (1H, d), 7.55∼7.50 (5H, m), 7.38∼7.16 (15H, m), 6.81∼6.80 (2H, m), 6.63 (2H, d), 6.55 (1H, s), 6.39 (1H, d), 6.28 (1H, d), 2.28 (3H, s) |
| A5-51 | δ=7.89 (1H, d), 7.87 (2H, d), 7.70 (1H, d), 7.66 (1H, d), 7.55∼7.28 (24H, m), 7.11 (1H, d), 6.69 (6H, d), 2.28 (3H, s) |
| A6-4 | δ=8.07 (1H, d), 8.02 (1H, d), 7.89 (1H, d), 7.87 (2H, d), 7.66 (1H, d), 7.57∼7.28 (20H, m), 6.98 (1H, d), 6.69 (2H, d), 6.25 (1H, s), 2.28 (3H, s) |
| A6-5 | δ=7.89 (1H, d), 7.87 (3H, d), 7.66∼7.28 (24H, m), 7.06 (1H, s), 6.85 (2H, s), 6.75 (1H, s), 6.58 (1H, d), 6.25 (1H, s), 2.28 (3H, s), 1.72 (6H, s) |
| A6-15 | δ=8.45 (1H, d), 7.98 (1H, d), 7.89 (1H, d), 7.87 (2H, d), 7.81 (1H, d), 7.66 (1H, d), 7.55∼7.27 (19H, m), 6.86 (1H, d), 6.69 (2H, d), 6.25 (1H, s), 2.28 (3H, s) |
| A6-30 | δ=7.89 (1H, d), 7.87 (2H, d), 7.66 (1H, d), 7.55∼7.28 (22H, m), 6.89 (1H, s), 6.88 (1H, d), 6.69 (2H, d), 6.59 (1H, d), 6.25 (1H, s), 2.28 (3H, s) |
| A6-37 | 5=7.89-7.74 (7H, m), 7.66 (1H, d), 7.54∼7.28 (19H, m), 6.69 (2H, d), 6.25 (1H, s), 2.28 (3H, s) |
| A6-39 | δ=8.45 (1H, d), 7.98 (1H, d), 7.89 (1H, d), 7.87 (2H, d), 7.80 (1H, d), 7.66 (1H, d), 7.55∼7.28 (18H, m), 7.06 (1H, s), 6.88 (1H, d), 6.69 (2H, d), 6.25 (1H, s), 2.28 (3H, s) |
| A6-40 | δ=8.45 (1H, d), 7.98 (1H, d), 7.89 (1H, d), 7.87 (2H, d), 7.73 (1H, d), 7.66 (1H, d), 7.55∼7.28 (19H, m), 6.86 (1H, d), 6.69 (2H, d), 6.25 (1H, s), 2.28 (3H, s) |
| A6-50 | 5=8.07 (1H, d), 8.02 (1H, d), 7.89 (1H, d), 7.87 (2H, d), 7.66 (1H, d), 7.57∼7.53 (8H, m), 7.38~7.20 (14H, m), 6.98 (1H, d), 6.81 (1H, t), 6.69 (2H, d), 6.63 (2H, d), 2.28 (3H, s) |
| A7-2 | δ=7.89 (1H, d), 7.87 (2H, d), 7.66 (1H, d), 7.55~7.28 (23H, m), 6.69 (4H, d), 6.28 (1H, d), 2.28 (3H, s) |
| A7-29 | δ=7.89 (1H, d), 7.87 (2H, d), 7.66 (1H, d), 7.55~7.20 (18H, m), 6.81 (1H, t), 6.69 (2H, d), 6.63 (2H, d), 6.28 (1H, d), 2.28 (3H, s) |
| A7-41 | δ=8.45 (1H, d), 7.98 (1H, d), 7.89∼7.80 (4H, m), 7.66 (1H, d), 7.55~7.20 (13H, m), 7.06 (1H, s), 6.88 (1H, d), 6.81 (1H, t), 6.63 (2H, d), 6.28 (1H, d), 2.28 (3H, s) |
| A7-49 | δ=7.89 (1H, d), 7.87 (2H, d), 7.77 (1H, d), 7.70 (1H, d), 7.66 (1H, d), 7.55~7.54 (4H, m), 7.38∼7.20 (10H, m), 6.81 (2H, t), 6.69 (2H, d), 6.63 (4H, d), 2.28 (3H, s) |
| B1-1 | 5=7.90-7.88 (3H, m), 7.64 (1H, d), 7.09 (2H, q), 7.38∼7.00 (23H, m) |
| B1-2 | 5=7.90-7.88 (3H, m), 7.75 (4H, m), 7.64 (1H, d), 7.55∼7.09 (26H, m), 6.97 (1H, d) |
| B1-6 | 5=7.90-7.86 (5H, m), 7.75 (2H, m), 7.64 (1H, d), 7.55∼7.09 (26H, m), 6.97 (1H, d)1.69 (6H, s) |
| B1-10 | δ=7.98 (1H, d), 7.90∼7.75 (8H, m), 7.49~7.18 (29H, m), 6.91 (1H, d), 2.28 (3H, s) |
| B1-11 | 5=7.88-7.97 (5H, m), 7.75 (2H, d), 7.64 (1H, d), 7.55∼7.18 (33H, m), 6.97 (1H, d) |
| B1-13 | 5=7.90-7.86 (5H, m), 7.75 (2H, m), 7.64 (1H, d), 7.49∼7.18 (35H, m), 6.97 (1H, d) |
| B1-14 | 5=8.08-7.98 (3H, m), 7.90∼7.88 (3H, m), 7.75 (2H, m), 7.64 (1H, d), 7.55~7.18 (29H, m), 6.97 (1H, d) |
| B2-1 | 5=7.90-7.88 (3H, m), 7.64 (1H, d), 7.09 (2H, q), 7.38∼7.00 (23H, m) |
| B2-2 | 5=7.90-7.88 (3H, m), 7.75 (4H, m), 7.64 (1H, d), 7.55∼7.09 (26H, m), 6.97 (1H, d) |
| B2-6 | 5=7.90-7.86 (5H, m), 7.75 (2H, m), 7.64 (1H, d), 7.55∼7.09 (26H, m), 6.97 (1H, d), 1.69 (6H, s) |
| B2-10 | δ=7.98 (1H, d), 7.90∼7.75 (8H, m), 7.49~7.18 (29H, m), 6.91 (1H, d), 2.28 (3H, s), |
| B2-11 | 5=7.88-7.97 (5H, m), 7.75 (2H, d), 7.64 (1H, d), 7.55~7.18 (33H, m), 6.97 (1H, d) |
| B2-13 | 5=7.90-7.86 (5H, m), 7.75 (2H, m), 7.64 (1H, d), 7.49∼7.18 (35H, m), 6.97 (1H, d) |
| B2-14 | δ=8.08∼7.98 (3H, m), 7.90∼7.88 (3H, m), 7.75 (2H, m), 7.64 (1H, d), 7.55~7.18 (29H, m), 6.97 (1H, d) |
| D-5 | δ=7.50 (1H, s), 7.34 (1H, s), 6.70 (1H, s), 6.37 (1H, s), 2.28 (3H, s), 1.72 (6H, s) |
| D-11 | δ=7.66 (1H, s), 7.54 (1H, d), 7.34 (1H, d), 7.19 (1H, s), 7.07 (2H, d), 6.69 (1H, d), 6.37 (1H, s), 2.28 (3H, s), 1.72 (6H, s) |

**[Table 4]**

| Compoun d | FD-MS | Compou nd | FD-MS |
|---|---|---|---|
| A1-1 | m/z=513.64 (C₃₈H₂₇NO=513.64) | A4-44 | m/z=679.80 (C₅₀H₃₃NO=679.25) |
| A1-2 | m/z=665.82 (C₅₀H₃₅NO=665.27) | A4-52 | m/z=741.91 (C₅₆H₃₉NO=741.30) |
| A1-6 | m/z=705.88 (C₅₃H₃₉NO=705.30) | A5-4 | m/z=639.78 (C₄₈H₃₃NO=639.26) |
| A1-10 | m/z=830.04 (C₆₃H₄₃NO=830.04) | A5-15 | m/z=695.87 (C₅₀H₃₃NOS=695.23) |
| A1-11 | m/z=906.14 (C₆₉H₄₇NO=906.14) | A5-19 | m/z=735.93 (C₅₃H₃₇NOS=735.26) |
| A1-13 | m/z=679.82 (C₅₀H₃₃NO₂=679.82) | A5-29 | m/z=589.72 (C₄₄H₃₁NO=589.24) |
| A1-14 | m/z=755.90 (C₅₆H₃₇NO₂=755.28) | A5-43 | m/z=679.80 (C₅₀H₃₃NO=679.25) |
| A1-30 | m/z=665.82 (C₅₀H₃₅NO=665.27) | A5-48 | m/z=751. 91 (C₅₇H₃₇NO=751.29) |
| A1-41 | m/z=695.87 (C₅₀H₃₃NOS=695.23) | A5-51 | m/z=741.91 (C₅₆H₃₉NO=741.30) |
| A1-47 | m/z=753.93 (C₅₇H₃₉NO=753.30) | A6-4 | m/z=639.78 (C₄₃H₃₃NO=639.26) |
| A1-51 | m/z=741.91 (C₅₆H₃₉NO=741.30) | A6-5 | m/z=781.98 (C₅₉H₄₃NO=781.33) |
| A2-1 | m/z=513.64 (C₃₈H₂₇NO=513.64) | A6-15 | m/z=695.87 (C₅₀H₃₃NOS=695.23) |
| A2-2 | m/z=665.82 (C₅₀H₃₅NO=665.27) | A6-30 | m/z=665.82 (C₅₀H₃₅NO=665.27) |
| A2-6 | m/z=705.88 (C₅₃H₃₉NO=705.30) | A6-37 | m/z=639.78 (C₄₈H₃₃NO=639.26) |
| A2-10 | m/z=830.04 (C₆₃H₄₃NO=830.04) | A6-39 | m/z=695.87 (C₅₀H₃₃NOS=695.23) |
| A2-11 | m/z=906.14 (C₆₉H₄₇NO=906.14) | A6-40 | m/z=695.87 (C₅₀H₃₃NOS=695.23) |
| A2-13 | m/z=679.82 (C₅₀H₃₃NO₂=679.82) | A6-50 | m/z=715.88 (C₅₄H₃₇NO=715.29) |
| A2-14 | m/z=755.90 (C₅₆H₃₇NO₂=755.28) | A7-2 | m/z=665.82 (C₅₀H₃₅NO=665.27) |
| A2-19 | m/z=735.93 (C₅₃H₃₇NOS=735.26) | A7-29 | m/z=589.72 (C₄₄H₃₁NO=589.24) |
| A2-38 | m/z=715.88 (C₅₄H₃₇NO=715.29) | A7-41 | m/z=619.77 (C₄₄H₂₉NOS=619.20) |
| A2-39 | m/z=695.87 (C₅₀H₃₃NOS=695.23) | A7-49 | m/z=589.72 (C₄₄H₃₁NO=589.24) |
| A2-48 | m/z=751.91 (C₅₇H₃₇NO=751.29) | B1-1 | m/z=575.71 (C₄₃H₂₉NO=575.71) |
| A2-54 | m/z=705.88 (C₅₃H₃₉NO=705.30) | B1-2 | m/z=727.91 (C₅₅H₃₇NO=727.91) |
| A3-2 | m/z=665.82 (C₅₀H₃₅NO=665.27) | B1-6 | m/z=767.97 (C₅₈H₄₁NO=767.97) |
| A3-3 | m/z=715.88 (C₅₄H₃₇NO=715.29) | B1-10 | m/z=892.11 (C₆₈H₄₅NO=892.11) |
| A3-4 | m/z=639.78 (C₄₃H₃₃NO=639.26) | B1-11 | m/z=968.21 (C₇₄H₄₉NO=968.21) |
| A3-14 | m/z=755.90 (C₅₆H₃₇NO₂=755.28) | B1-13 | m/z=741.89 (C₅₅H₃₅NO₂=741.89) |
| A3-31 | m/z=741.91 (C₅₆H₃₉NO=741.30) | B1-14 | m/z=817.99 (C₆₁H₃₉NO₂=817.99) |
| A3-33 | m/z=739.90 (C₅₆H₃₇NO=739.29) | B2-1 | m/z=575.71 (C₄₃H₂₉NO=575.71) |
| A3-34 | m/z=689.84 (C₅₂H₃₅NO=689.27) | B2-2 | m/z=727.91 (C₅₅H₃₇NO=727.91) |
| A3-36 | m/z=705.88 (C₅₃H₃₉NO=705.30) | B2-6 | m/z=767.97 (C₅₈H₄₁NO=767.97) |
| A3-49 | m/z=589.72 (C₄₄H₃₁NO=589.24) | B2-10 | m/z=892.11 (C₆₈H₄₅NO=892.11) |
| A4-2 | m/z=665.82 (C₅₀H₃₅NO=665.27) | B2-11 | m/z=968.21 (C₇₄H₄₉NO=968.21) |
| A4-6 | m/z=705.88 (C₅₃H₃₉NO=705.30) | B2-13 | m/z=741. 89 (C₅₅H₃₅NO₂=741.89) |
| A4-17 | m/z=719.87 (C₅₃H₃₇NO₂=719.28) | B2-14 | m/z=817.99 (C₆₄H₃₉NO₂=817.99) |
| A4-32 | m/z=741.91 (C₅₆H₃₉NO=741.30) | D-5 | m/z=732. 04 (C₅₃H₁₃D₂₆NO=731.47) |
| A4-35 | m/z=629.79 (C₄₇H₃₅NO=629.27) | D-11 | m/z=728.02 (C₅₃H₁₇D₂₂NO=727.44) |
| A4-42 | m/z=735. 93 (C₅₃H₃₇NOS=735.26) | | |

### <Experimental Example>

### <Experimental Example 1>

### (1) Manufacture of Organic Light Emitting Device

A transparent ITO electrode thin film obtained from glass for an organic light emitting device (manufactured by Samsung-Corning Co., Ltd.) was ultrasonic cleaned using trichloroethylene, acetone, ethanol and distilled water consecutively for 5 minutes each, stored in isopropanol, and used. Next, the ITO substrate was installed in a substrate folder of a vacuum deposition apparatus, and the following 4,4',4"-tris(N,N-(2-naphthyl)-phenylamino)triphenylamine (2-TNATA) was introduced to a cell in the vacuum deposition apparatus.

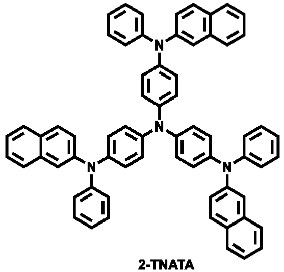

Subsequently, the chamber was evacuated until the degree of vacuum therein reached 10⁻⁶ torr, and then 2-TNATA was evaporated by applying a current to the cell to deposit a hole injection layer having a thickness of 600 Å on the ITO substrate. To another cell in the vacuum deposition apparatus, the following N,N'-bis(α-naphthyl)-N,N'-diphenyl-4,4'-diamine (NPB) was introduced, and evaporated by applying a current to the cell to deposit a hole transfer layer having a thickness of 300 Å on the hole injection layer.

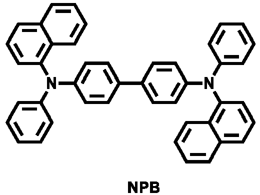

After forming the hole injection layer and the hole transfer layer as above, a blue light emitting material having a structure as below was deposited thereon as a light emitting layer. Specifically, in one side cell in the vacuum deposition apparatus, H1, a blue light emitting host material, was vacuum deposited to a thickness of 200 Å, and D1, a blue light emitting dopant material, was vacuum deposited thereon by 5 wt% with respect to the host material.

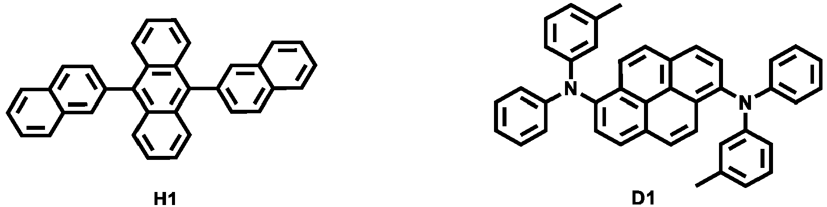

Subsequently, a compound of the following Structural Formula E1 was deposited to a thickness of 300 Å as an electron transfer layer.

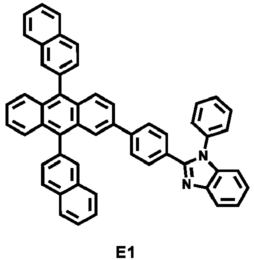

As an electron injection layer, lithium fluoride (LiF) was deposited to a thickness of 10 Å, and an Al cathode was employed to a thickness of 1,000 Å, and as a result, an organic light emitting device was manufactured (Comparative Example 1).

Meanwhile, all the organic compounds required to manufacture the OLED device were vacuum sublimation purified under 10⁻⁸ torr to 10⁻⁶ torr by each material to be used in the organic light emitting device manufacture.

Organic light emitting devices (Comparative Example 2 and Examples 1 to 75) were additionally manufactured in the same manner as in the manufacturing process of the organic light emitting device (Comparative Example 1) except that compounds described in the following Table 5 were used instead of NPB used when forming the hole transfer layer in the manufacturing process of the organic light emitting device (Comparative Example 1).

Compound (HTL1) used when forming the hole transfer layer in Comparative Example 2 is as follows.

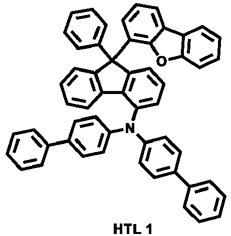

### 2) Driving Voltage and Light Emission Efficiency of Organic Light Emitting Device

For each of the organic light emitting devices (Comparative Examples 1 and 2 and Examples 1 to 75) manufactured as above, electroluminescent (EL) properties were measured using M7000 manufactured by McScience Inc., and with the measurement results, T₉₅ (unit: h, time), a time taken for luminance to become 95% with respect to initial luminance, was measured when standard luminance was 700 cd/m² through a lifetime measurement system (M6000) manufactured by McScience Inc..

Results of measuring driving voltage, light emission efficiency, color coordinate (CIE) and lifetime of the manufactured organic light emitting devices according to the above-described measurement method are as shown in the following Table 5.

**[Table 5]**

| | Compound | Driving Voltage (V) | Light Emission Efficiency (cd/A) | Lifetime (T95) |
|---|---|---|---|---|
| Example 1 | A1-1 | 4.76 | 6.33 | 68 |
| Example 2 | A1-2 | 4.82 | 6.23 | 63 |
| Example 3 | A1-6 | 4.61 | 6.56 | 57 |
| Example 4 | A1-10 | 4.58 | 6.98 | 58 |
| Example 5 | A1-11 | 4.69 | 6.89 | 67 |
| Example 6 | A1-13 | 4.67 | 6.55 | 66 |
| Example 7 | A1-14 | 4.83 | 6.68 | 60 |
| Example 8 | A1-30 | 4.94 | 6.84 | 67 |
| Example 9 | A1-41 | 4.62 | 6.53 | 63 |
| Example 10 | A1-47 | 4.75 | 6.48 | 55 |
| Example 11 | A1-51 | 4.78 | 6.82 | 52 |
| Example 12 | A2-1 | 4.90 | 6.73 | 56 |
| Example 13 | A2-2 | 4.82 | 6.79 | 62 |
| Example 14 | A2-6 | 4.73 | 6.43 | 64 |
| Example 15 | A2-10 | 4.88 | 6.84 | 72 |
| Example 16 | A2-11 | 4.56 | 6.97 | 75 |
| Example 17 | A2-13 | 4.80 | 6.63 | 64 |
| Example 18 | A2-14 | 4.85 | 6.82 | 57 |
| Example 19 | A2-19 | 4.79 | 6.75 | 56 |
| Example 20 | A2-38 | 4.75 | 6.77 | 63 |
| Example 21 | A2-39 | 4.66 | 6.44 | 62 |
| Example 22 | A2-48 | 4.59 | 6.58 | 72 |
| Example 23 | A2-54 | 4.60 | 6.80 | 58 |
| Example 24 | A3-2 | 4.62 | 6.72 | 61 |
| Example 25 | A3-3 | 4.81 | 6.50 | 55 |
| Example 26 | A3-4 | 4.63 | 6.22 | 54 |
| Example 27 | A3-14 | 4.70 | 6.11 | 54 |
| Example 28 | A3-31 | 4.73 | 6.23 | 59 |
| Example 29 | A3-33 | 4.84 | 6.30 | 62 |
| Example 30 | A3-34 | 4.72 | 6.98 | 67 |
| Example 31 | A3-36 | 4.69 | 6.89 | 59 |
| Example 32 | A3-49 | 4.78 | 6.95 | 62 |
| Example 33 | A4-2 | 4.92 | 6.93 | 64 |
| Example 34 | A4-6 | 4.64 | 6.84 | 59 |
| Example 35 | A4-17 | 4.75 | 6.91 | 53 |
| Example 36 | A4-32 | 4.88 | 6.35 | 67 |
| Example 37 | A4-35 | 4.73 | 6.44 | 60 |
| Example 38 | A4-42 | 4.81 | 6.51 | 58 |
| Example 39 | A4-44 | 4.70 | 6.30 | 63 |
| Example 40 | A4-52 | 4.74 | 6.43 | 56 |
| Example 41 | A5-4 | 4.66 | 6.54 | 55 |
| Example 42 | A5-15 | 4.88 | 6.73 | 64 |
| Example 43 | A5-19 | 4.56 | 6.81 | 72 |
| Example 44 | A5-29 | 4.80 | 6.85 | 67 |
| Example 45 | A5-43 | 4.85 | 6.75 | 68 |
| Example 46 | A5-48 | 4.88 | 6.82 | 57 |
| Example 47 | A5-51 | 4.72 | 6.49 | 53 |
| Example 48 | A6-4 | 4.66 | 6.66 | 66 |
| Example 49 | A6-5 | 4.55 | 6.40 | 64 |
| Example 50 | A6-15 | 4.60 | 6.42 | 56 |
| Example 51 | A6-30 | 4.62 | 6.47 | 56 |
| Example 52 | A6-37 | 4.83 | 6.39 | 61 |
| Example 53 | A6-39 | 4.73 | 6.74 | 53 |
| Example 54 | A6-40 | 4.77 | 6.87 | 54 |
| Example 55 | A6-50 | 4.87 | 6.61 | 57 |
| Example 56 | A7-2 | 4.67 | 6.80 | 55 |
| Example 57 | A7-29 | 4.55 | 6.75 | 61 |
| Example 58 | A7-41 | 4.62 | 6.82 | 69 |
| Example 59 | A7-49 | 4.68 | 6.44 | 54 |
| Example 60 | B1-1 | 5.05 | 6.09 | 47 |
| Example 61 | B1-2 | 5.10 | 6.10 | 49 |
| Example 62 | B1-6 | 4.98 | 5.88 | 55 |
| Example 63 | B1-10 | 4.80 | 6.01 | 49 |
| Example 64 | B1-11 | 5.15 | 5.84 | 54 |
| Example 65 | B1-13 | 5.08 | 5.99 | 51 |
| Example 66 | B1-14 | 4.97 | 6.03 | 51 |
| Example 67 | B2-1 | 5.10 | 6.11 | 53 |
| Example 68 | B2-2 | 5.08 | 6.12 | 56 |
| Example 69 | B2-6 | 4.99 | 6.03 | 48 |
| Example 70 | B2-10 | 5.20 | 6.05 | 54 |
| Example 71 | B2-11 | 5.01 | 6.10 | 50 |
| Example 72 | B2-13 | 4.86 | 6.17 | 57 |
| Example 73 | B2-14 | 4.91 | 6.12 | 52 |
| Example 74 | D-5 | 4.90 | 6.89 | 84 |
| Example 75 | D-11 | 4.62 | 6.81 | 78 |
| Comparative Example 1 | NPB | 6.01 | 5.43 | 38 |
| Comparative Example 2 | HTL1 | 5.22 | 5.66 | 45 |

As seen from the results of Table 5, Examples 1 to 75 that are organic light emitting devices using the compound represented by Chemical Formula 1 of the present disclosure as a material of the hole transfer layer had lower driving voltage and significantly improved light emission efficiency and lifetime compared to Comparative Examples 1 and 2 that are organic light emitting devices not using the compound represented by Chemical Formula 1 of the present disclosure as a material of the hole transfer layer.

When comparing NPB, the hole transfer layer material of Comparative Example 1, and the compound represented by Chemical Formula 1 of the present disclosure, the hole transfer layer material of Examples 1 to 75, having an arylamine group is similar, however, the compound represented by Chemical Formula 1 of the present disclosure is different in that the arylamine group is substituted with a substituent having a fluorenyl group and a dibenzofuran group linked. By the arylamine group being substituted with a substituent having a fluorenyl group and a dibenzofuran group linked as above, pi-pi stacking of the aromatic ring is suppressed, and this is considered to prevent device properties from declining by increasing a driving voltage of the organic light emitting device.

In addition, when comparing HTL1, the hole transfer layer material of Comparative Example 2, and the compound represented by Chemical Formula 1 of the present disclosure, the hole transfer layer material of Examples 1 to 75, having a substituent having a fluorene group and a dibenzofuran group linked as the arylamine group is similar, however, HTL1 of Comparative Example 2 and the compound represented by Chemical Formula 1 of the present disclosure are different in the position of substitution.

The amine group of the compound represented by Chemical Formula 1 of the present disclosure is substituted based on the dibenzofuran group, which leads to higher hole mobility compared to when the amine group is substituted based on the fluorene group as in HTL1 of Comparative Example 2, and by the amine group substituted based on the dibenzofuran group, an electron cloud distribution of the HOMO (Highest Occupied Molecular Orbital) of the compound of the present disclosure is distributed to the dibenzofuran group resulting in a proper energy level. As a result, it is considered that superior results are obtained in driving voltage, efficiency and lifetime by increasing a charge balance between holes and electrons in the light emitting layer.

### <Experimental Example 2>

### (1) Manufacture of Organic Light Emitting Device

A transparent ITO electrode thin film obtained from glass for an organic light emitting device (manufactured by Samsung-Corning Co., Ltd.) was ultrasonic cleaned using trichloroethylene, acetone, ethanol and distilled water consecutively for 5 minutes each, stored in isopropanol, and used. Next, the ITO substrate was installed in a substrate folder of a vacuum deposition apparatus, and the following 4,4',4"-tris(N,N-(2-naphthyl)-phenylamino)triphenylamine (2-TNATA) was introduced to a cell in the vacuum deposition apparatus.

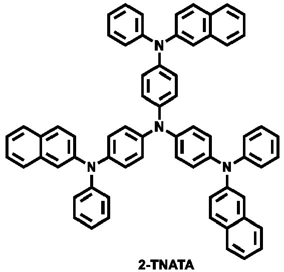

Subsequently, the chamber was evacuated until the degree of vacuum therein reached 10⁻⁶ torr, and then 2-TNATA was evaporated by applying a current to the cell to deposit a hole injection layer having a thickness of 600 Å on the ITO substrate. To another cell in the vacuum deposition apparatus, the following N,N'-bis(α-naphthyl)-N,N'-diphenyl-4,4'-diamine (NPB) was introduced, and evaporated by applying a current to the cell to deposit a hole transfer layer having a thickness of 300 Å on the hole injection layer.

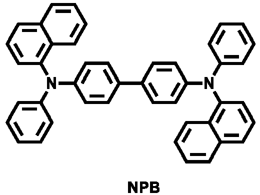

After forming the hole injection layer and the hole transfer layer as above, a blue light emitting material having a structure as below was deposited thereon as a light emitting layer. Specifically, in one side cell in the vacuum deposition apparatus, H1, a blue light emitting host material, was vacuum deposited to a thickness of 200 Å, and D1, a blue light emitting dopant material, was vacuum deposited thereon by 5% with respect to the host material.

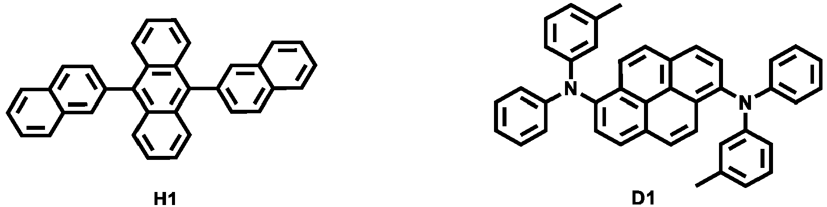

Subsequently, a compound of the following Structural Formula E1 was deposited to a thickness of 300 Å as an electron transfer layer.

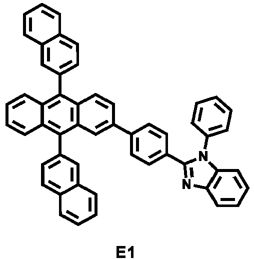

As an electron injection layer, lithium fluoride (LiF) was deposited to a thickness of 10 Å, and an Al cathode was employed to a thickness of 1,000 Å, and as a result, an organic light emitting device was manufactured (Comparative Example 3).

Meanwhile, all the organic compounds required to manufacture the OLED device were vacuum sublimation purified under 10⁻⁸ torr to 10⁻⁶ torr by each material to be used in the organic light emitting device manufacture.

Organic electroluminescent devices (Examples 76 to 150 and Comparative Example 4) were additionally manufactured in the same manner as in the manufacturing process of the organic light emitting device (Comparative Example 3) except that, after forming the hole transfer layer NPB to a thickness of 250 Å, an electron blocking layer having a thickness of 50 Å was formed on the hole transfer layer using compounds shown in the following Table 6.

### 2) Driving Voltage and Light Emission Efficiency of Organic Light Emitting Device

For each of the organic light emitting devices (Comparative Examples 3 and 4 and Examples 76 to 150) manufactured as above, electroluminescent (EL) properties were measured using M7000 manufactured by McScience Inc., and with the measurement results, T₉₅ (unit: h, time), a time taken for luminance to become 95% with respect to initial luminance, was measured when standard luminance was 700 cd/m² through a lifetime measurement system (M6000) manufactured by McScience Inc..

Results of measuring driving voltage, light emission efficiency, color coordinate (CIE) and lifetime of the manufactured organic light emitting devices according to the above-described measurement method are as shown in the following Table 6.

**[Table 6]**

| | Compound | Driving Voltage (V) | Light Emission Efficiency (cd/A) | Lifetime (T95) |
|---|---|---|---|---|
| Example 76 | A1-1 | 4.57 | 6.67 | 63 |
| Example 77 | A1-2 | 4.66 | 6.81 | 68 |
| Example 78 | A1-6 | 4.97 | 6.93 | 62 |
| Example 79 | A1-10 | 4.52 | 6.60 | 66 |
| Example 80 | A1-11 | 4.52 | 6.77 | 61 |
| Example 81 | A1-13 | 4.70 | 6.54 | 60 |
| Example 82 | A1-14 | 4.69 | 6.41 | 67 |
| Example 83 | A1-30 | 4.81 | 6.89 | 62 |
| Example 84 | A1-41 | 4.75 | 6.50 | 74 |
| Example 85 | A1-47 | 4.58 | 6.74 | 59 |
| Example 86 | A1-51 | 4.62 | 6.32 | 62 |
| Example 87 | A2-1 | 4.76 | 6.54 | 66 |
| Example 88 | A2-2 | 4.69 | 6.48 | 64 |
| Example 89 | A2-6 | 4.75 | 6.35 | 72 |
| Example 90 | A2-10 | 4.82 | 6.57 | 58 |
| Example 91 | A2-11 | 4.58 | 7.03 | 57 |
| Example 92 | A2-13 | 4.54 | 6.93 | 60 |
| Example 93 | A2-14 | 4.68 | 6.85 | 59 |
| Example 94 | A2-19 | 4.47 | 6.72 | 57 |
| Example 95 | A2-38 | 4.76 | 6.94 | 55 |
| Example 96 | A2-39 | 4.73 | 6.48 | 63 |
| Example 97 | A2-48 | 4.55 | 6.79 | 69 |
| Example 98 | A2-54 | 4.52 | 6.59 | 68 |
| Example 99 | A3-2 | 4.73 | 6.93 | 67 |
| Example 100 | A3-3 | 4.69 | 6.88 | 75 |
| Example 101 | A3-4 | 4.77 | 6.74 | 61 |
| Example 102 | A3-14 | 4.75 | 6.58 | 64 |
| Example 103 | A3-31 | 4.62 | 6.38 | 69 |
| Example 104 | A3-33 | 4.62 | 6.67 | 65 |
| Example 105 | A3-34 | 4.74 | 6.74 | 63 |
| Example 106 | A3-36 | 4.69 | 6.91 | 72 |
| Example 107 | A3-49 | 4.55 | 6.65 | 70 |
| Example 108 | A4-2 | 4.66 | 6.64 | 64 |
| Example 109 | A4-6 | 4.56 | 6.52 | 65 |
| Example 110 | A4-17 | 4.54 | 6.47 | 61 |
| Example 111 | A4-32 | 4.70 | 6.59 | 69 |
| Example 112 | A4-35 | 4.81 | 6.90 | 67 |
| Example 113 | A4-42 | 4.76 | 6.83 | 66 |
| Example 114 | A4-44 | 4.97 | 6.55 | 71 |
| Example 115 | A4-52 | 4.77 | 6.52 | 63 |
| Example 116 | A5-4 | 4.48 | 6.47 | 61 |
| Example 117 | A5-15 | 4.70 | 6.43 | 63 |
| Example 118 | A5-19 | 4.69 | 7.00 | 67 |
| Example 119 | A5-29 | 4.63 | 6.82 | 66 |
| Example 120 | A5-43 | 4.75 | 6.93 | 73 |
| Example 121 | A5-48 | 4.68 | 6.97 | 64 |
| Example 122 | A5-51 | 4.61 | 6.81 | 63 |
| Example 123 | A6-4 | 4.76 | 6.62 | 67 |
| Example 124 | A6-5 | 4.66 | 6.85 | 60 |
| Example 125 | A6-15 | 4.75 | 6.81 | 62 |
| Example 126 | A6-30 | 4.75 | 6.77 | 67 |
| Example 127 | A6-37 | 4.58 | 6.71 | 70 |
| Example 128 | A6-39 | 4.52 | 6.52 | 62 |
| Example 129 | A6-40 | 4.69 | 6.44 | 60 |
| Example 130 | A6-50 | 4.79 | 6.58 | 66 |
| Example 131 | A7-2 | 4.66 | 6.58 | 69 |
| Example 132 | A7-29 | 4.82 | 6.54 | 62 |
| Example 133 | A7-41 | 4.62 | 6.51 | 69 |
| Example 134 | A7-49 | 4.55 | 6.63 | 61 |
| Example 135 | B1-1 | 5.10 | 6.31 | 55 |
| Example 136 | B1-2 | 4.96 | 6.24 | 56 |
| Example 137 | B1-6 | 4.97 | 6.23 | 52 |
| Example 138 | B1-10 | 4.82 | 6.30 | 59 |
| Example 139 | B1-11 | 5.12 | 6.37 | 61 |
| Example 140 | B1-13 | 5.03 | 6.24 | 61 |
| Example 141 | B1-14 | 5.11 | 6.11 | 57 |
| Example 142 | B2-1 | 4.97 | 6.39 | 62 |
| Example 143 | B2-2 | 5.05 | 6.40 | 54 |
| Example 144 | B2-6 | 4.98 | 6.31 | 56 |
| Example 145 | B2-10 | 5.11 | 6.12 | 59 |
| Example 146 | B2-11 | 4.96 | 6.14 | 63 |
| Example 147 | B2-13 | 5.01 | 6.22 | 54 |
| Example 148 | B2-14 | 5.04 | 6.35 | 57 |
| Example 149 | D-5 | 4.65 | 6.70 | 87 |
| Example 150 | D-11 | 4.50 | 6.57 | 82 |
| Comparative Example 3 | NPB | 5.27 | 5.88 | 48 |
| Comparative Example 4 | HTL1 | 5.22 | 6.01 | 50 |

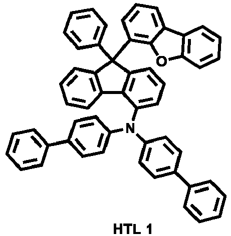

As seen from the results of Table 6, Examples 76 to 150 that are organic light emitting devices using the compound represented by Chemical Formula 1 of the present disclosure as a material of the electron blocking layer had lower driving voltage and significantly improved light emission efficiency and lifetime compared to Comparative Examples 3 and 4 that are organic light emitting devices using NPB and HTL1 compounds as the electron blocking layer material.

Generally, when electrons pass through a hole transfer layer and migrate to an anode without binding in a light emitting layer, efficiency and lifetime of an organic light emitting device are reduced. Herein, when using a compound having a high LUMO (Lowest Unoccupied Molecular Orbital) level as an electron blocking layer, electrons to migrate to an anode after passing through a light emitting layer are blocked by an energy barrier of the electron blocking layer, and such a phenomenon of reducing efficiency and lifetime of the organic light emitting device may be prevented. In other words, when a compound having a high LUMO (Lowest Unoccupied Molecular Orbital) level as an electron blocking layer, holes and electrons are more likely to form excitons, which increases possibility of being emitted as light in the light emitting layer.

Accordingly, by the compound of the present disclosure having a higher LUMO level and a wider band gap compared to the compounds of Comparative Examples 3 and 4, an excellent electron blocking ability is obtained when using the compound of the present disclosure as the electron blocking layer of the organic light emitting device, and it is considered that superior results are obtained in all aspects of driving, efficiency and lifetime by holes and electrons forming a charge balance, and thereby emitting light inside the light emitting layer instead of at an interface of the hole transfer layer.

### <Reference Numeral>

- 100:: Substrate
- 200:: Anode
- 300:: Organic Material Layer
- 301:: Hole Injection Layer
- 302:: Hole Transfer Layer
- 303:: Light Emitting Layer
- 304:: Hole Blocking Layer
- 305:: Electron Transfer Layer
- 306:: Electron Injection Layer
- 400:: Cathode

## Claims

1. A hetero-cyclic compound represented by the following Chemical Formula 1: 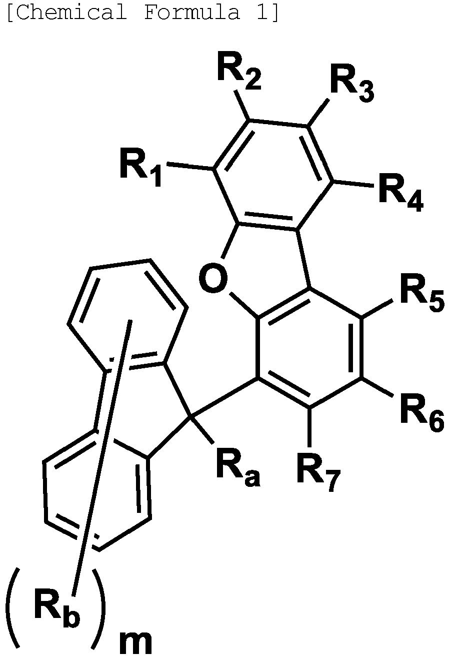 wherein, in Chemical Formula 1,
Rₐ is a halogen group; a cyano group; a substituted or unsubstituted alkyl group having 1 to 60 carbon atoms; or a substituted or unsubstituted aryl group having 6 to 60 carbon atoms;
R_{b} is hydrogen; deuterium; a halogen group; a cyano group; a substituted or unsubstituted alkyl group having 1 to 60 carbon atoms; a substituted or unsubstituted aryl group having 6 to 60 carbon atoms; or a substituted or unsubstituted heteroaryl group having 2 to 60 carbon atoms;
when Rₐ is a substituted or unsubstituted aryl group having 6 to 60 carbon atoms, only one of R₁, R₂ and R₄ to R₇ is -(La)p-A, and the rest of R₁ to R₇ are the same as or different from each other and each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms;
when Rₐ is a halogen group; a cyano group; or a substituted or unsubstituted alkyl group having 1 to 60 carbon atoms, only one of R₁ to R₇ is -(La)p-A, and the rest of R₁ to R₇ are the same as or different from each other and each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms;
La is a direct bond; a substituted or unsubstituted arylene group having 6 to 60 carbon atoms or a substituted or unsubstituted heteroarylene group having 2 to 60 carbon atoms, p is an integer of 0 to 3, and when p is 2 or greater, Las are the same as or different from each other;
A is a substituted or unsubstituted amine group; and
m is an integer of 0 to 8, and when m is 2 or greater, R_{b}s are the same as or different from each other.

2. The hetero-cyclic compound of Claim 1, wherein the substituted or unsubstituted means being substituted with one or more substituents selected from the group consisting of deuterium; a halogen group; a cyano group; linear or branched alkyl having 1 to 60 carbon atoms; linear or branched alkenyl having 2 to 60 carbon atoms; linear or branched alkynyl having 2 to 60 carbon atoms; monocyclic or polycyclic cycloalkyl having 3 to 60 carbon atoms; monocyclic or polycyclic heterocycloalkyl having 2 to 60 carbon atoms; monocyclic or polycyclic aryl having 6 to 60 carbon atoms; monocyclic or polycyclic heteroaryl having 2 to 60 carbon atoms; -SiRR'R"; -P(=O)RR'; alkylamine having 1 to 20 carbon atoms; monocyclic or polycyclic arylamine having 6 to 60 carbon atoms; and monocyclic or polycyclic heteroarylamine having 2 to 60 carbon atoms, or being unsubstituted, or being substituted with a substituent linking two or more substituents selected from among the substituents illustrated above, or being unsubstituted, and R, R' and R" are the same as or different from each other and each independently hydrogen; deuterium; halogen; substituted or unsubstituted alkyl having 1 to 60 carbon atoms; substituted or unsubstituted aryl having 6 to 60 carbon atoms; or substituted or unsubstituted heteroaryl having 2 to 60 carbon atoms.

3. The hetero-cyclic compound of Claim 1, wherein Chemical Formula 1 is represented by any one of the following Chemical Formulae 2 to 7: 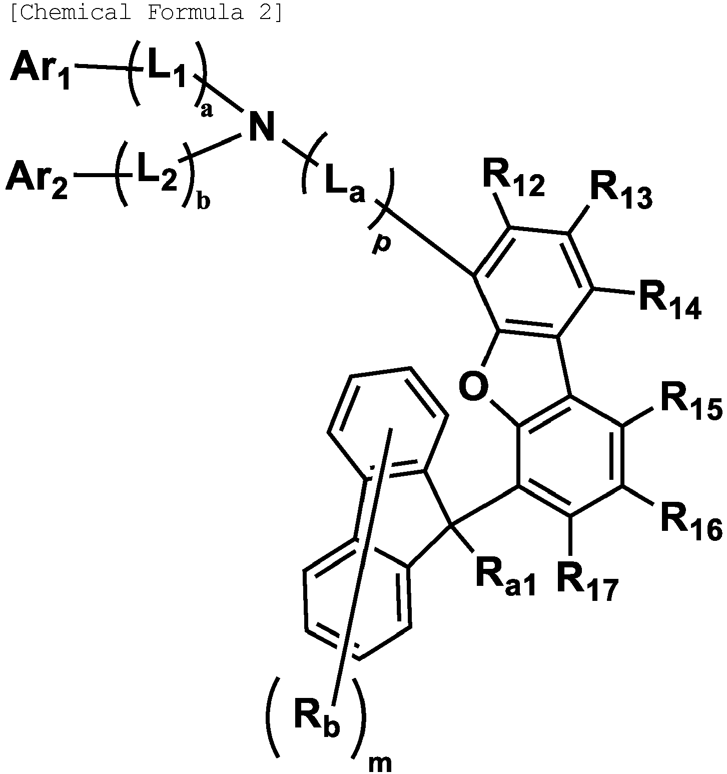 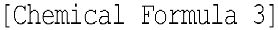 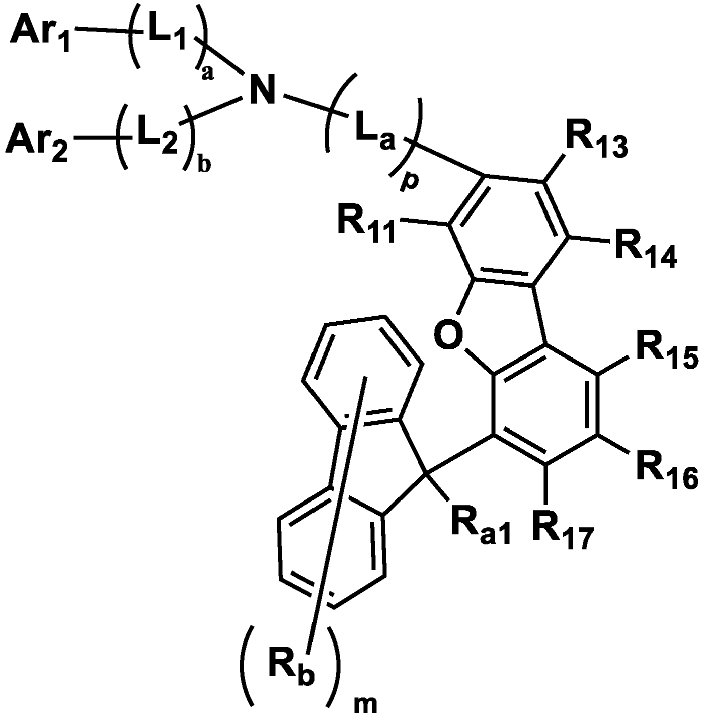 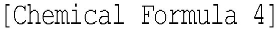 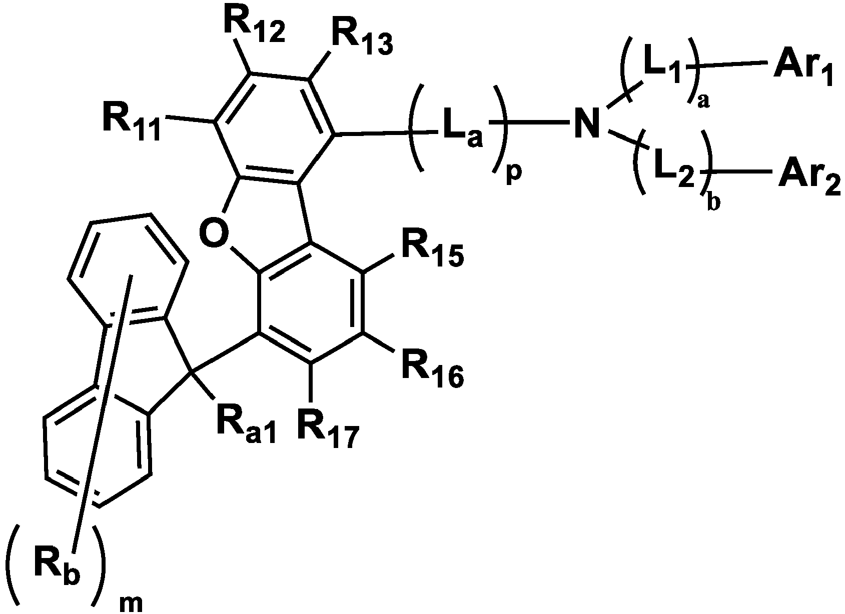 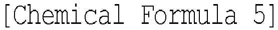 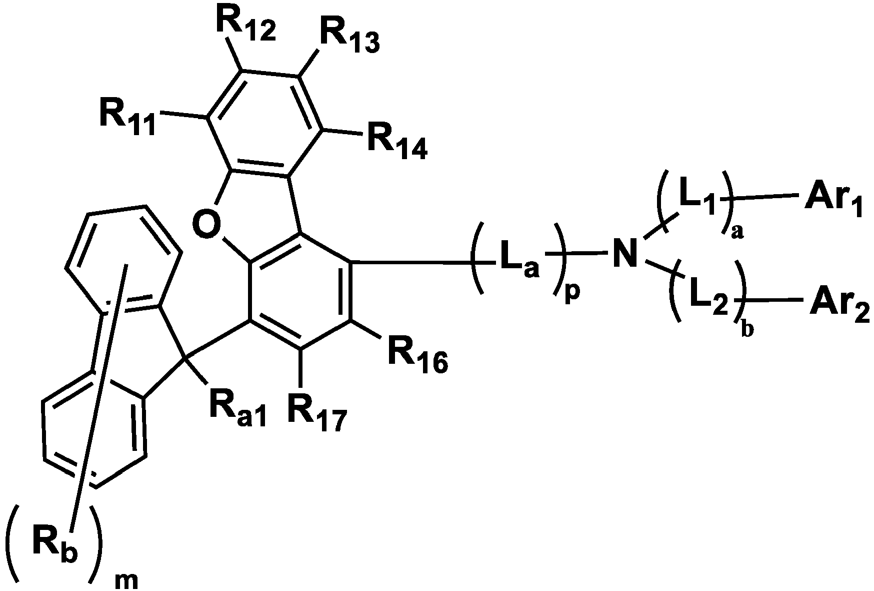 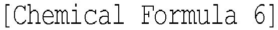 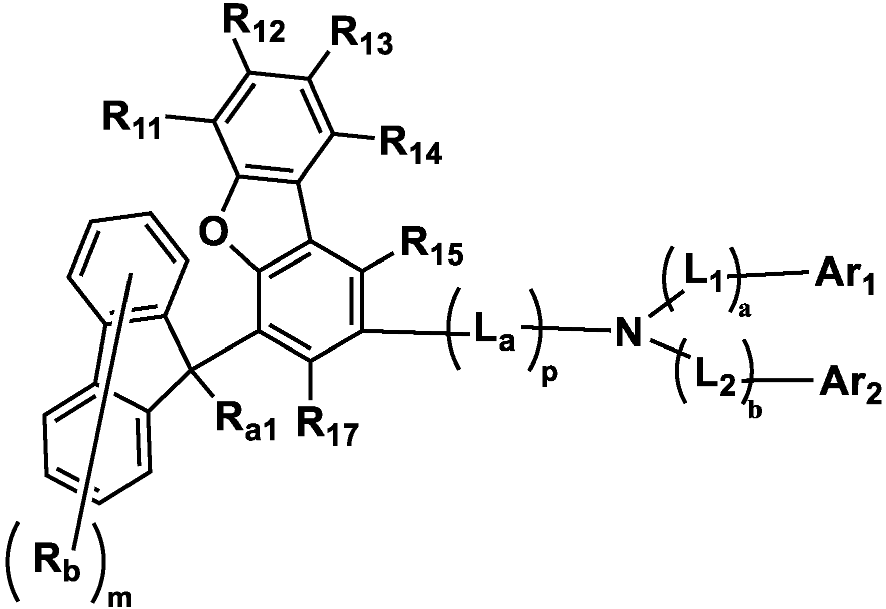 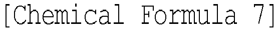 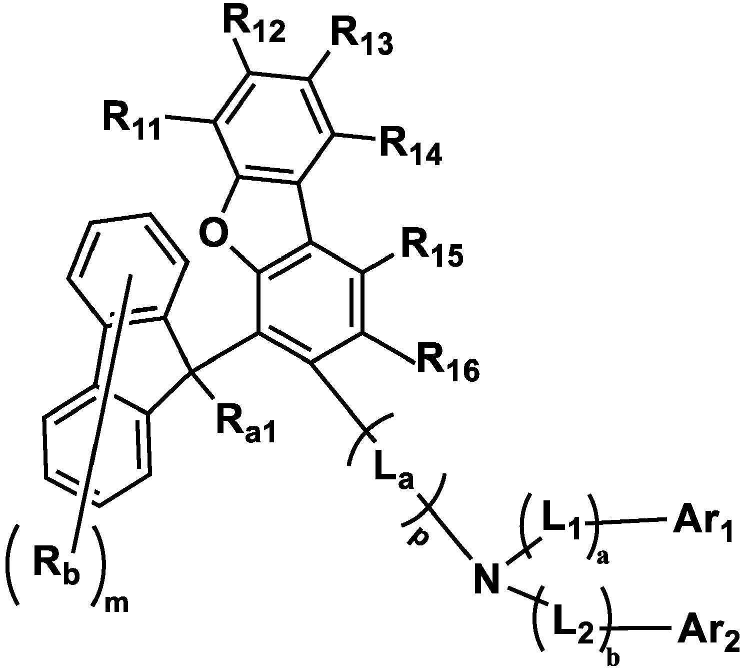 in Chemical Formulae 2 to 7,
Rₐ₁ is a substituted or unsubstituted aryl group having 6 to 60 carbon atoms;
L₁ and L₂ are the same as or different from each other, and each independently a direct bond; a substituted or unsubstituted arylene group having 6 to 60 carbon atoms or a substituted or unsubstituted heteroarylene group having 2 to 60 carbon atoms;
Ar₁ and Ar₂ are the same as or different from each other, and each independently a halogen group; a cyano group; a substituted or unsubstituted alkyl group having 1 to 60 carbon atoms; a substituted or unsubstituted aryl group having 6 to 60 carbon atoms or a substituted or unsubstituted heteroaryl group having 2 to 60 carbon atoms;
R₁₁ to R₁₇ are the same as or different from each other, and each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms; and
a and b are 0 or 1, and Lₐ, p, R_{b} and m have the same definitions as in Chemical Formula 1.

4. The hetero-cyclic compound of Claim 1, wherein Chemical Formula 1 is represented by any one of the following Chemical Formulae 8 to 14: 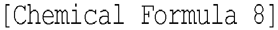 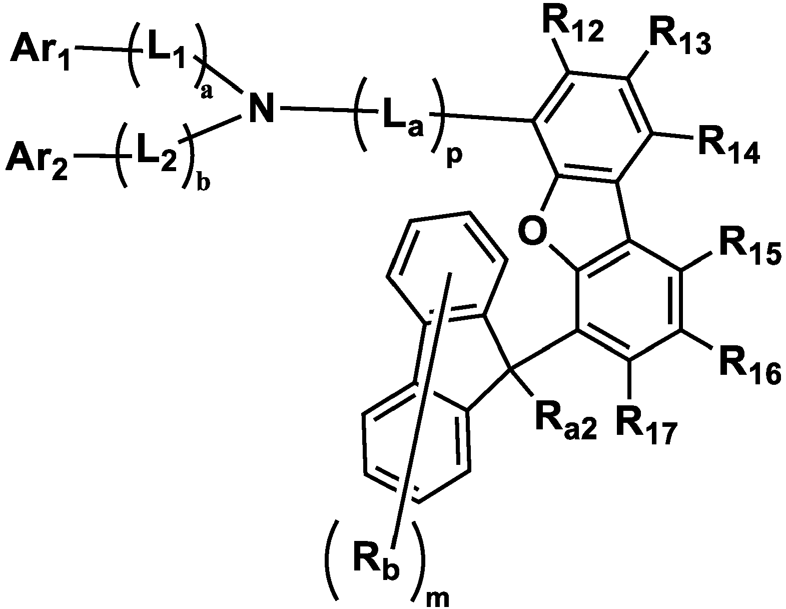 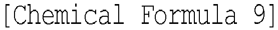 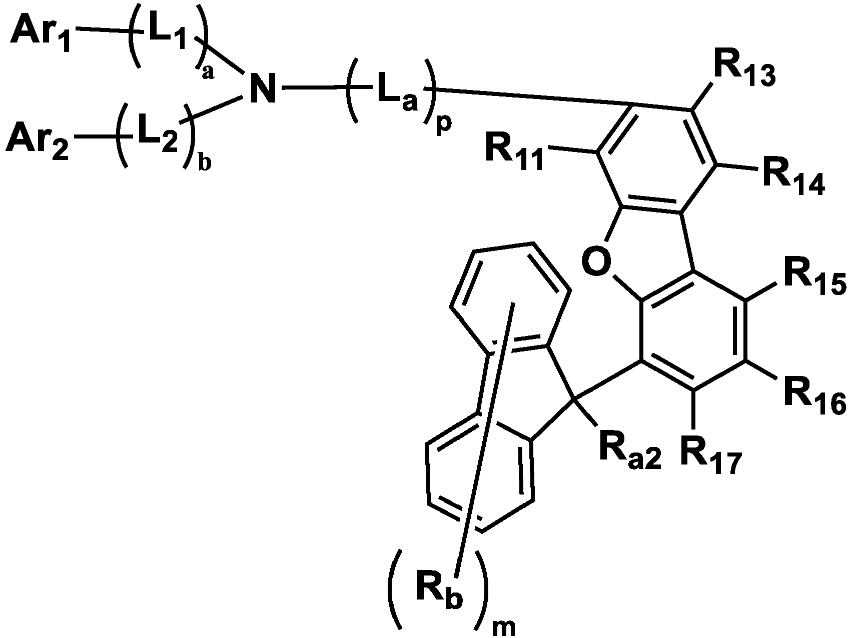 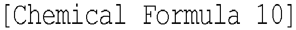 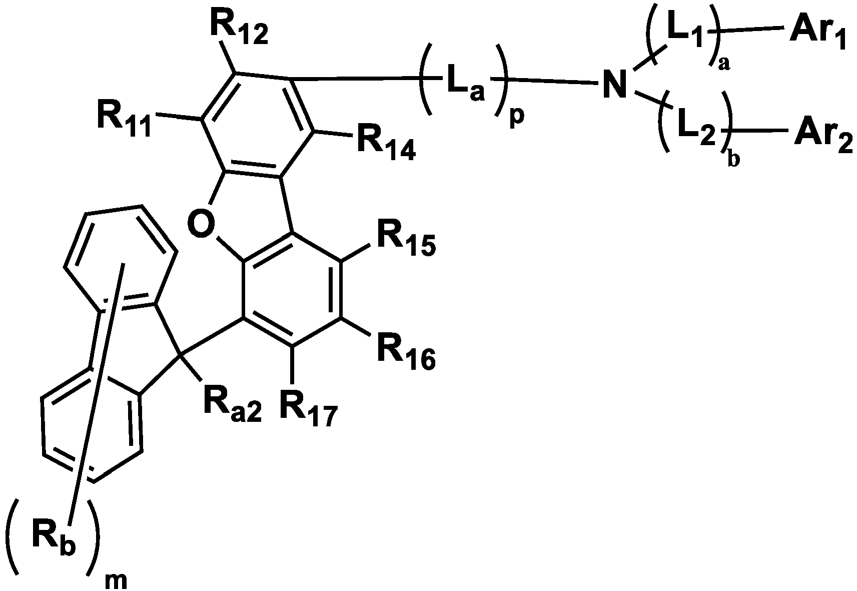 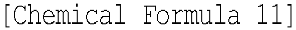 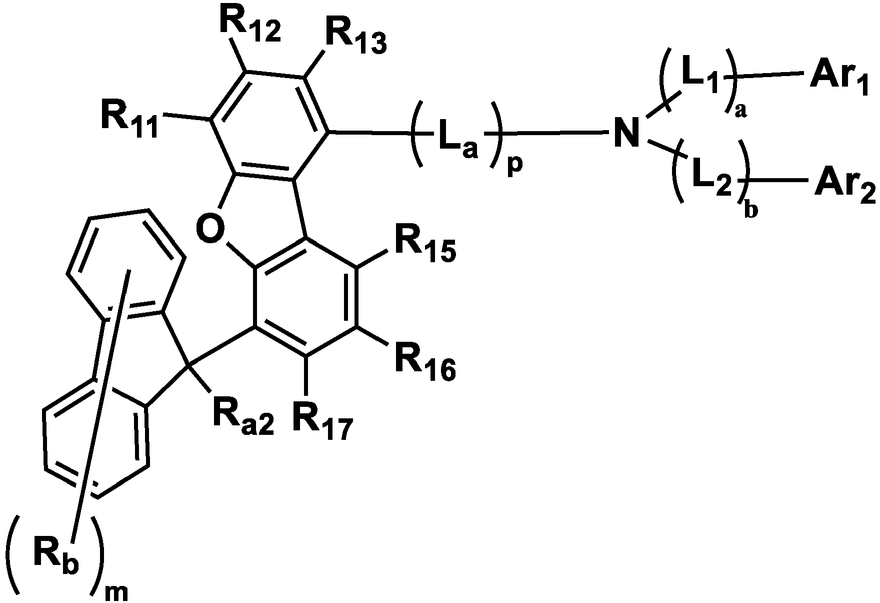 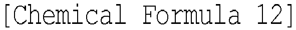 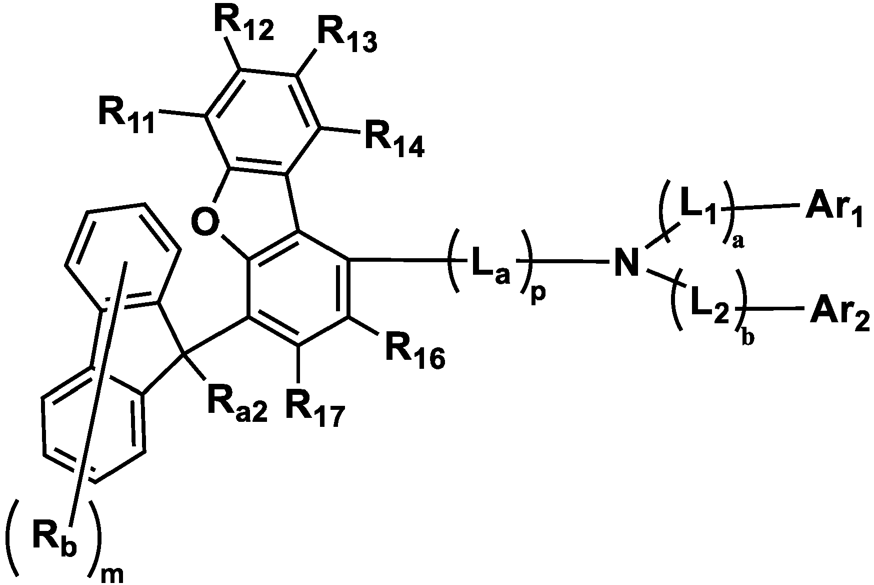 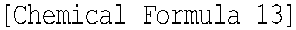 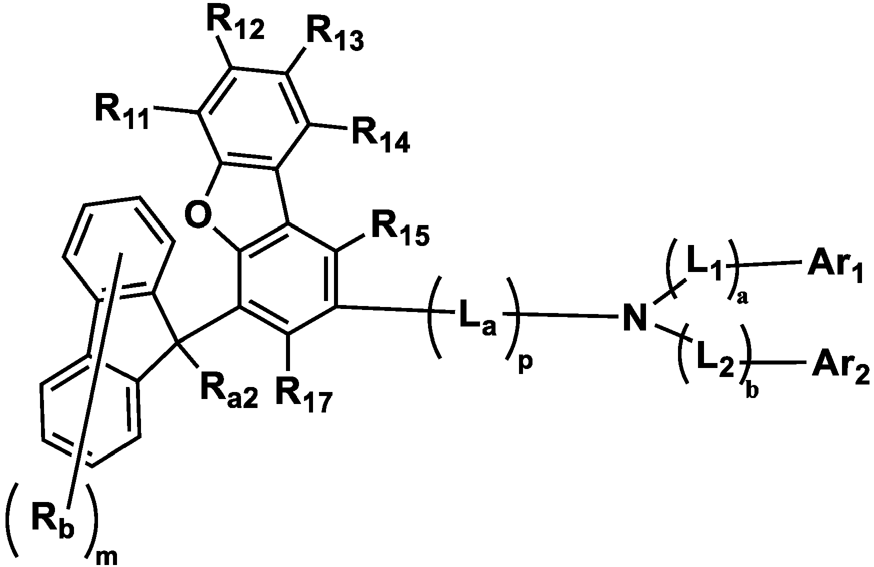 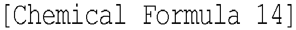 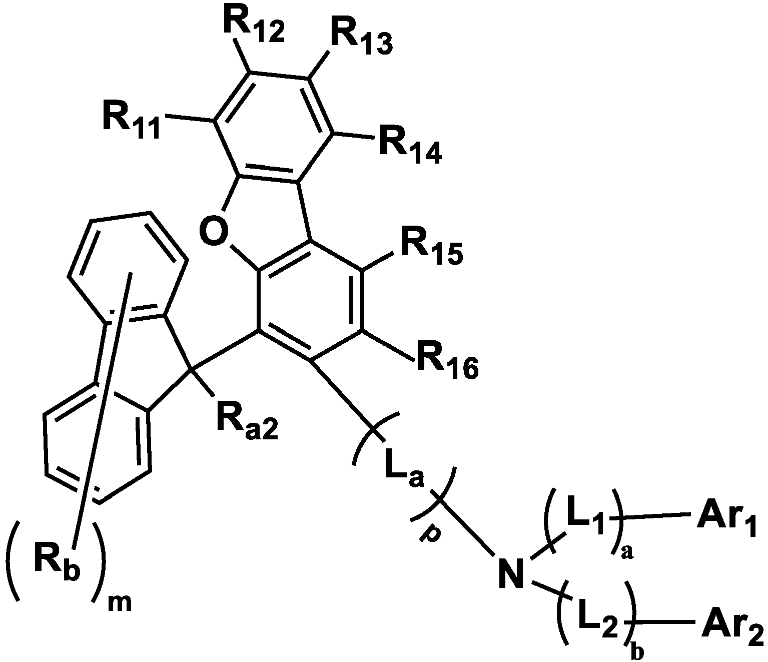 in Chemical Formulae 8 to 14,
Rₐ₂ is a halogen group; a cyano group; or a substituted or unsubstituted alkyl group having 1 to 60 carbon atoms, and L₁ and L₂ are the same as or different from each other and each independently a direct bond; a substituted or unsubstituted arylene group having 6 to 60 carbon atoms or a substituted or unsubstituted heteroarylene group having 2 to 60 carbon atoms;
Ar₁ and Ar₂ are the same as or different from each other, and each independently a halogen group; a cyano group; a substituted or unsubstituted alkyl group having 1 to 60 carbon atoms; a substituted or unsubstituted aryl group having 6 to 60 carbon atoms or a substituted or unsubstituted heteroaryl group having 2 to 60 carbon atoms;
R₁₁ to R₁₇ are the same as or different from each other, and each independently selected from the group consisting of hydrogen; deuterium; a halogen group; a cyano group; a substituted or unsubstituted alkyl group having 1 to 60 carbon atoms; a substituted or unsubstituted aryl group having 6 to 60 carbon atoms; and a substituted or unsubstituted heteroaryl group having 2 to 60 carbon atoms; and
a and b are 0 or 1, and Lₐ, p, R_{b} and m have the same definitions as in Chemical Formula 1.

5. The hetero-cyclic compound of Claim 1, wherein the compound represented by Chemical Formula 1 has a deuterium content of greater than or equal to 10% and less than or equal to 100%.

6. The hetero-cyclic compound of Claim 1, wherein Chemical Formula 1 is represented by any one of the following compounds:

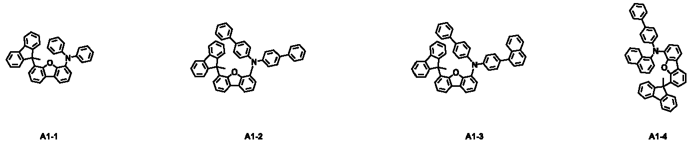

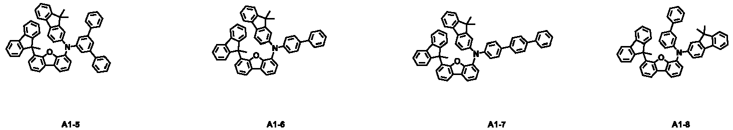

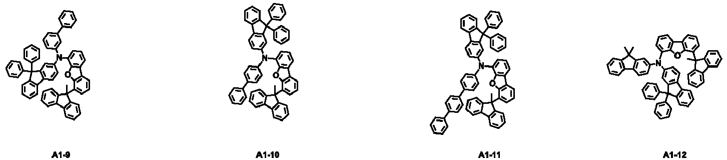

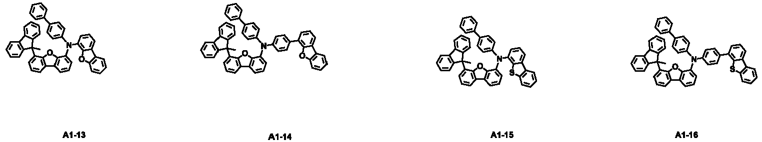

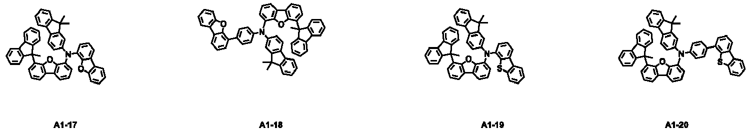

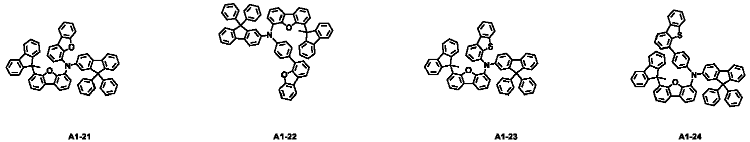

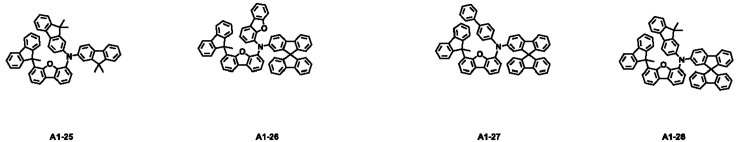

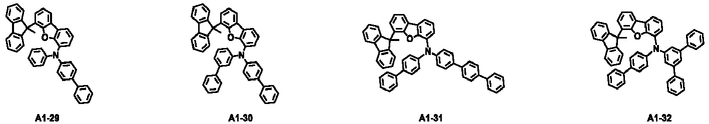

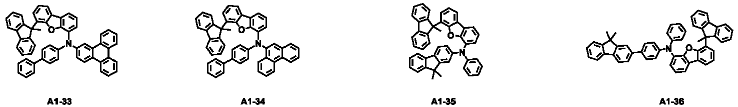

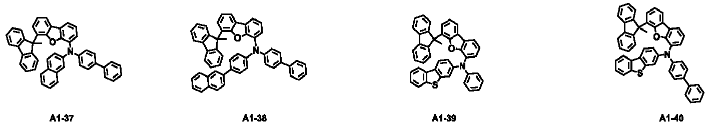

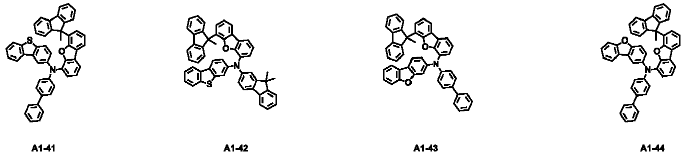

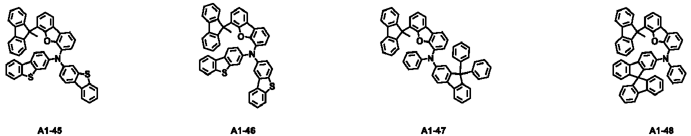

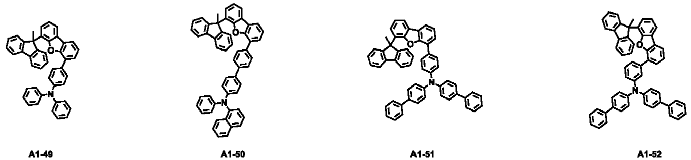

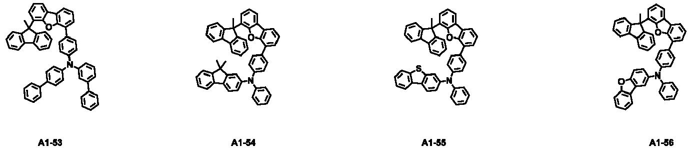

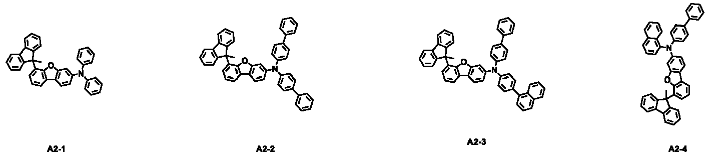

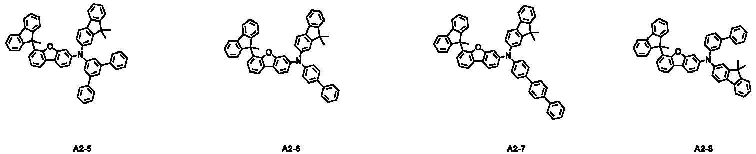

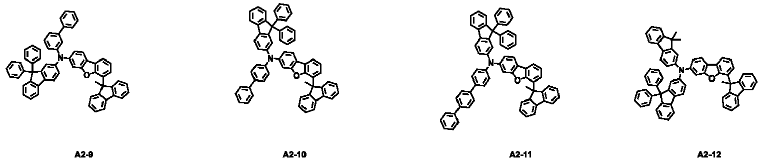

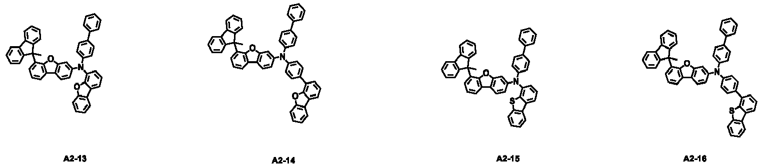

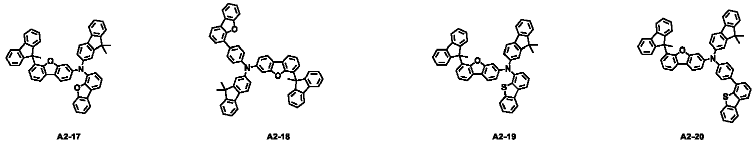

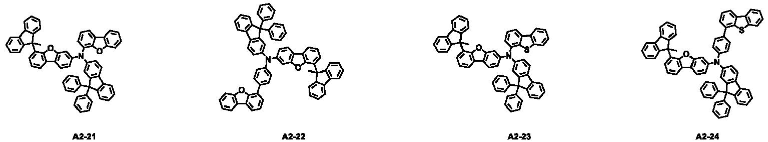

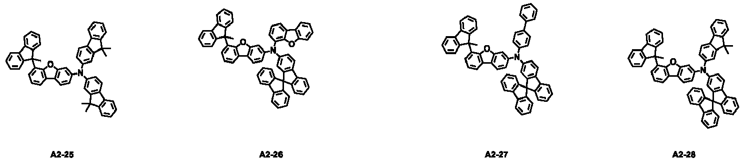

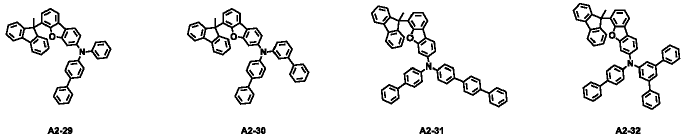

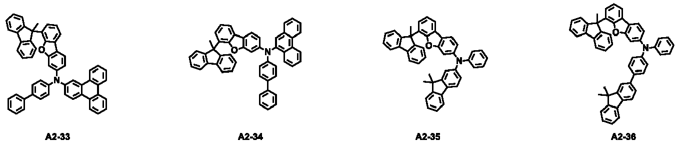

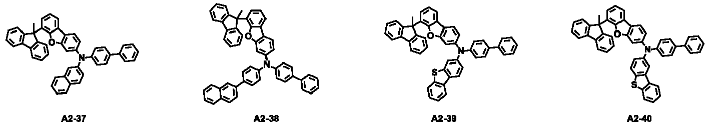

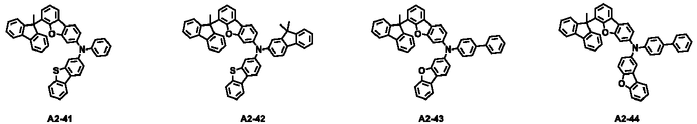

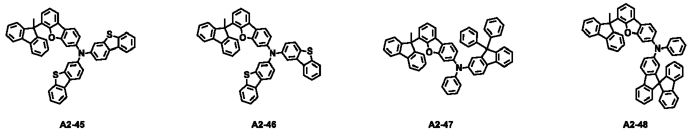

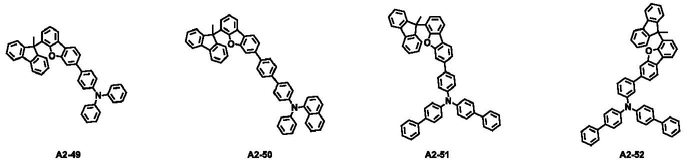

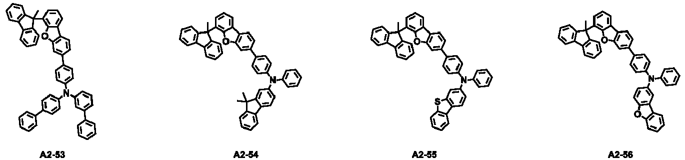

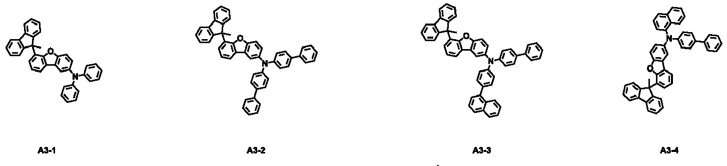

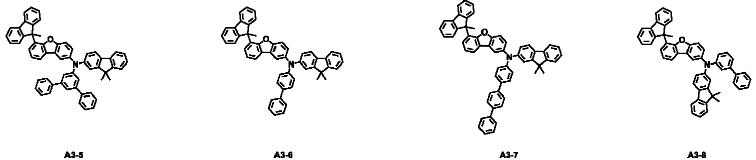

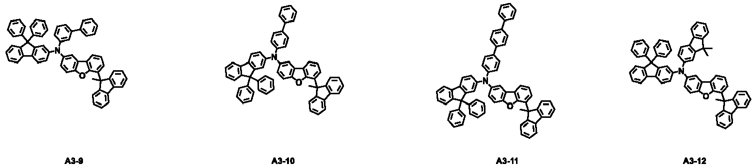

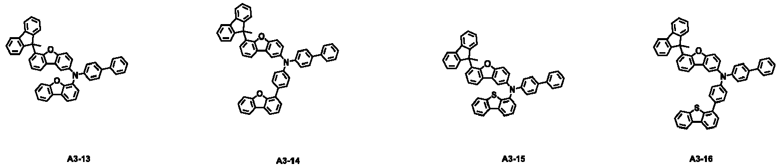

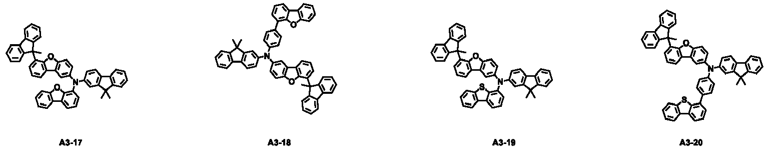

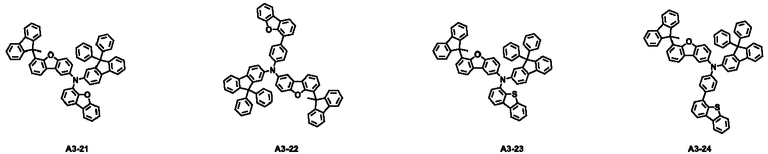

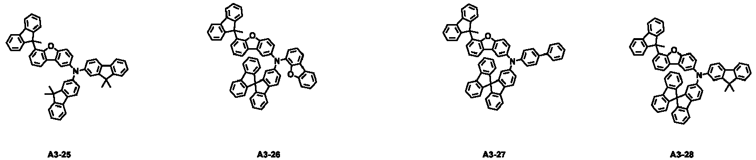

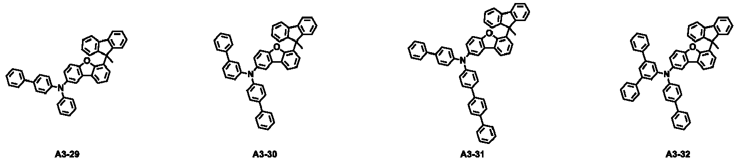

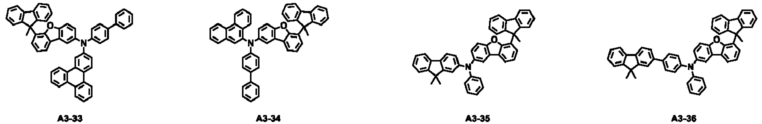

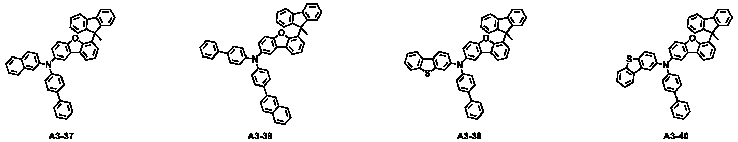

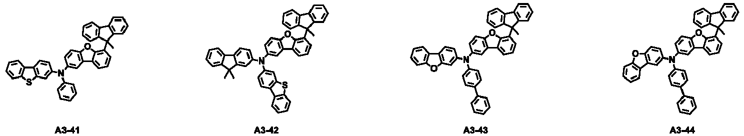

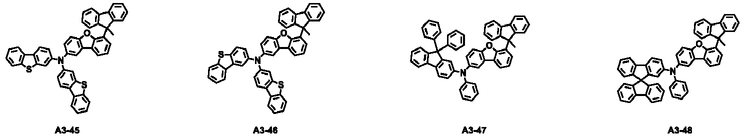

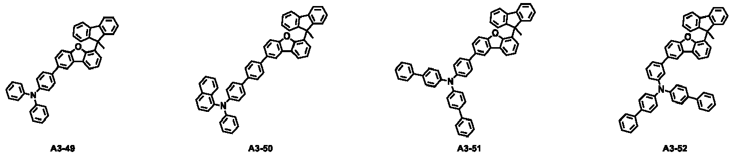

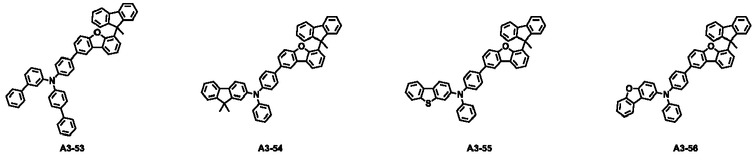

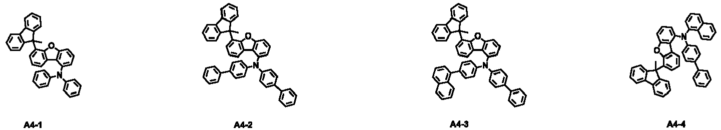

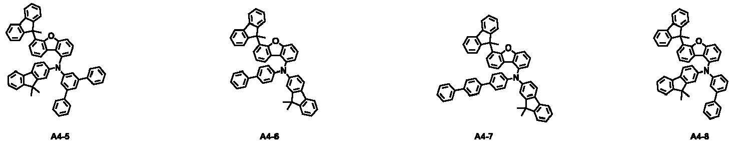

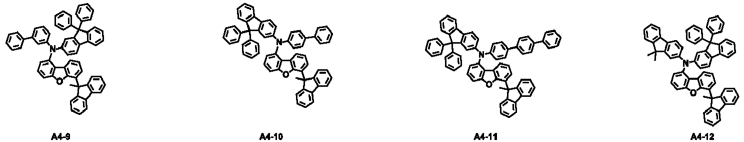

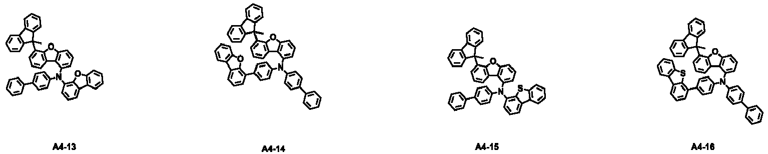

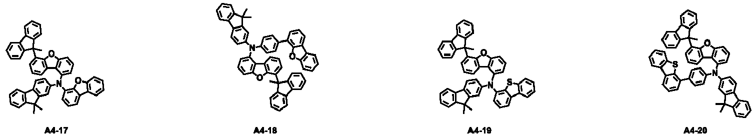

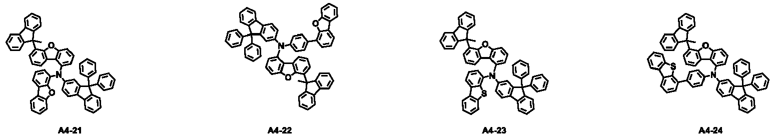

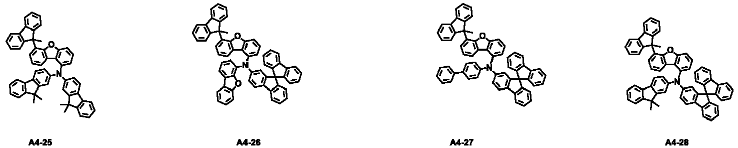

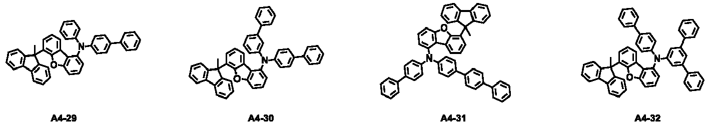

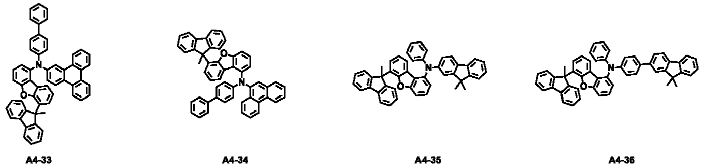

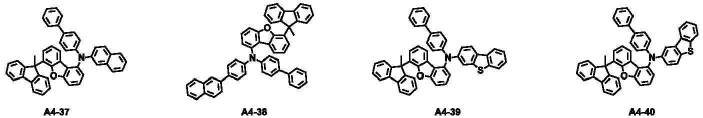

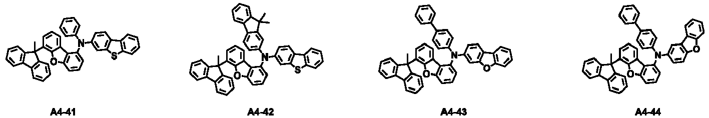

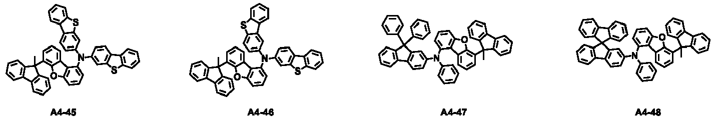

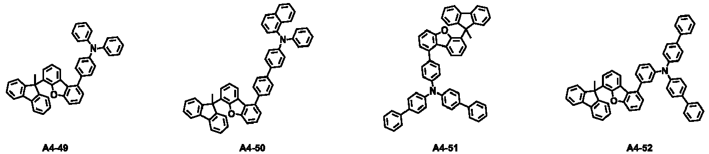

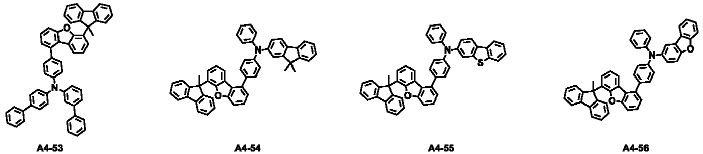

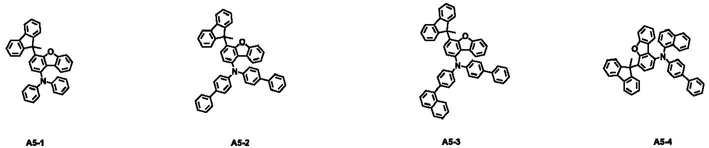

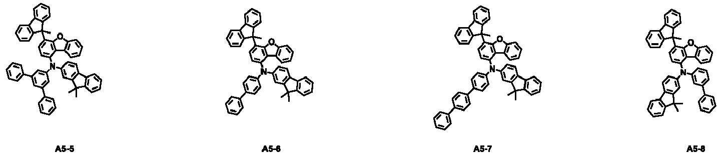

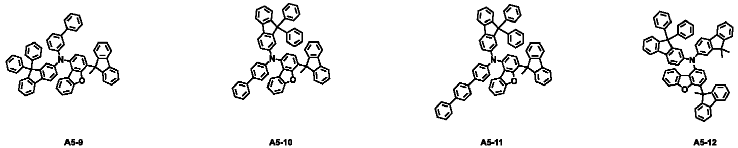

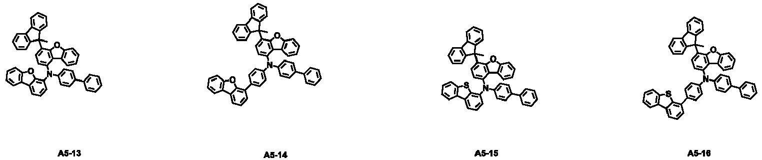

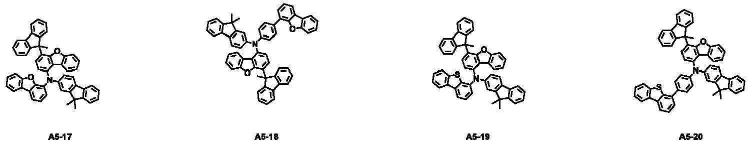

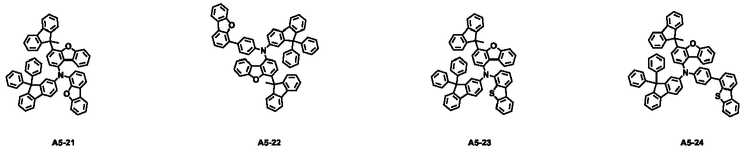

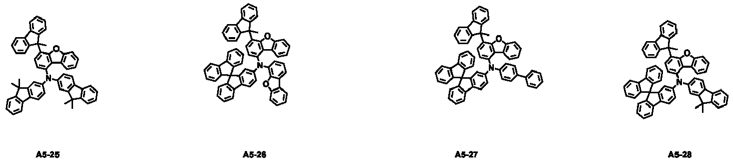

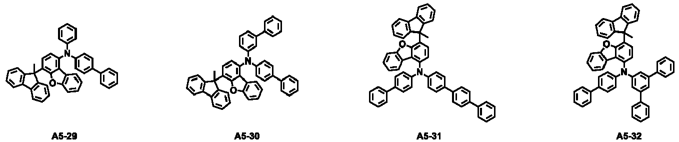

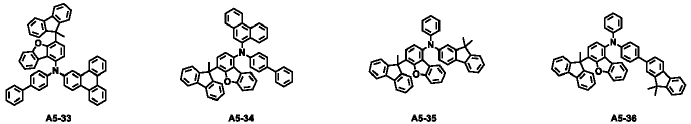

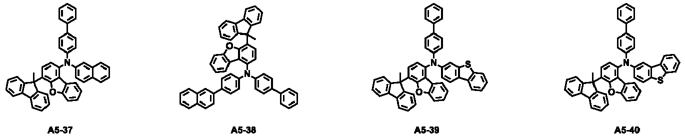

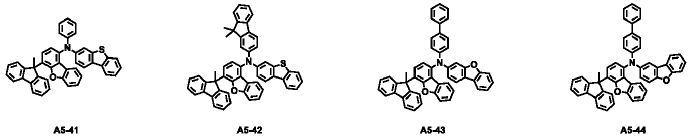

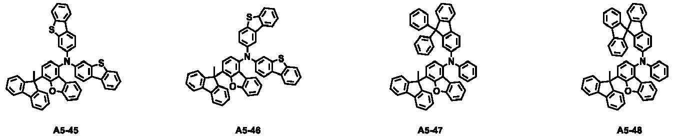

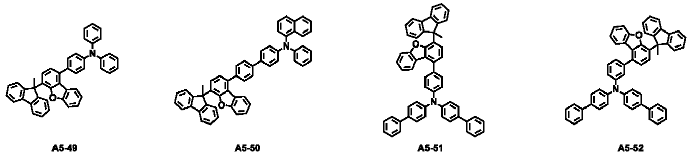

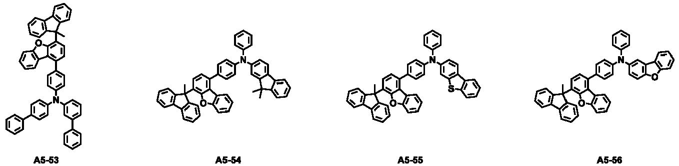

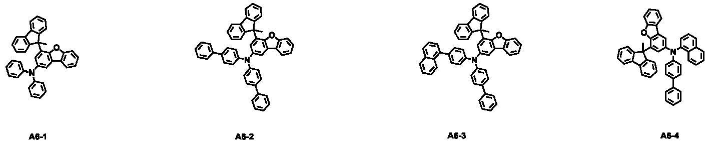

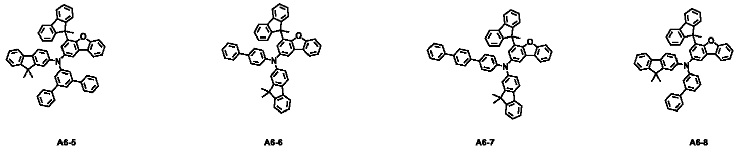

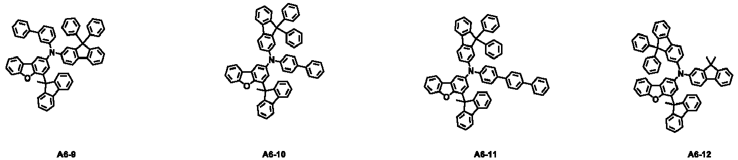

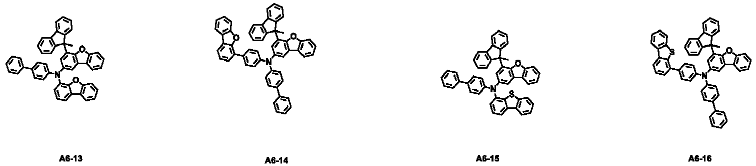

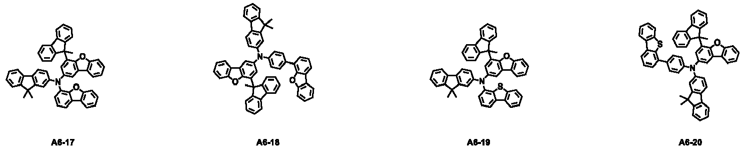

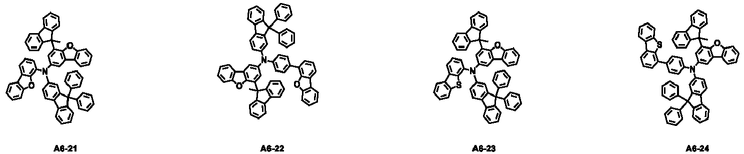

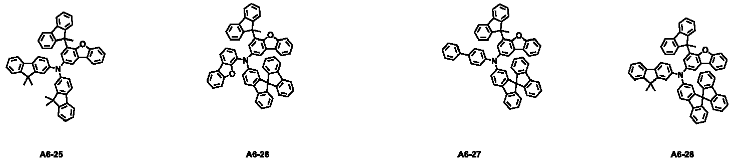

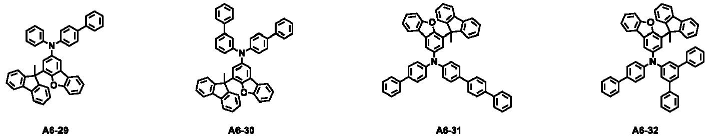

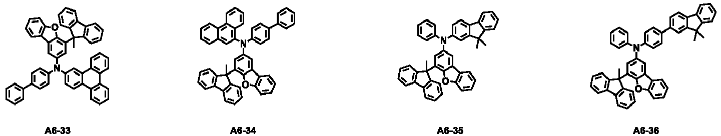

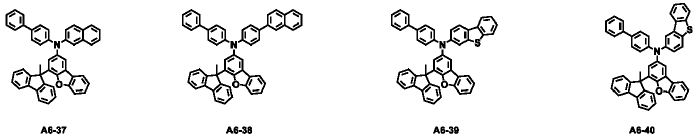

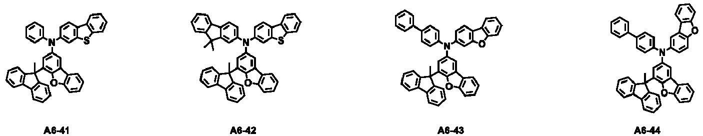

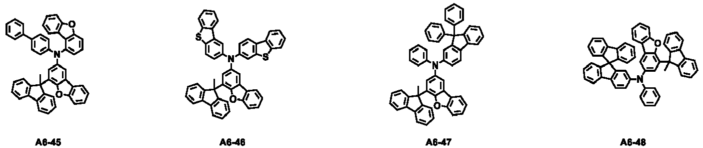

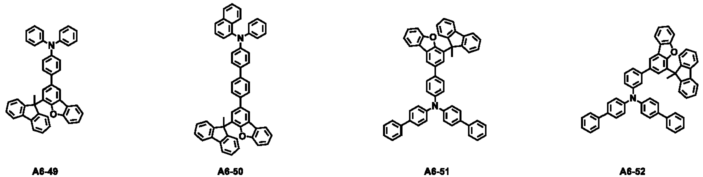

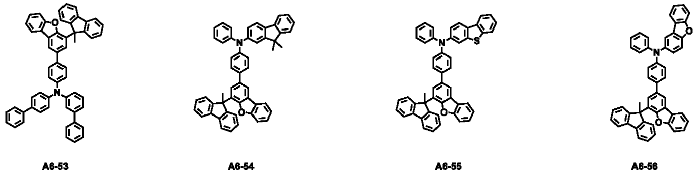

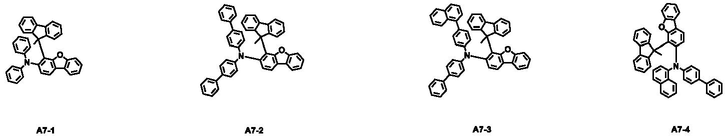

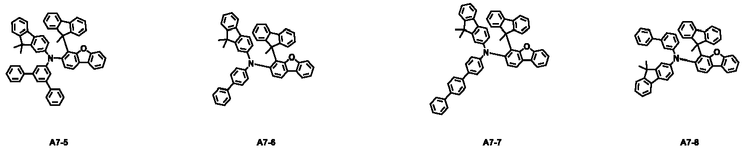

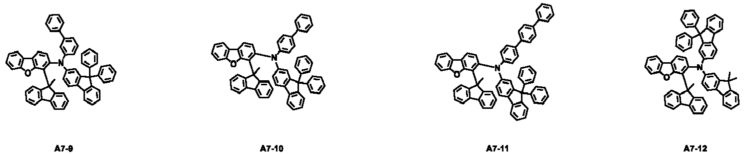

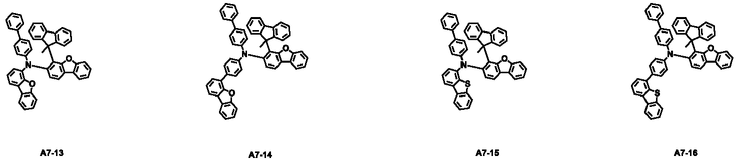

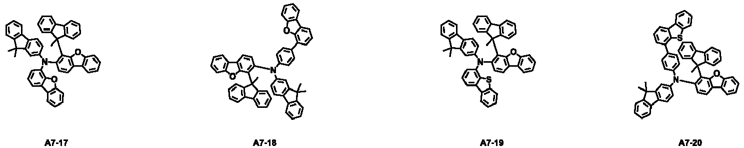

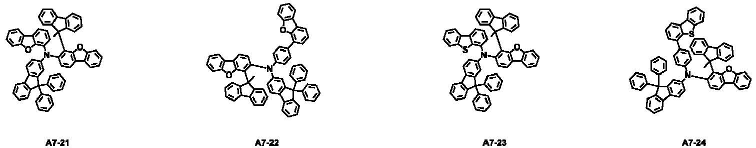

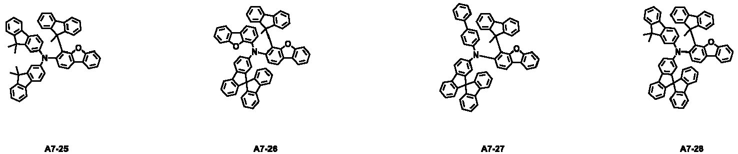

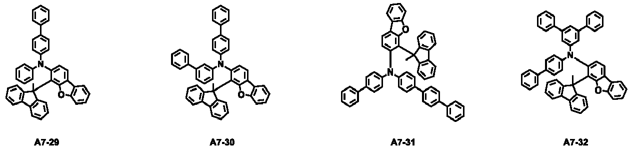

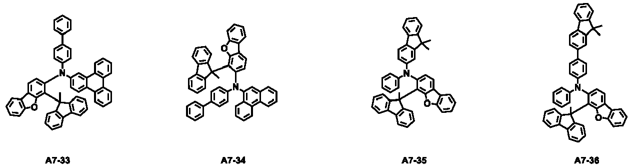

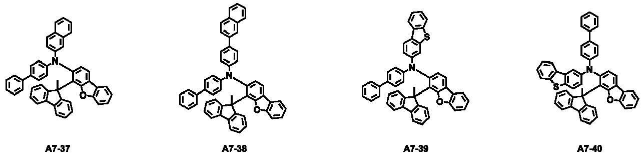

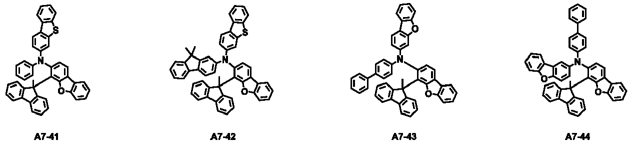

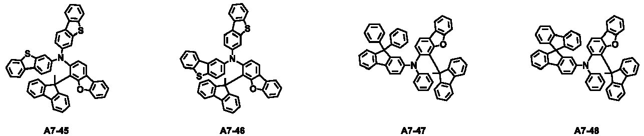

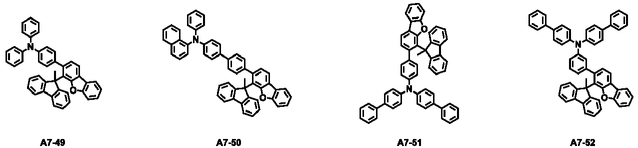

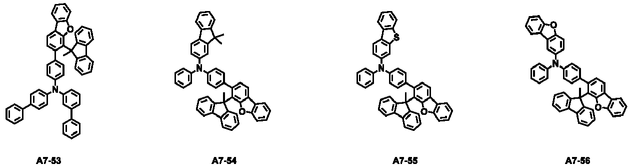

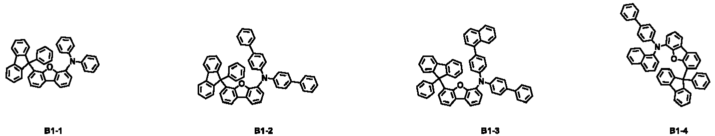

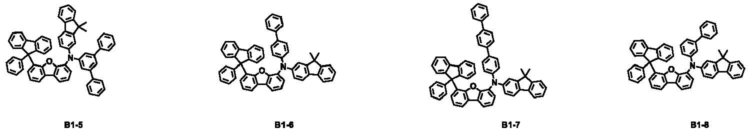

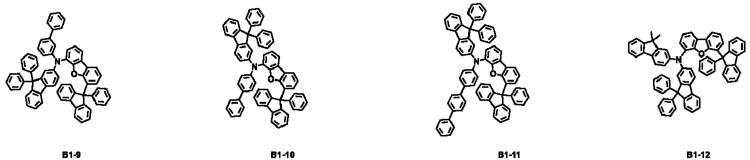

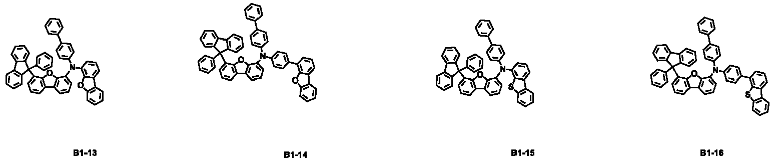

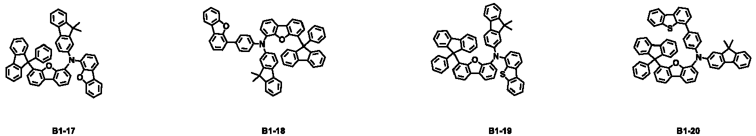

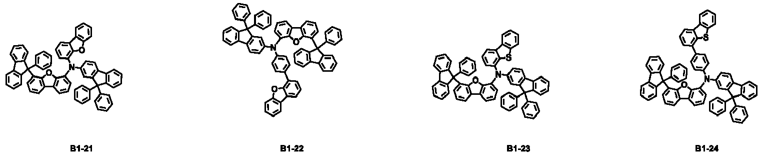

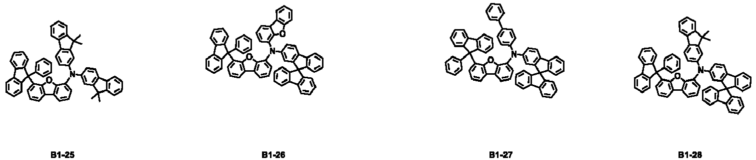

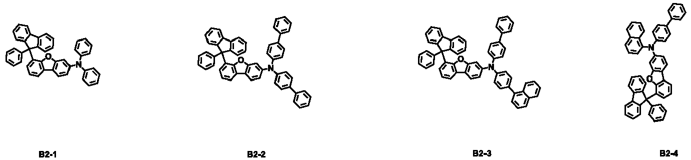

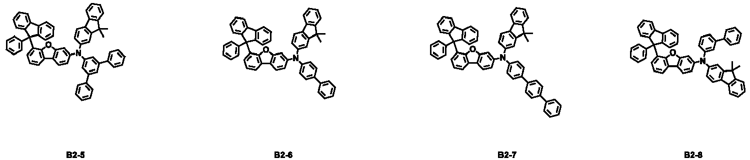

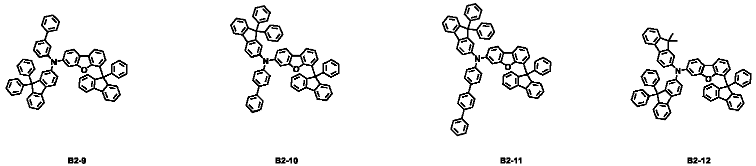

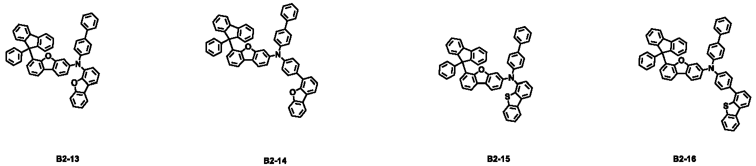

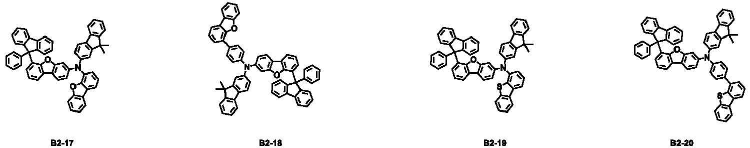

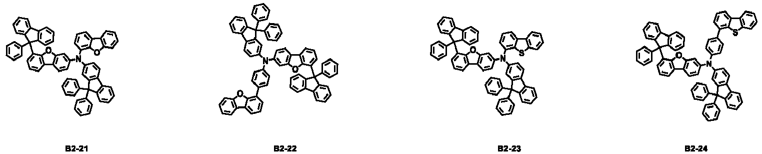

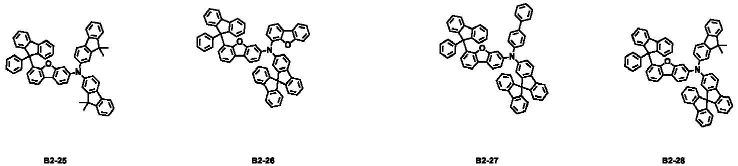

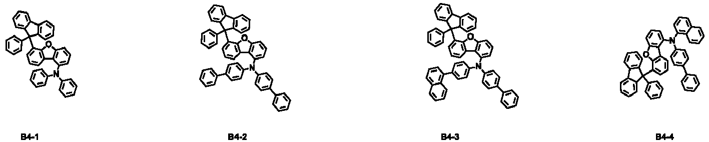

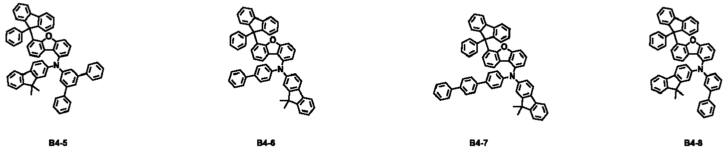

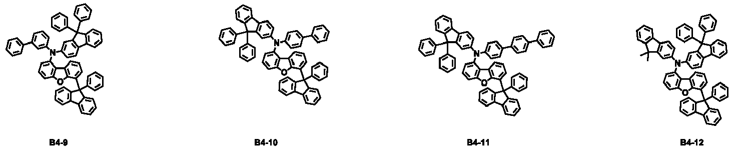

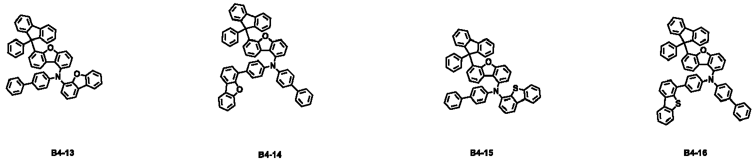

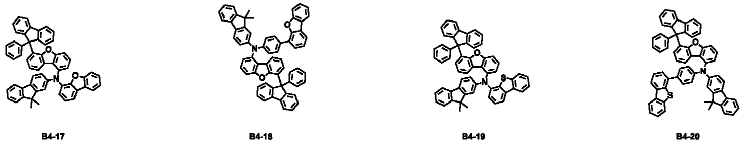

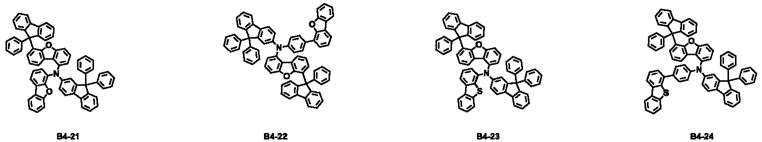

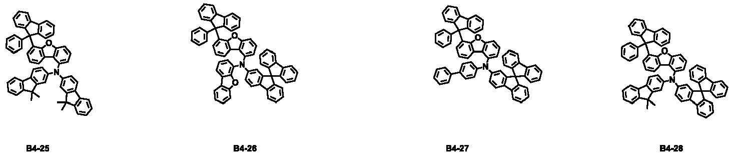

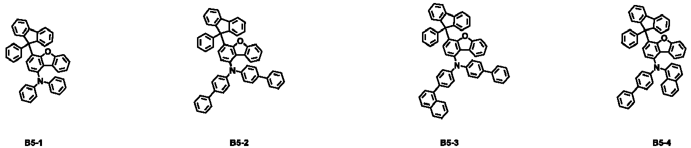

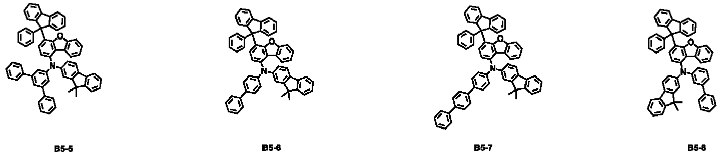

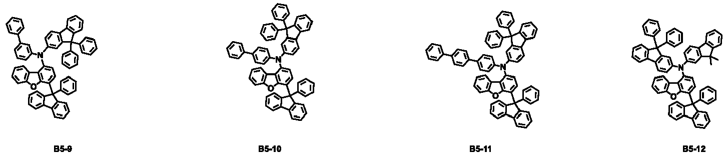

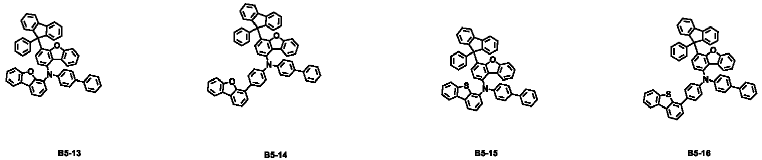

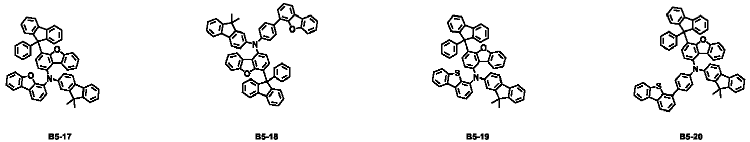

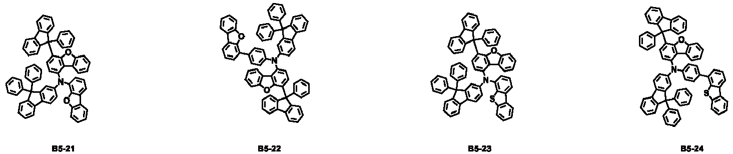

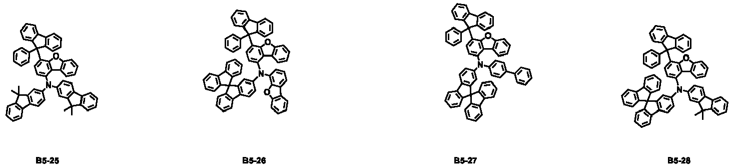

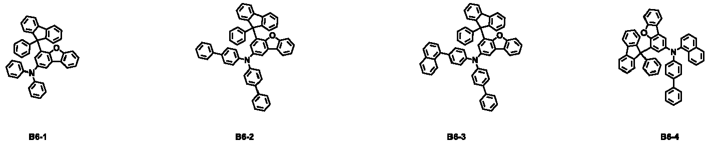

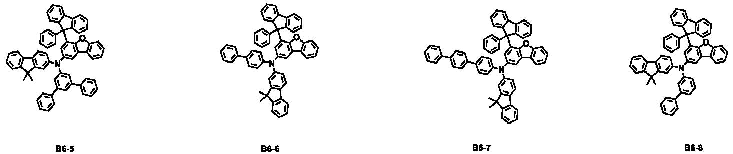

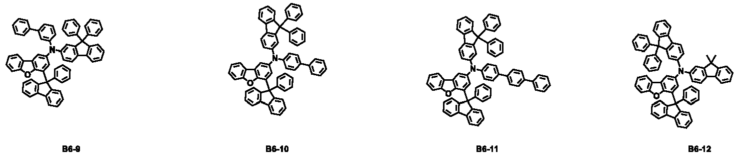

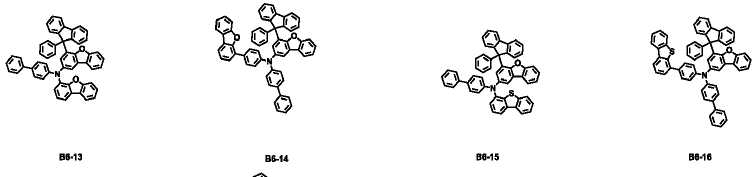

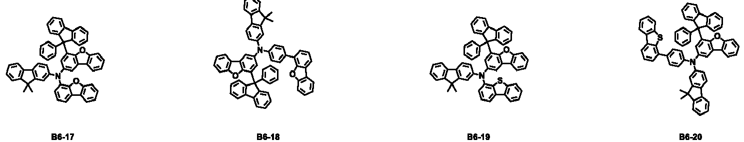

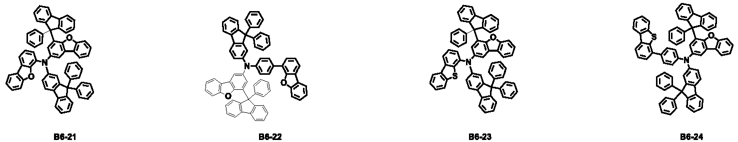

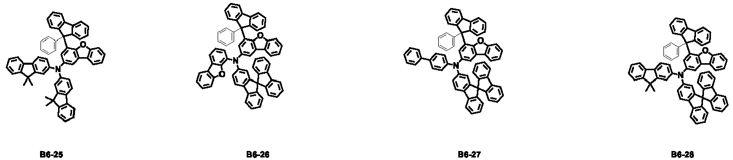

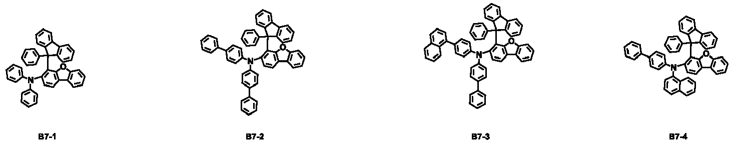

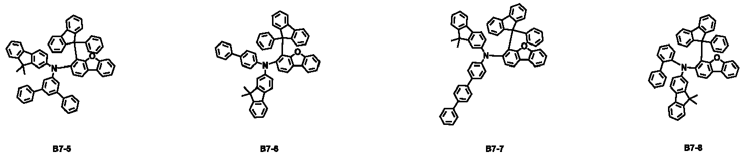

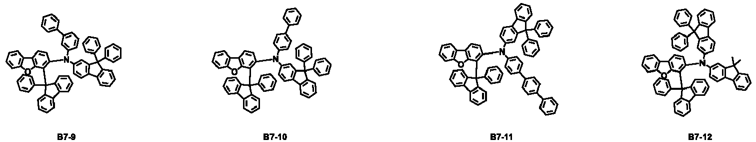

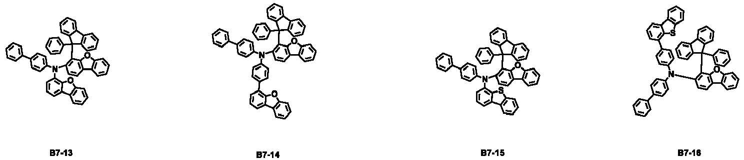

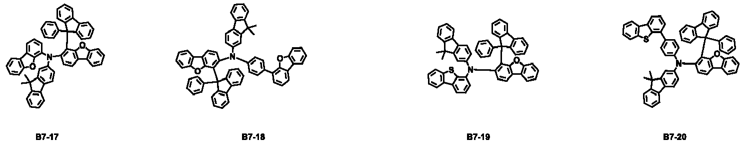

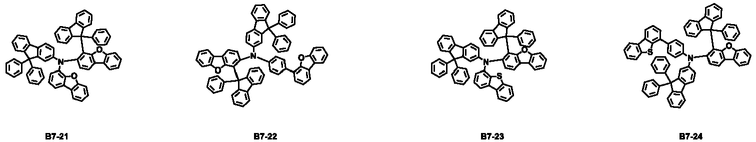

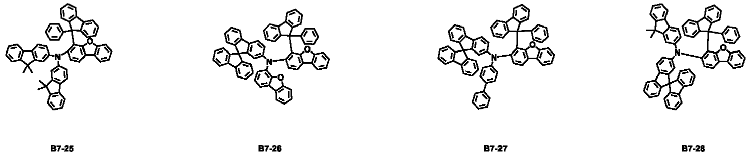

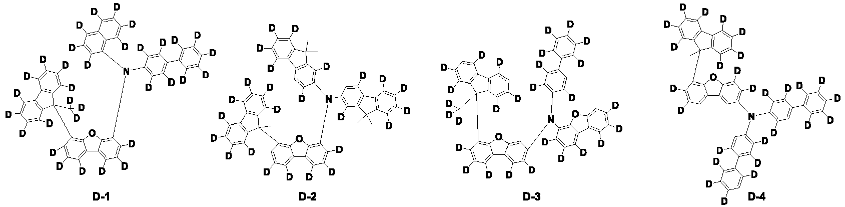

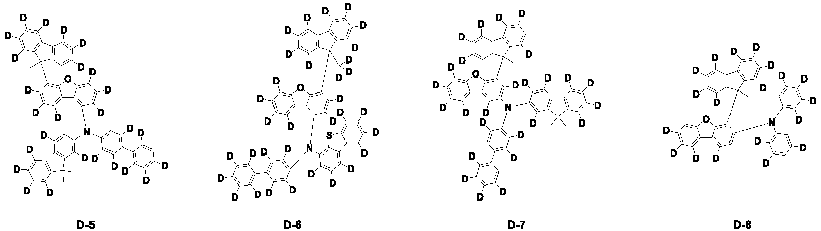

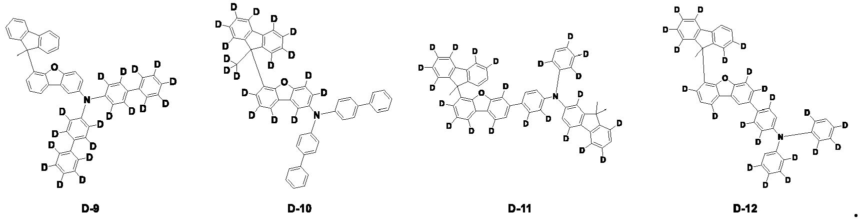

7. An organic light emitting device comprising:
an anode;
a cathode; and
one or more organic material layers provided between the anode and the cathode,
wherein one or more layers of the organic material layers comprise the hetero-cyclic compound of any one of Claims 1 to 6.

8. The organic light emitting device of Claim 7, wherein the organic material layer comprises a hole transfer layer, and the hole transfer layer comprises the hetero-cyclic compound.

9. The organic light emitting device of Claim 7, wherein the organic material layer comprises an electron blocking layer, and the electron blocking layer comprises the hetero-cyclic compound.

10. The organic light emitting device of Claim 7, further comprising one, two or more layers selected from the group consisting of a light emitting layer, a hole injection layer, a hole transfer layer, an electron blocking layer, an electron injection layer, an electron transfer layer, a hole auxiliary layer and a hole blocking layer.
